# EUROPEAN PATENT SPECIFICATION

(11) **EP 2 639 297 B1**
(45) Date of publication and mention of the grant of the patent: **03.01.2018**
(21) Application number: 11839764.5
(22) Date of filing: 08.11.2011
(51) Int. Cl.: C12N 5/074

(54) **METHOD FOR PRODUCING PERIPHERAL BLOOD MONOCYTE-DERIVED PLURIPOTENT STEM CELLS**
VERFAHREN ZUR HERSTELLUNG VON AUS PERIPHERBLUT-MONOZYTEN ABGELEITETEN PLURIPOTENTEN STAMMZELLEN
PROCÉDÉ DE PRODUCTION DE CELLULES SOUCHES PLURIPOTENTES DÉRIVÉES DE MONOCYTES DE SANG PÉRIPHÉRIQUE

(30) Priority: 09.11.2010 JP 2010250993
(43) Date of publication of application: 18.09.2013
(73) Proprietor: National Institute of Advanced Industrial Science and Technology, Tokyo 100-8921 (JP); Japan Biological Informatics Consortium, Tokyo 135-8073 (JP)
(72) Inventor: NAKANISHI, Mahito, Tsukuba-shi Ibaraki 305-8565 (JP); NISHIMURA, Ken, Tsukuba-shi Ibaraki 305-8560 (JP); SANO, Masayuki, Tsukuba-shi Ibaraki 305-8565 (JP); OHTAKA, Manami, Tsukuba-shi Ibaraki 305-8560 (JP)
(74) Representative: Gibbs, Richard
(86) International application number: PCT/JP2011/075715
(87) International publication number: WO 2012/063817

(56) References cited:
- EP-A1- 2 434 012
- EP-A1- 2 559 757
- EP-A1- 2 612 911
- WO-A1-2010/030003
- WO-A1-2010/134526
- JP-A- 2006 325 531
- SI-TAYEB KARIM ET AL: "Generation of human induced pluripotent stem cells by simple transient transfection of plasmid DNA encoding reprogramming factors", BMC DEVELOPMENTAL BIOLOGY, BIOMED CENTRAL LTD., LONDON, GB, vol. 10, no. 1, 3 August 2010 (2010-08-03) , page 81, XP021072142, ISSN: 1471-213X, DOI: 10.1186/1471-213X-10-81
- NOEMI FUSAKI ET AL: "Efficient induction of transgene-free human pluripotent stem cells using a vector based on Sendai virus, an RNA virus that does not integrate into the host genome", PROCEEDINGS OF THE JAPAN ACADEMY. SERIES B, PHYSICAL ANDBIOLOGICAL SCIENCES, TOKYO, JP, vol. 85, no. 8, 1 October 2009 (2009-10-01), pages 348-362, XP002663242, ISSN: 0386-2208, DOI: DOI:10.2183/PJAB.85.348
- KEN NISHIMURA ET AL.: 'Saiboshitsu Jizoku Hatsugengata RNA Vector no Seishitsu Kento to Iryo Oyo' JOURNAL OF JAPANESE BIOCHEMICAL SOCIETY 2008, pages 4T26 - 7, XP008138458
- MASAYUKI SANO ET AL.: 'siRNA o Mochiita Saibo kara no Sendai Virus Vector no Sentakuteki Jokyo' JOURNAL OF JAPANESE BIOCHEMICAL SOCIETY 2008, page 2P-1429, XP008148138
- 'Sendai Virus Vector Riyo' GANKA SHINIKUI IPS SAIBOYO SAIBO O JURITSU 01 August 2008, XP008138459
- NISHIMURA, K. ET AL.: 'Persistent and stable gene expression by a cytoplasmic RNA replicon based on a noncytopathic variant Sendai virus.' J. BIOL. CHEM. vol. 282, no. 37, 14 September 2007, pages 27383 - 91, XP008135575
- SEKI, T. ET AL.: 'Generation of induced pluripotent stem cells from human terminally differentiated circulating T cells.' CELL STEM CELL vol. 7, no. 1, 02 July 2010, pages 11 - 4, XP008162341
- LOH, YH. ET AL.: 'Reprogramming of T cells from human peripheral blood.' CELL STEM CELL vol. 7, no. 1, 02 July 2010, pages 15 - 19, XP055107989
- STADTFELD, M. ET AL.: 'A reprogrammable mouse strain from gene-targeted embryonic stem cells.' NAT. METHODS vol. 7, no. 1, January 2010, pages 53 - 5, XP055083447
- NISHIO, M. ET AL.: 'Recombinant Sendai viruses with L1618V mutation in their L polymerase protein establish persistent infection, but not temperature sensitivity.' VIROLOGY vol. 329, no. 2, 24 November 2004, pages 289 - 301, XP004613860
- NISHIMURA, K. ET AL.: 'Development of defective and persistent Sendai virus vector: a unique gene delivery/expression system ideal for cell reprogramming.' J. BIOL. CHEM. vol. 286, no. 6, 11 February 2011, pages 4760 - 71, XP002684867

## Description

### TECHNICAL FIELD

The present invention relates to a method of producing a pluripotent stem cell derived from a peripheral blood monocyte.

### BACKGROUND ART

Along with the progression toward an aging society, diseases caused by tissue degeneration and damage are increasing rapidly. For example, the diseases include cerebral infarction, cardiac infarction and renal failure that increase in frequency with age due to metabolic syndromes, as well as Alzheimer's disease, Parkinson's disease and osteoporosis due to age-related tissue degeneration. In addition, type I diabetes, multiple sclerosis, chronic rheumatoid arthritis, thermal burn, spinal damage from injury, and genetic diseases caused by congenital abnormalities in the genetic code, are all diseases caused by tissue degeneration and damage. A number of regeneration therapies are being developed as a means for treating these diseases.

Regeneration therapies can be roughly classified into two groups: guided regeneration therapies designed to activate tissue stem cells residing in a patient's tissue in various ways; and cell replacement therapies designed to the transplant stem cells or stem cell-derived somatic cells or tissues. The regeneration potential of tissue stem cells is however often limited. Development of cell replacement therapies is therefore essential to the practical application of regeneration therapies. In particular, with regard to genetic diseases, it is conceivable to perform cell replacement therapies using cells which have been genetically repaired or replaced ex vivo.

A regeneration therapy-based treatment for diseases caused by tissue degeneration/damage requires the preparation of large amounts of stem cells or stem cell-derived somatic tissues. Thus, pluripotent stem cells capable of self-renewal over long periods of time while maintaining their differentiation potential into various tissue types are essential for the development of cell replacement therapies. To date only a few pluripotent stem cells have been reported that meet the requirement and include embryo-stem cells (ES cells) derived from early embryos, and EG cells derived from primordial germ cells. These cells cannot be directly used in cell replacement therapies however because a cell having genomic information non-identical to that of a patient's cell causes transplantation rejection. Therefore, as a prerequisite to performing cell replacement therapies in a safe and efficient manner, pluripotent stem cells are required to have genomic information identical to that of a patient's cell so as to avoid immunological rejection after transplantation.

It is conceivable to generate such pluripotent stem cells from a patient's tissue cell while changing a property thereof in some way. As means for generating a pluripotent stem cell having genomic information identical to that of a patient's cell, there has been known a technique of introducing or transfecting genes into a human normal tissue cell using a retroviral vector so as to forcibly induce expression of the genes to generate a human induced pluripotent stem cell (human iPS cell) closely resembling a human ES cell. For example, there have been reported a method of transfecting four types of genes consisting of Oct3/4, Sox2, Klf4 and c-Myc into a human skin-derived normal fibroblast cell using a retroviral or lentiviral vector so as to induce expression of the genes to generate a human iPS cell (see Non-Patent Document 1), and a method of transfecting four types of genes consisting of Oct3/4, Sox2, Nanog and LIN28 into a human skin-derived normal fibroblast cell using a lentiviral vector so as to induce expression of the genes to generate a human iPS cell (see Non-Patent Document 2). Further, it has been reported that by using the above-described foreign genes, human iPS cells can be prepared from various tissue cells including skin-derived fibroblast cells (see Non-Patent Documents 1 and 2), hair root-derived keratinocytes (see Non-Patent Document 3), bone marrow-derived mesenchymal stem cells (see Non-Patent Document 4), neural stem cells (see Non-Patent Document 5), adipose tissue-derived mesenchymal stem cells (see Non-Patent Document 6), mesenteric cells (see Non-Patent Document 7), dental pulp cells (see Non-Patent Document 8), tooth root cells (see Non-Patent Document 9), peripheral blood-derived mononuclear cells (see Non-Patent Documents 10 and 11), peripheral blood-derived T cells (see Non-Patent Documents 10-12), and hematopoietic progenitor cells (see Non-Patent Document 13).

It is desirable that living human tissue cells as a raw material for human iPS cells are capable of being collected by a method which does not give a strong invasion into the human body and is free from the risk of contamination with microorganisms (bacteria and viruses). Among the above-listed human tissue cells, skin-derived fibroblast cells, bone marrow-derived mesenchymal stem cells, neural stem cells, adipose tissue-derived mesenchymal stem cells, mesenteric cells, dental pulp cells and dental root cells must respectively be collected by incision of the skin, perforation of the bone marrow, craniotomy, liposuction, surgical operation, tooth extraction and gingivectomy, all being strongly invasive methods. Therefore, these cells are not desirable as a raw material for human iPS cells. Hematopoietic progenitor cells can be collected not only by perforation of the bone marrow but also from peripheral blood after pretreatment such as administration of granulocyte colony-stimulating factor (G-CSF). However, such pretreatment involes compulsive proliferation of hematopoietic stem cells and thus may increase the risk of leukemia. Such pretreatment is undesirable from the viewpoint of safety. Further, the skin, hair root and dental root are directly exposed to the air and cells derived from these tissues are most likely to have been contaminated with environmental microorganisms, so they are not desirable as a raw material for human iPS cells.

Further, it is desirable that a living human tissue cell as a raw material for human iPS cells has the lowest possoble levels of mutations resulting from damage added to genome information. This is a condition that need to be satisfied to avoid the risk of carcinogenesis, especially when human iPS cells are to be used for medical purposes. Among the above-listed human tissue cells, skin-derived fibroblast cells and hair root-derived keratinocytes are known to repeatedly undergo damage to the genome by UV rays and repair thereof (see Non-Patent Document 14). Thus, it is believed that these cells have a higher risk of mutation than other tissue cells and are not desirable as a raw material for human iPS cells.

Considering the above-described conditions, a living human tissue cell as a raw material for human iPS cells is desirably a cell contained in pheripheral blood which does not require a strongly invasive collection method or a risky pre-treatment and which can be easily obtained by blood collection of only about 10 mL as usually carried out at clinical sites. Examples of cells contained in the peripheral blood of healthy adults include, but are not limited to, lymphocytes (T cells, B cells and NK cells), granulocytes (neutrophils, basophils and eosinophils), monocytes, erythrocytes and platelets.

A living human tissue cell as a raw material for human iPS cells must be a cell that has genetic information necessary for maintaining iPS cells (nuclear genes and mitocondrial genes) and organelles (mitocondria, endoplasmic reticula, etc.). In view of this, erythrocytes and platelets which lack nucleus, and granulocytes (neutrophils, basophils and eosinophils) which almost lack mitocondria and endoplasmic reticula/Golgi apparatuses are not appropriate as a raw material for humn iPS cells.

Further, in order to secure complete pluripotency, it is desirable that a living human tissue cell as a raw material for human iPS cells is a cell which has complete genetic information identical to that of a fertilized egg and which does not have irreversible recombinations, mutations and gene deletions in its genetic information in association with cell differentiation. Specific examples of cells which have irreversible recombinations, mutations and gene deletions in their genetic information include peripheral blood-derived T cells in which rereversible recombinations have occurred in T cell receptor genes and peripheral blood-derived B cells in which irreversible recombinations have occurred in antibody genes. This means that, in principle, only one type of T cell receptor or antibody can be produced from iPS cells prepared from peripheral blood-derived T cells or B cells; such iPS cells are unable to differentiate to hematopoietic stem cells which are required to have the capacity to produce a wide variety of T cells and B cells. Therefore, among the above-listed cells, peripheral blood-derived T cells and peripheral blood-derived B cells are undesirable as a raw material for human iPS cells.

Further, since human iPS cells are prepared on mouse or nonself human-derived feeder cells, it is desirable that a living human tissue cell as a raw material for human iPS cells does not have cytotoxicity to heterologous cells and allogeneic nonself cells. Therefore, among the above-listed cells, NK cells which recognize non-autologou cells and show non-specific cytotoxicity are undesirable as a raw material for human iPS cells.

On the other hand, among the above (paragraph [0007])-listed cells, monocytes have an intact nucleus and mitcondria/organelles and retain complete genomic information that has not undergone irreversible changes such as recombination and deletion. Besides, methods for purification of monocytes have been established; they are Ficoll centrifugation in which blood cells are fractionated by difference in specific gravity and a purification method using anti-CD 14 antibody bound magnetic beads. By combining these techniques, it is possible to recover a cell population of very high purity (98% or more) in a short period of time and in an aseptic manner. Thus, monocytes are the only human tissue cell that satisfies all of the above (paragraphs [0005]-[0010])-described conditions as a raw material for superior human iPS cells. Therefore, establishing a method of preparing human iPS cells using monocytes as a raw material has critical significance in the attempt of putting pluripotent stem cells into practical use.

On the other hand, any of the cells currently used as a raw material for preparing human iPS cells proliferates through cell division under laboratory culture conditions, and it is believed that the ability to proliferate through cell division is necessary for preparation of human iPS cells (see Non-Patent Document 15). In contrast, monocytes are cells at the terminal stage of differentiation from hematopoietic stem cells and do not have proliferative capacity. Conditions to cause cell division in monocytes *in vitro* are not known. Therefore, at present, it is believed that preparation of iPS cells from monocytes is not easy to accomplish.

Peripheral blood-derived mononuclear cells obtained by partially purifying peripheral blood by Ficoll centrifugation are composed of lymphocytes (about 80%) and monocytes (about 20%). Therefore, the human iPS cells prepared from peripheral blood mononuclear cells according to the methods described in Non-Patent Documents 10 and 11 may probably contain monocytes-derived cells. In fact, the human iPS cells reported in Non-Patent Documents 10 and 11 contained iPS cells free from DNA recombination in T cell receptor or antibody gene, suggesting that these cells are derived from mononuclear cells other than T cells and B cells. However, it is unknown from what cell are derived the iPS cells that are believed to be non-T cells. No report has been published to date that shows the preparation of human iPS cells using purified monocytes.

It is known that the nature and safety of self-derived human iPS cells for use in cell replacement therapy are greatly affected by the method of preparation of such iPS cells. In the methods reported in Non-Patent Documents 1 and 2 that perform gene expression using a retroviral vector or lentiviral vector, the genes used in the preparation of human iPS cells are inserted into the chromosomes and remain therein. This means that human iPS cells prepared by these techniques do not satisfy the condition of "pluripotent stem cells having genetic information identical to that of a patient" as a requiremeent for cell replacement therapy. In the human iPS cells prepared from peripheral blood mononuclear cells by a method using a lentiviral vector as described in Non-Patent Documents 10 and 11, genes used in the preparation of human iPS cells are also inserted into the chromosomes and remain therein. Therefore, the thus prepared human iPS cells do not satisfy the condition of "pluripotent stem cells having genetic information identical to that of a patient" as a requiredment for in cell replacement therapy.

Further, this phenomenon of gene insertion evokes the following problems in terms of ensuring safety in cell replacement therapy. Briefly, when a foreign gene is inserted at non-specific positions on chromosomes, genes in the vicinity of the insertion site may be abnormally activated to cause adverse effects such as oncogenic transformation of cells. For example, it is known that when genes are inserted with a retroviral vector at non-specific positions on chromosomes of a human bone marrow stem cell that maintains replication competence over a long period of time, oncogenes whose transcription is inhibited in normal cells are abnormally activated due to the effect of inserted foreign genes, causing oncogenic transformation of cells to occur at high frequency (see Non-Patent Document 16).

This phenomenon of gene insertion further evokes the following problems in terms of ensuring safety in cell replacement therapy. Briefly, in iPS cells prepared by inserting foreign genes into chromosomes, expression of the foreign genes is inhibited as long as the undifferentiated state is maintained. However, as the iPS cell differentiates to a tissue cell, expression of the foreign genes is induced and the tissue cell may turn cancerous. For example, the mouse individual derived from mouse iPS cells prepared by introducing four genes, Oct3/4, Sox2, Klf4 and c-Myc, into skin-derived normal fibroblast cells with a retroviral vector is known to develop cancer at high frequency as a result of reactivation of the foreign c-Myc gene introduced thereinto (see Non-Patent Document 17). In addition to expression of c-Myc gene, it has been pointed out that expression of Klf4 and Oct3/4 genes may also lead to oncogenic transformation of cells (see Non-Patent Document 18).

In order to solve the various problems resulting from gene insertion into chromosomes, human iPS cells must be prepared by a method that will not leave foreign genes on chromosomes. As one example of such technology, it has been reported that after preparing human iPS cells with four genes, Oct3/4, Sox2, Klf4 and c-Myc, being inserted at random into chromosomes, the inserted genes can be removed by introducing Cre recombinase (see Non-Patent Document 19). This technology is also used in the preparation of iPS cells from peripheral blood mononuclear cells as disclosed in Non-Patent Document 11. (However, removal of the reprogramming genes is not reported in Non-Patent Document 11). According to the above technology, promoter regions necessary for expressing the reprogramming genes still remain on chromosomes. Therefore, the genetic information of the prepared iPS cells is not completely identical to the genetic information of a corresponding normal cell, and insertion mutation might possibly occur.

Yu et al. reported that seven genes, Oct3/4, Sox2, Klf4, c-Myc, Nanog, LIN28 and SV40 T antigen, were expressed simultaneously in human normal fibroblast cells using a circular DNA vector (EBV vector) having the replication origin of Epstein-Barr virus (EBV) and EBNA1 gene and being capable of extrachromosomal replication, and that foreign gene-free human iPS cells could be prepared utilizing spontaneous dropping off of the vector (see Non-Patent Document 20). Recently, Fusaki et al. reported a method in which Oct3/4, Sox2, Klf4 and c-Myc genes were expressed in human skin-derived fibroblast cells or peripheral blood-derived T cells using Sendai virus as a vector capable of expressing foreign genes without inserting them into chromosomes, to thereby prepare pluripotent stem cells (see Non-Patent Documents 21, 12 and 1). In this method, four reprogramming genes were loaded on separate vectors, and mixed infection was carried out. It was reported that pluripotent stem cells were prepared at a maximum efficiency of 1%. Another method was reported in which synthetic mRNAs encoding Oct3/4, Sox2, Klf4 and c-Myc proteins were introduced into human fibroblast cells to thereby prepare foreign gene-free human iPS cells at a maximum efficiency of 2% at the highest (see Non-Patent Document 22). However, neither of these methods is known to be capable of preparing human iPS cells from monocytes.

Accordingly, there has been reported no method that can be used to prepare human pluripotent stem cells required for cell replacement therepies or the like and which satisties the following two conditions: 1) using, as a raw material, human peripheral blood-derived monocytes that retain complete genetic information and are yet obtainable by blood collection which is less invasive and has a smaller risk of contamination with microorganisms; and 2) being a technology that enables preparation of human pluripotent stem cells where no externally introduced reprogramming genes are left in order to secure safety.

### LIST OF PRIOR ART DOCUMENTS

### [PATENT DOCUMENTS]

Patent Document 1: PCT/JP2009/062911

### [NON- PATENT DOCUMENTS]

Non-Patent Document 1: Takahashi, et al., Cell, 131, 861-872, 2007
Non-Patent Document 2: Yu, et al., Science, 318, 1917-1920,2007
Non-Patent Document 3: Aasen, et al., Nature Biotechnology, 26, 1276-1284, 2008
Non-Patent Document 4: Park, et al., Cell, 134, 877-886, 2008
Non-Patent Document 5: Kim, et al., Nature, 461, 649-653, 2009
Non-Patent Document 6: Sun, et al., Proc. Natl. Acad. Sci. USA, 106 15720-15725, 2009
Non-Patent Document 7: Li, et al., Cell Reprogram., 12 237-247, 20108
Non-Patent Document 8: Oda, et al., J. Biol. Chem., 285, 29270-29278, 2010
Non-Patent Document 9: Egusa, et al., PLos One, 5, e12743, 2010
Non-Patent Document 10: Loh, et al, Cell Stem Cell, 7, 15-19, 2010
Non-Patent Document 11: Staerk, et al., Cell Stem Cell, 7, 20-24, 2010
Non-Patent Document 12: Seki, et al., Cell Stem Cell, 7, 11-14, 2010
Non-Patent Document 13: Loh, et al., Blood, 113 5476-5479, 2009
Non-Patent Document 14: Ikehata, Environ. Mol. Mutagen., 41, 280-292, 2003
Non-Patent Document 15: Hanna, et al., Nature, 462, 595-601, 2009
Non-Patent Document 16: Hacein-Bey-Abina, et al., Science, 302, 415-419, 2003
Non-Patent Document 17: Takahashi and Yamanaka, Cell, 126, 663-676, 2006
Non-Patent Document 18: Jaenishi and Young, Cell, 132, 567-582, 2008
Non-Patent Document 19: Woltjen, et al., Nature, 458, 766-770, 2009
Non-Patent Document 20: Yu, et al., Science, 324, 797-801, 2009
Non-Patent Document 21: Fusaki, et al., Proc. Jpn. Acad. Ser. B85, 348-362, 2009
Non-Patent Document 22: Warren, et al., Cell Stem Cell, 7, 1-13, 2010

### SUMMARY OF THE INVETION

### [PROBLEM TO BE SOLVED BY THE INVENTION]

Therefore, a problem to be solved by the present invention is to ensure that induced pluripotent stem cells (hereinafter, sometimes referred to as "iPS cells") which have genetic information identical to that of a patient and yet have nature close to that of ES cells can be prepared from human peripheral blood monocytes without leaving foreign genes within the resultant cells after use for their preparation. If this problem is solved, pluripotent stem cells which are capable of avoiding immunological rejection of transplanted cells and possibilities of tumorigenesis resulting from insertion of foreign genes into chromosomes and gene damage can be prepared from a cell material obtained by blood collection which is a common medical practice that can be performed with minimum invasion.

### [MEANS FOR SOLVING THE PROBLEM]

The above-described problem can be solved by using a gene expression system that does not have activities for altering human genome through interactions such as recombination, insertion, repair, etc. and which yet is applicable to monocytes. The present inventors have found that ES/iPS cell markers are expressed efficiently by loading genes encoding the human gene products of reprogramming genes Oct3/4, Sox2, Klf4 and c-Myc on a sustained expression-inducing Sendai viral vector (Japanese Patent No. 4478788 and WO 2008/129971) and by then introducing the resultant vector into monocytes highly purified from human peripheral blood. The above-described reprogramming gene-loaded vector has no foreign genes integrated into chromosomes and yet can be easily and swiftly removed after reprogramming if siRNA is used. The present inventors have found that by introducing this vector into purified monocytes, it is possible to prepare highly safe, induced pluripotent stem cells (iPS cells) having genetic information identical to that of the individual who supplied the monocytes. Thus, the present invention has been achieved.

The present invention is specifically described as follows:
(1) A method of producing an induced pluripotent stem cell, comprising the steps of infecting a peripheral blood-derived mononuclear cell with a reprogramming gene-loaded Sendai virus vector to reprogram the mononuclear cell; and then artificially removing the reprogramming gene-loaded Sendai virus vector from the reprogrammed cell, wherein the reprogramming gene-loaded Sendai virus vector has Sendai virus genes and a reprogramming gene(s) on the same genome, the Slendai virus genes comprising NP gene, P/C gene, M gene, F gene, HN gene and L gene, provided that M gene, F gene and HN gene are all lacking in their respective functions and that the L protein expressed by the L gene of the reprogramming gene-loaded Sendai virus vector has a valine as an amino acid residue at position 1618 that enables sustained gene expression, wherein the peripheral blood-derived mononuclear cell is a monocyte.
(2) The method of (1), wherein the reprogramming gene-loaded Sendai virus vector is removed from the reprogrammed cell by siRNA.
(3) The method of (1), wherein the reprogramming gene-loaded Sendai virus vector comprises, on its genome, a target sequence for a microRNA expressed in a pluripotent stem cell.
(4) The method of (1), wherein the peripheral blood-derived mononuclear cell is derived from human.
(5) An induced pluripotent stem cell prepared by the method of any one of (1) - (4).

### [EFFECT OF THE INVENTION]

Since the Sendai viral vector used in the present invention is installed with a plurality of reprogramming genes simultaneously and is capable of expressing those genes at a time in the same cell, operations for reprogramming a differentiated cell are extremely simple, efficient and highly reproducible. This is a remarkable characteristic which is not seen in a method in which a plurality of vectors are separately loaded with different reprogramming genes and in mixed for use. The reprogramming gene-loaded Sendai viral vector used in the present invention expresses reprogramming genes persistently as it is retained continuously and stably in the cytoplasm. Therefore, the vector has no potential to insert foreign genes into the chromosome and thus will not cause malignant transformation of cells. The viral vector used in the present invention is extremely safe. By using this vector, induced pluripotent stem cells (hereinafter, sometimes referred to as iPS cells) which have genetic information identical to that of a patient and yet have pluripotency close to that of ES cells can be prepared from 10⁴ or less human monocytes that can be collected with minimum invasion.

After inducing expression of the reprogramming gene in the cell cytoplasm, the reprogramming gene-loaded Sendai viral vector can be easily removed from the cell using siRNA that targets a preselected sequence that is incorporated into the Sendai virus genome. Alternatively, the vector can be removed more easily from the cell using the function of endogenous microRNA (miRNA). As a result, even greater safety is obtained and, at the same time, an iPS cell which has genetic information completely identical to that of the individual who supplied the differentiated cell can be obtained. Considering these points, the obtained iPS cells have great potential for application to humans.

Moreover, the wide host cell specificity/cellular specificity of the Sendai virus means that pluripotent stem cells can be established from a wide variety of human tissue cells other than fibroblast cells (i.e. blood cells). This makes it possible to confirm the function of the pluripotent stem cells using a nonhuman animal.

Therefore, according to the present invention, human iPS cells that have complete genetic information and which yet are highly safe in the absence of foreign reprogramming genes can be prepared from human peripheral blood monocytes obtained by blood collection -a method that is less invasive and has a smaller risk of contamination with microorganisms- in an extremely efficient and simple manner with high reproducibility. This is a great advance that has been impossible to achive by those methods that use DNA vectors (such as adenoviral or EBV vector) or conventional Sendai viral vectors, which have been used in iPS cell preparation. With this advance it has become possible to prepare human iPS cells easily from peripheral blood even in general medical institutions, which greatly contributes to progress in a wide range of technical fields including regenerative therapy (especially, cell replacement therapy and gene therapy), research in drug development using iPS cells from patients with various genetic backgrounds, manufacturing of biopharmaceuticals using human cells, elucidation of causes of cancer and intractable diseases and development of effective treatments, and so on.

The present specification encompasses the contents disclosed in the specification and/or drawings of Japanese Patent Application No. 2010-250993 based on which the present patent application claims priority.

### BRIEF DESCRIPTION OF THE DRAWINGS

FIG. 1 shows an outline for preparing a template cDNA for producing hOct4/hSox2/hKlf4/hc-Myc sustained expression-inducing Sendai viral vector Version 1.
FIG 2 shows an outline for preparing a template cDNA for producing hOct4/hSox2/hKlf4/hc-Myc sustained expression-inducing Sendai viral vector Version 2.
FIG. 3 is a diagram showing the preparation of a template cDNA for producing a hOct4/hSox2/hKlf4/hc-Myc sustained expression-inducing Sendai viral vector Version 3.
FIG. 4 a diagram showing the preparation of a template cDNA for producing hOct4/hSox2/hKlf4/hc-Myc sustained expression-inducing Sendai viral vector Version 4.
FIG. 5 is a graph obtained by quantitatively measuring a temporal change in removal of a sustained expression-type Sendai viral vector from a cell, using siRNA.
FIG 6 depicts a photograph and a graph showing a comparison between the gene expression patterns of recombinant exogenous genes cloned into a single common sustained expression-type Sendai viral vector versus where each exogenous gene is cloned into an individual sustained expression-type Sendai viral vector.
FIG. 7 is a series of time-lapse photographs using a phase contrast microscope showing expression of alkaline phosphatase in a human embryonic fibroblast cell infected with the hOct4/hSox2/hKlf4/hc-Myc sustained expression-inducing Sendai viral vector.
FIG 8 depicts a photograph showing expression of endogenous human Nanog gene in a human embryonic fibroblast cell on the 14th day after infection with the hOct4/hSox2/hKlf4/hc-Myc sustained expression-inducing Sendai viral vector.
FIG. 9 depicts a photograph showing expression of SSEA-4 antigen in a human embryonic fibroblast cell on the 25th day after infection with the hOct4/hSox2/hKlf4/hc-Myc sustained expression-inducing Sendai viral vector.
FIG. 10 depicts two photographs showing expression of Sendai virus NP gene and endogenous human Nanog gene in a human iPS marker-expressing cell after removal of a sustained expression-inducing Sendai viral vector using an siRNA.
FIG 11 depicts a photograph showing expression of SSEA-4 antigen and endogenous human Oct4 protein in a human iPS marker-expressing cell after removal of a sustained expression-inducing Sendai viral vector using an siRNA.
FIG 12 depicts a photograph of a tissue slice of a teratoma derived from a human iPS marker-expressing cell after removal of a hOct4/hSox2/hKlf4/hc-Myc sustained expression-inducing Sendai viral vector therefrom.
FIG. 13 depicts a graph showing the temporal change in emergence efficiency of a mouse iPS marker-expressing cell, after transfection with either a single common sustained expression-type Sendai viral vector comprising four types of reprogramming genes or after transfection with individual sustained expression-type Sendai viral vectors each comprising a different reprogramming gene.
FIG. 14 depicts photographs showing the establishment of a human iPS marker-expressing cell using the hOct4/hSox2/hKlf4/hc-Myc sustained expression-inducing Sendai viral vector Version 3.
FIG. 15 depicts a photograph of a human iPS marker-expressing cell established from adult human peripheral blood mononuclear cells using a hOct4/hSox2/hKlf4/hc-Myc sustained expression-inducing Sendai viral vector.
FIG. 16 shows the purity of purified human peripheral blood-derived monocytes.
FIG 17 shows the expression of human iPS/ES cell markers in human peripheral blood-derived monocytes on day 8 of their infection with hOct4/hSox2/hKlf4/hc-Myc sustained expression-inducing Sendai viral vectors.
FIG. 18 shows photographs of phase-contrast microscopy images of human iPS cells prepared from human peripheral blood-derived monocytes using hOct4/hSox2/hKlf4/hc-Myc sustained expression-inducing Sendai viral vectors.
FIG. 19 shows the expression of human iPS/ES cell markers in human iPS cells prepared from human peripheral blood-derived monocytes using hOct4/hSox2/hKlf4/hc-Myc sustained expression-inducing Sendai viral vectors.
FIG 20 shows the analysis of T cell receptor genes in the genome DNA of human iPS cells prepared from human peripheral blood-derived monocytes using hOct4/hSox2/hKlf4/hc-Myc sustained expression-inducing Sendai viral vectors.
FIG. 21 shows the results of observation of teratoma tissue sections derived from human peripheral blood monocytes-derived iPS cells that had been freed of hOct4/hSox2/hKlf4/hc-Myc sustained expression-inducing Sendai viral vectors.
FIG 22 shows the results of analysis of the capacity of redifferentiation into blood cells of human peripheral blood monocytes-derived iPS cells that had been freed of hOct4/hSox2/hKlf4/hc-Myc sustained expression-inducing Sendai viral vectors.
FIG. 23 shows the results of gene expression in human peripheral blood monocytes-derived iPS cells established with hOct4/hSox2/hKlf4/hc-Myc sustained expression-inducing Sendai viral vectors, as compared with gene expressions in human fibroblast cell-derived iPS cells and human ES cells.
FIG. 24 shows the results of examination of the rearrangement of T cell receptor β chain (TCRB) gene in the genome of human peripheral blood monocytes-derived iPS cells established with hOct4/hSox2/hKlf4/hc-Myc sustained expression-inducing Sendai viral vectors.
FIG. 25 shows the results of examination of the rearrangement of T cell receptor γ chain (TCRG) gene in the genome of human peripheral blood monocytes-derived iPS cells established with hOct4/hSox2/hKlf4/hc-Myc sustained expression-inducing Sendai viral vectors.
FIG 26 shows the results of examination of the rearrangement of T cell receptor δ chain (TCRD) gene in the genome of human peripheral blood monocytes-derived iPS cells established with hOct4/hSox2/hKlf4/hc-Myc sustained expression-inducing Sendai viral vectors.
FIG. 27 shows the results of examination of the rearrangement of immunoglobulin heavy chain (IGH) gene in the genome of human peripheral blood monocytes-derived iPS cells established with hOct4/hSox2/hKlf4/hc-Myc sustained expression-inducing Sendai viral vectors.

### DESCRIPTION OF EMBODIMENTS

A vector loaded with a reprogramming gene for use in producing an induced pluripotent stem cell in the present invention is a Sendai virus particle which has an NP gene, a P/C gene and an L gene each derived from a Sendai virus, and at least one of F, M and HN genes of the Sendai virus is functionally deleted (this vector will hereinafter be referred to as "Sendai virus vector").

As used in this specification, the term "gene" or "gene material" encompasses negative-strand RNA or cDNA and positive-strand RNA or cDNA complementary thereto. In other words, any vector capable of synthesizing either one of such genes or gene materials by means of transcription or reverse transcription should be construed as being included in the present invention.

As used in this specification, the term "induced pluripotent stem cell (iPS cell)" means a cell which expresses a morphology similar to an embryonic stem cell (ES cell), and an embryonic stem cell-specific marker, and has a self-renewal ability in vitro. It is known that the iPS cell has the potential to differentiate into any of the three germ layers in vivo or in vitro. The marker includes a well-known type, such as Nanog, SSEA-4, TRA-1-60, TRA-1-81, Oct4 and the like.

### [CONSTITUENT MATERIALS OF SENDAI VIRUS VECTOR]

The Sendai virus vector is a gene transfection/expression vector in which any exogenous gene is inserted into a genome of a Sendai virus or used to replace a gene of a Sendai virus thus enabling the expression of the exogenous gene. A Sendai virus has an NP gene, a P/C gene, an F gene, an M gene, an NH gene and an L gene, wherein each of the NP, P/C and L genes is involved in the transcription and replication of the Sendai virus, and each of the F, M and HN genes is involved in formation of a virus particle. Therefore, a Sendai virus vector subjected to functional deletion of the F, M and NH genes is incapable of forming new virus particles by itself, i.e., is non-transmissible, after transfection into a cell.

The Sendai virus vector used in the present invention comprises an L gene which encodes an L protein having a valine residue at position 1618. This mutation was found in the amino-acid sequence of an L protein derived from the Sendai virus strain Cl.151. It is known that the Sendai virus strain CL 151 exhibits temperature-sensitive growth, where almost no virus particle is produced at 38 °C, but at 32 °C, the replication cycle becomes active and permits the production of virus particles. The Sendai virus strain C1.151 was first reported by Tetsuya Yoshida, PhD, in 1979 (Yoshida, et al., (1979), Virology, 92, 139-154).

In the Sendai virus strain Cl.151 having a gene which encodes an L protein having a valine residue at position 1618, an ability to induce interferon activity is lowered, and therefore a sustained infectious ability is exhibited without cytotoxicity, so that, when an exogenous gene is borne thereby, expression of the gene will be maintained in the cell for a long period of time. For example, as the L gene, it is possible to use a mutated L gene obtained by mutating the L gene of the Sendai virus strain Nagoya in such a manner that a leucine residue at position 1618 of the L gene is replaced by valine. As described herein, the L protein having a valine residue at position 1618 will be referred to occasionally as a "mutated-L protein,".

Thus, as long as the L gene has the above mutation, each of the NP, C/P and L genes as constituent genes of the Sendai virus vector used in the present invention may have a base sequences of a Sendai virus strain with cytotoxicity and without a sustained infectious ability, such as a Sendai virus strain Nagoya or Z, as well as a base sequence of the Sendai virus strain Cl. 151.

A transcriptional termination sequence of a Sendai virus may be artificially inserted into the 3'-terminal end of the leader RNA, which makes it possible to reduce the copy number of anti-genomic RNAs and further lower the interferon-inducing ability.

As a prerequisite to sustained infection of a Sendai virus in an animal cell, it is essential that the Sendai virus has the mutated-L gene, and all of the F, M and HN genes are derived from the Sendai virus strain Cl.151. Thus, the Sendai virus vector having the mutated-L gene and the Sendai virus strain Cl.151-derived F, M and HN genes together can have a sustained infectious ability without cytotoxicity, so that, when an exogenous gene is installed on the Sendai virus vector, the expression of the gene will be maintained in the cell over a long period of time. In Sendai virus vectors based on the strain Cl.151, one or more (including "all") of the strain Cl. 151-derived F, M and HN genes may be functionally deleted, without interfering with the ability to drive the expression of the gene loaded thereon. In this case, although the functional deletion of only one of the three genes allows the transmissibility of the vector to be significantly suppressed, it is preferable to functionally delete all of the F, M and HN genes in order to fully suppress the transmissibility. The functional deletion of one or more of the F, M and HN genes may be achieved by simply deleting a part or all of the three genes, or by insertion or replacement by a given exogenous gene.

A full-length cDNA of the Sendai virus strain Cl.151 has already been registered in the GenBank (Accession Number AB275416),

### [REPROGRAMMING GENE]

A reprogramming gene is inserted into the Sendai virus vector of the present invention. The reprogramming gene may include: a combination of Oct3/4, Sox2 and Klf4 genes of human, mouse or any other mammal; the combination plus c-Myc gene of human, mouse or any other mammal; and the combination plus one or more of Nanog, Lin28, Esrrb, UTFI, TERT (telomerase catalytic subunit) and SV40 T antigen genes, but is not limited thereto.

### [TEMPLATE VECTOR FOR PREPARING REPROGRAMMING GENE-LOADED SENDAI VIRUS VECTOR]

In the present invention, the NP, P/C and mutated-L genes as constituent materials of the Sendai virus vector are inserted into a cloning vector such as phage, together with the reprogramming genes. In this process, all of the required reprogramming genes can be inserted together. This allows an aftermentioned reprogramming gene-loaded Sendai virus vector to contain all of the genes required for reprogramming, so that the reprogramming can be quite efficiently performed without the need for transfecting each reprogramming gene into a different vector as in the conventional techniques.

The recombinant vector obtained in the above manner can serve as a template for preparing a reprogramming gene-loaded Sendai virus vector used in the present invention, i.e., a Sendai virus particle bearing the reprogramming genes. This recombinant vector will hereinafter be referred to as "template vector".

In regard to the template vector, the NP, P/C and mutated-L genes and the reprogramming genes are incorporated into a vector such as phage, in the following order: NP - P/C - reprogramming genes (a marker gene may further be transfected thereinto) - mutated L.

The reprogramming genes or the marker gene may also be used to functionally delete at least one of the F, M and NH genes of the Sendai virus vector, by inserting it into the at least one gene or by replacing the sequence of the at least one gene therewith.

A selectable marker gene, such as a drug-resistance gene, may also be inserted into the template vector. This makes it possible to facilitate screening of a target cell into which the template vector or the Sendai virus vector is inserted.

More specifically, the template vector is prepared by combining the constituent materials of the Sendai virus vector comprising the above genes, and the reprogramming gene cDNA and/or the marker gene cDNA, together in the above order, in such a manner as to allow a (+) strand genomic RNA to be formed in a cell. For example, the constituent material cDNA is incorporated into a cloning vector, such as λ DASH II. A T7 promoter sequence and three guanidine residues are then arranged on the upstream side of the incorporated full-length cDNA (i.e.. at the 3'-terminal end of the genomic RNA) in this order, and a hairpin ribozyme sequence of a tobacco ringspot virus and a termination sequence of T7 RNA polymerase are then arranged on the downstream side of the full-length cDNA (i.e. at the 5'-terminal end of the genomic RNA) in this order.

The T7 promoter sequence is added to allow a (+) strand genomic RNA to be biosynthesized from the 3'-terminal end of the genomic RNA by T7 RNA polymerase, and three guanidine residues are added to enhance the efficiency of RNA transcription by the T7 RNA polymerase (S. Leyrer, et al., (1998) J. Virol. Methods, 75; 47-58). The hairpin ribozyme sequence of the tobacco ringspot virus is added to allow the transcript (+) strand genomic RNA to be accurately cut at one end, and the termination sequence of T7 RNA polymerase is added to allow the RNA transcription by the T7 RNA polymerase to accurately terminate.

### [PREPARATION OF REPROGRAMMING GENE-LOADED SENDAI VIRUS VECTOR]

The template vector with the reprogramming genes prepared in the above manner can then be transfected into a viral vector-producing cell in order to prepare a reprogramming gene-loaded Sendai virus vector.

In order to transcribe (+) strand anti-genomic RNA from the template vector in a virus-producing cell, it is necessary to supply T7 RNA polymerase. For example, the viral vector-producing cell can be infected with T7 RNA polymerase-expression vaccinia virus, or may be a cell strain cloned to constantly express T7 RNA polymerase.

In the cell strain (BHK/T7 cell) capable of constantly expressing humanized T7 RNA polymerase, the expression level of T7 RNA polymerase is significantly increased as compared with a cell strain (BSR-T7-5 cell) capable of expressing conventional bacterial T7 RNA polymerase. As a result of production of recombinant viruses using the BHK/T7 cell, recombinant viruses can be efficiently collected. In other words, the use of a cell strain having an enhanced expression level of T7 RNA polymerase is effective for producing recombinant viruses efficiently.

In the virus vector-producing cell transfected with the template vector, T7 RNA polymerase drives transcription of the template vector DNA to RNA, initially from the T7 promoter. However, in the produced RNA molecule, sequences downstream of the hairpin ribozyme sequence are cleaved off and removed by the hairpin ribozyme sequence, so that a (+) strand anti-genomic RNA molecule is generated corresponding to a DNA portion which includes the NP gene, the P/C gene, the reprogramming genes and the mutated-L gene in the template vector, and may further include a marker gene.

An expression vector for producing NP, P and L gene products may be additionally transfected into the viral vector-producing cell having the (+) strand anti-genomic RNA transcribed from the template vector by the T7 RNA polymerase. In this case, the NP, P and L gene products are bound to the (+) strand anti-genomic RNA to form an RNP complex (nucleocapsid). Then, using the RNP complex as a template, a (-) strand genomic RNA is transcribed from the (+) strand anti-genomic RNA by the RNA polymerase in the viral vector-producing cell. The (-) strand genomic RNA is bound to NP, P and mutated-L gene products in the viral vector-producing cell to form a RNP complex including the (-) strand genomic RNA.

In the template vector used in the above manner, one or more of the strain Cl. 151-derived M, F and HN genes are functionally deleted to allow the vector to become non-transmissible. Thus, the RNP complex formed based on this template vector is suppressed from formation of virus particles required for infecting a tissue cell. Therefore, an expression vector capable of expressing a missing one or more of the F, M and HN gene products is transfected into the virus vector-producing cell comprising the RNP complex (nucleocapsid) with the (-) strain genomic RNA formed in the above manner. The transfected cell is then incubated at a virus-particle production temperature of 32 °C.

Consequently, the RNP complex (nucleocapsid) including the (-) strand genomic RNA is incorporated into viral vector particles to reconstruct reprogramming gene-loaded Sendai virus particles. As described above, in the present invention, an expression vector loaded with a missing one or more of the F, M and HN genes is separately transfected into a virus-producing cell to form virus particles. This makes it possible to harvest the virus particles from a culture supernatant of the viral vector-producing cell. In cases where two or more of the F, M and HN genes are missing, an expression system may comprise a plurality of vectors each transfected with a respective one of the missing genes, or may comprise a vector transfected with all of the missing genes.

In the above virus-particle production process, with a view to assisting the formation of virus protein to efficiently produce virus particles, it is preferable to transfect an expression vector having an NP gene, a P/C gene and an L gene into the cell, in addition to the functionally-deleted or defective genes.

In addition, a drug-resistance gene may be inserted into a target viral vector-producing cell as discussed above. In this case, it becomes possible to select the viral vector-producing cell through incubation in culture medium containing an appropriate drug. Alternatively, a target viral vector-producing cell may be isolated using a marker gene, such as the EGFP gene.

The reprogramming gene-loaded Sendai virus vector obtained in the above manner is in the form of a virus particle that is capable of sustainedly infecting a differentiated cell. However, as one or more of the F, M and HN genes of the vector are functionally deleted, the formation of virus particles required for the infection or transmission is suppressed after the transfection. Thus, the vector is non-transmissible. In addition, the L gene of the vector is mutated such that leucine of the L protein at position 1618 is replaced with valine. Thus, the vector has sustained infectious ability without interferon-inducing ability, so that it can sustainedly express the reprogramming genes while being retained in the cell over a long period of time.

### [REPROGRAMMING OF DIFFERENTIATED CELL]

The reprogramming gene-loaded Sendai virus vector obtained in the form of a virus particle in the above manner is then transfected into a target differentiated cell to be subjected to reprogramming. For example, a human peripheral blood-derived monocyte was used as the target differentiated cell. The target differentiated cell is not limited to human cells, but may include differentiated cells of an animal, such as mouse, rat, hamster, guinea pig, rabbit, dog, cat, monkey, bovine, pig, sheep, goat or chicken, which is known to be capable of being infected with a Sendai virus. This is because the Sendai virus is capable of infecting various cells derived from an extremely wide variety of animal species. A known cell incapable of being infected with the Sendai virus is only equine-derived cells and B-lymphocytes of various animal species (Nakanishi, et al., J. Cont. Rel., 54, 61-68, 1998). This feature is an advantage significantly different from other viral vectors that exhibit a narrow host range, such as a retroviral vector, a lentiviral vector and an adenoviral vector; or other gene expression systems usable only in human cells, such as an EBV vector; or plasmid vectors, transposon and EBV vectors that can be transfected into only limited cell species due to a need for being used in combination with a physical gene introduction process.

In the reprogramming gene-loaded Sendai virus vector used in the present invention, an L gene is mutated to have no interferon induction activity, and M, F and HN genes in a wild-type Sendai virus are functionally deleted. Thus, the vector exhibits a sustained infectious ability without cytotoxicity, and, after infection of a differentiated cell, stably exists in the cytoplasm of the infected cell independently with respect to the chromosome. Even after cell division, this state is maintained. This feature is not observed in other types of Sendai virus vectors without a mutated-L gene or with at least one of the M, F and HN genes of the wild-type Sendai virus. Thus, the use of the Sendai virus vector used in the present invention makes it possible to maintain expression of the reprogramming genes for 10 to 20 days, which is required for the completion of the reprogramming process. Reprogramming can therefore be completed by a single gene transfection. This advantage is not available with adenoviral vectors or plasmid vectors that are only capable of inducing transient gene expression. For example, in cases where Oct3/4, Sox2, Klf4 and c-Myc are loaded on the reprogramming gene-loaded Sendai virus vector used in the present invention, even if the vector is transfected into a cell only once, a cell capable of sustained expression of SSEA-4 and TRA-1-60 as markers of an embryo-stem cell (ES cell) appears after the 7th to 14the days.

As long as the Sendai virus vector used in the present invention is used, there is no possibility that a part or all of the introduced genes are inserted at non-specific positions on a chromosome. Thus, resulting iPS cells are significantly safe because there is no risk of causing canceration. In this regard, the Sendai virus vector of the present invention is significantly advantageous, compared to a system causing genes to be inserted at non-specific positions on a chromosome, such as a retroviral vector or a lentiviral vector/transposon. The Sendai virus vector of the present invention is also significantly advantageous, compared to a system where a possibility of gene insertion into chromosomes is hardly denied, such the an adenovirus vector or a plasmid vector (including the EBV vector). For example, it is known that c-Myc, Oct4 or Lin28 in the reprogramming genes causes cell canceration or heteromorphism by itself. Even these genes can be safely used in combination with the Sendai virus vector used in the present invention.

A vector system for use in reprogramming a cell requires a property capable of establishing pluripotent stem cells having uniform properties with excellent reproducibility. In this connection, it is necessary that a plurality of (e.g., four) types of reprogramming genes can be simultaneously transfected into a single cell, and the transfected reprogramming genes are always expressed at a constant rate. In cases where a plurality of types of genes are transfected into a cell by a Sendai virus vector, the following two processes are contemplatable: one process of loading all of the genes on a single common vector as illustrated in inventive examples; and the other process of loading the genes on respective ones of a plurality of vectors and infecting a cell with the vectors after mixing them together, as illustrated in the Non-Patent Documents 12 and 21 and the Patent Document 1. A difference in gene expression between the two processes was verified by comparing the following two cases: one case where the Enhanced Green Fluorescent Protein (EGFP) gene and the Kusabira-Orange (KO) gene are loaded on a single common vector; and the other case where the two genes are loaded on respective viral vectors, individually. The result shows that it is necessary to load a plurality of types of genes on a single common vector in order to allow exogenous genes to be expressed at a constant ratio with excellent reproducibility (Example 7). It was also ascertained that all of the reprogramming genes have to be loaded on a single common vector in order to induce iPS marker-expressing cell with higher efficiency (Example 11). As above, it became evident that, based on the use of the reprogramming gene-loaded Sendai virus vector used in the present invention where four types of reprogramming genes are loaded on a single common vector, pluripotent stem cells having uniform properties can be established with significantly high efficiency while constantly ensuring excellent reproducibility.

### [REMOVAL OF REPROGRAMMING GENES]

The reprogramming gene-loaded Sendai virus vector used in the present invention is transfected into a differentiated cell in an infectious manner, and the reprogramming genes are sustainedly expressed in the cytoplasm of the cell to reprogram the cell. In order to make the genetic information of the reprogrammed cell identical to that of the original or pre-programming cell, the reprogramming genes which have become unneeded have to be removed from the cell. In the present invention, the entire reprogramming gene-loaded Sendai virus vector is removed using a siRNA. The siRNA is designed to target the L gene of the Sendai virus vector. According to experimental results by the inventors, the reprogramming gene-loaded Sendai virus vector can be completely removed by targeting the L gene, although the reprogramming gene-loaded Sendai virus vector can also be removed to some extent by targeting the NP gene or the P gene. For example, a target region of the L gene may be a region including a portion encoding a nucleotide residue of the L protein at position 527 or 1913. The target region is not particularly limited to such a region, buy may be any other suitable region. The siRNA is transfected into the cell 5 to 20 days after infecting the differentiated cell with the reprogramming gene-loaded Sendai virus vector.

It is contemplatable that microRNA (miRNA) existing in a cell may be used instead of siRNA. miRNA is a small RNA transcribed from the genome of an animal cell, and capable of interacting with DNA, mRNA and protein to adjust the functions thereof. In then interaction with RNA, there exists a mechanism where the miRNA binds to a target sequence on the mRNA to induce decomposition of the mRNA or suppress translation of the mRNA to thereby suppress gene expression. It is known that a target sequence for a specific miRNA is artificially inserted into a protein-noncoding region of mRNA transcribed from certain gene, to allow expression of the gene to be suppressed in a cell where the miRNA is expressed, based on the above mechanism. Thus, a target sequence for miRNA appearing only in a reprogrammed cell may be added to an L, NP or P gene-noncoding region of the Sendai virus vector. In this case, expression of the L, NP or P gene can be suppressed based on the above mechanism, in the same manner as that based on the siRNA, so as to remove the reprogramming gene-loaded Sendai virus vector.

The miRNA to be used for the above purpose may include, but is not limited to, mir-302a that is specifically expressed, for example, in human or mouse ES cells. For example, the technique of removing the reprogramming gene-loaded Sendai virus vector using miRNA has an advantage of being able to automatically remove the Sendai virus vector without the need for externally transfecting siRNA.

As above, the reprogramming gene-loaded Sendai virus vector can be removed by using siRNA that targets the L gene, or by culture at high temperatures, or through the use of a Sendai virus vector in which a target sequence for miRNA is added to the noncoding region of the L, NP or P genes. Thus, a resulting iPS cell does not have the exogenous genes inserted into the chromosome, so that it has genomic information identical to that of a donor individual of the differentiated cell. Further, it has a long-term self-renewal capability in vitro.

Various examples of the present invention are described below. It is understood that the present invention is not limited to the following examples.

### EXAMPLES

### EXAMPLE 1: Preparation of Cells for constructing Sustained Expression-Type Sendai Viral vectors

A cDNA (SEQ ID NO: 1 in the following Sequence Listing) encoding T7 RNA polymerase where codons are optimized to improve expression in an animal cell, was cloned into a plasmid pCX4SRalpha-neo vector for preparing a retroviral vector (Akagi, et al., PNAS, 100 13567-13572, 2003). A cDNA encoding Sendai virus strain C1.151 M protein (Nishimura, et al., JBC, 282, 27383-27391, 2007) was first cloned into a plasmid pCX4SRalpha-puro vector for preparing a retroviral vector (Akagi, et al., PNAS, 100 13567-13572, 2003). The plasmid DNAs were then introduced into respective PLAT-E packaging cells (Morita, et al., Gene Therapy, 7, 1063-1066, 2000) using Lipofectamine 2000 (Life Technologies), and retroviruses (T7 RNA polymerase recombinant retrovirus and 151M recombinant retrovirus) obtained from a culture supernatant. The T7 RNA polymerase recombinant retrovirus was transfected into BHK-21 cells. The infected BHK-21 cells were then transferred to a Dulbecco's Modified Minimal Essential Medium (DMEM) containing 800 µg/ml of G418 and 10% of fetal bovine serum (FCS), and G418-resistant cells (BHK/T7 (SE)) which stably express T7 RNA polymerase were isolated. Subsequently, the 151M recombinant retrovirus was transfected into BHK/T7(SE) cells and the infected BHK/T7 (SE) cells were transferred to a DMEM containing 800 µg/ml of G418, 15 µg/ml of puromycin and 10% of FCS. G418 + puromycin-resistant cells (BHK/T7/151M (SE)) which stably express T7 RNA polymerase and an M protein were isolated.

### EXAMPLE 2: Preparation of hOct4/hSox2/hKlf4/hc-Myc Sustained Expression-Inducing Sendai Viral Vector Version 1

### Preparation of Vector cDNA

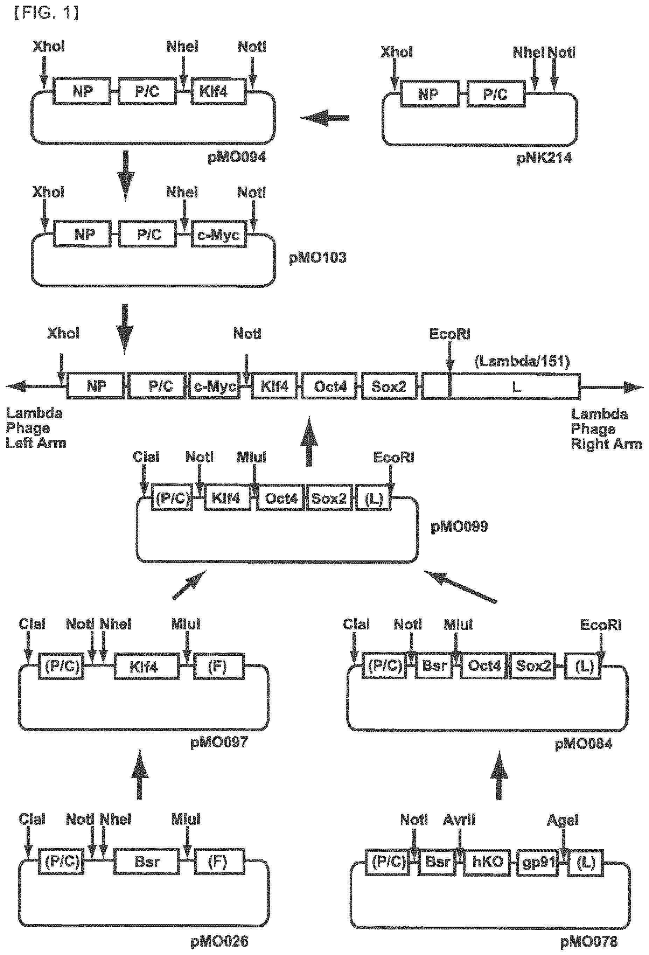

A double-stranded DNA (SEQ ID NO: 2 in the Sequence Listing) including Avr II recognition sequence, human Oct4 ORF, Sendai virus (SeV) genome cDNA (bases 6617 to 6666), human Sox2 ORF and Age I recognition sequence in this order was synthesized, and then cloned into the plasmid vector pUC57 (the cloning was entrusted to GenScript Inc.) (pUC57-OctSox). A DNA sequence cut from the pUC57-OctSox with Avr II and Age I was inserted between Arv II and Age I sites of a plasmid vector pMO078 (SEQ ID NO: 3 in the Sequence Listing) where Cla I recognition sequence, SeV strain Cl.151 genome cDNA (bases 2871 to 3650), Not I recognition sequence, a blasticidin S-resistance gene, Mlu I recognition sequence, SeV strain Cl.151 genome cDNA (bases 4728 to 4828), Avr II recognition sequence, humanized Kusabira-Orange gene, SeV strain Cl.151 genome cDNA (bases 6617 to 6666), gp91phox gene, Age I recognition sequence and SeV strain Cl.151 genome cDNA (bases 8442 to 10479) had been inserted into a plasmid pBluescript II SK(+) (Agilent Technologies Inc.) in this order. In this manner, a plasmid pMO084 was obtained (FIG 1).

A double-stranded DNA (SEQ ID NO: 4 in the Sequence Listing) including Nhe I recognition sequence, human Klf4 ORF, Sendai virus transcription termination sequence, Sendai virus transcription initiation sequence and Not I recognition sequence in this order was synthesized, and then cloned into a plasmid vector pUC57 (the cloning was entrusted to GenScript Inc.) (pUC57-KLF4). Human Klf4 gene was amplified by PCR from pUC57-KLF4 using the following two primers: 5'-ACTAGCTAGCAGTCTGACATGGCTGTCAGCGACGCGCT-3' (SEQ ID NO: 5 in the Sequence Listing; N-terminal side) and 5'-GGTCCACGCGTTTAAAAATGCCTCTTCATGTG-3' (SEQ ID NO: 6 in the Sequence Listing; C-terminal side). The ends of the resultant double-stranded DNA were cut with Nhe I and Mlu I, and then the DNA was inserted between Nhe I and Mlu I sites of pMO026 [a plasmid vector in which Cla I recognition sequence, SeV strain Cl.151 genome cDNA (bases 2871 to 3650), Not I recognition sequence, Nhe I recognition sequence, blasticidin S resistance gene, Mlu I recognition sequence and SeV strain C1.151 genome cDNA (bases 4728 to 5335) had been inserted into pBluescript II SK(+) in this order] (SEQ ID NO: 7 in the Sequence Listing) to thereby obtain pMO097. Subsequently, Cla I-Mlu I fragment from pMO097 was linked to Cla I-Mlu I fragment from pMO084 to thereby obtain pMO099 (FIG. 1).

A DNA sequence cut from the pUC57-KLF4 with Nhe I and Not I was inserted between Nhe I and Not I sites of a plasmid vector pNK214 (SEQ ID NO: 8 in the Sequence Listing) in which SeV strain Nagoya genome cDNA (bases 1 to 43), Sendai virus transcription termination sequence, SeV strain Nagoya genome cDNA (bases 56 to 2870), SeV strain Cl.151 genome cDNA (bases 2871 to 3656), Nhe I recognition sequence and Not I recognition sequence had been inserted into pBluescript II SK(+) (Agilent Technologies Inc.) in this order. In this manner, plasmid pMO094 (SEQ ID NO: 9 in the Sequence Listing) was obtained (FIG 1). Human c-Myc gene was amplified by PCR from plasmid pJL1 comprising full-length human c-Myc cDNA using the following primers: 5'-ACTAGCTAGCTTAGACGCTGGATTTTTTTCGGGTAGTGG-3' (SEQ ID NO: 10 in the Sequence Listing; N-terminal side) and 5'-GTCCGACGTCCTTACGCACAAGAGTTCCGT-3' (SEQ ID NO: 11 in the Sequence Listing; C-terminal side). The ends of the resultant double-stranded DNA were cut with Nhe I and Aat II, and then the DNA was inserted between Nhe I-Aat II sites of pMO094 to thereby obtain pMO103 (FIG 1).

After obtaining these plasmids, a DNA fragment containing T7 promoter sequence through SeV: 1-3655 was cut out from pMO103 and a DNA fragment containing SeV: 3655-10480 was cut out from pMO099. These fragments were combined with a DNA fragment containing SeV: 10480-1538+λDASHII right arm obtained by digesting λ/151 (Nishimura, et al., JBC, 282, 27383-27391, 2007) with EcoR I and cloned together to thereby prepare λ/SeVp (Mp+myc, ΔM::Klf4, ΔF::Oct4, ΔHN::Sox2) (FIG 1) (cDNA complementary to the full-length genome of h-cMyc/hOct4/hSox2/hKlf4 sustained expression-inducing Sendai viral vector Version 1 is shown in SEQ ID NO: 12 in the Sequence Listing).

### (2) Preparation of hOct4/hSox2/hKlf4/hc-Myc Sustained Expression-Inducing Sendai Viral Vector Version 1

BHK/T7/151M (SE) cells were seeded on 6-well plates at a density of 5 × 10⁵ cells/well, and washed after 24 hr cultivation. λ/SeVp (Mp+myc, ΔM::Klf4, ΔF::Oct4, ΔHN::Sox2) phage DNA, NP protein expression plasmid pGEM/NP, P protein expression plasmid pGEM/P, L protein expression plasmid pGEM/L (pGEM/NP, pGEM/P and pGEM/L are described in Garcin, et al., EMBO J., 14, 6087-6094, 1995), F protein expression plasmid pSRD-FZmut and HN protein expression plasmid pMKIT-NaHN (pSRD-FZmut and pMKIT-NaHN are described in Taira, et al., Arch. Virol., 140, 187-194, 1995) were suspended in 300 µL of Opti-MEM at quantitative ratios of 2 µg, 1 µg, 1 µg, 1 µg, 1 µg and 1 µg, respectively. The thus obtained suspension was mixed with 300 µL of Opti-MEM (Life Technologies) containing 10 µL of Lipofectamine 2000. The mixture was left at room temperature for 20 minutes and added to the cells, which were then cultured for 4 hours. Then, the cells were washed again, and after addition of 10% FCS-containing DMEM, cultured further at 32°C for 3 days. Then, the cells were cultured further at 37°C for 3 days. The resultant cells were stained by the fluorescence antibody technique using antibodies to Sendai virus NP protein and antibodies to hOct4, hSox2 and hKlf4 gene products to thereby confirm that reconstruction of the vector genome had occurred in the transfected cells. This cell population was used as hOct4/hSox2/hKlf4/hc-Myc sustained expression-inducing Sendai viral vector Version 1 producing cells without further cloning.

2 µg each of three defective gene-expression plasmids, pMKIT-151M (Taira, et al., Arch. Virol., 140, 187-194, 1995), pSRD-ZFmut and pMKIT/NaHN, was introduced into 5.0 x 10⁵ hOct4/hSox2/hKlf4/hc-Myc sustained expression-inducing Sendai viral vector Version 1 producing cells using Lipofectamine 2000. After 4 hours, the cells were washed, and, after addition of 10% FCS-containing DMEM thereto, cultured further at 32°C for 4 to 9 days. Subsequently, the culture supernatant containing the hOct4/hSox2/hKlf4 sustained expression-inducing Sendai viral vector was harvested, filtered through a 0.45 µm filter and, if necessary, ultracentrifuged to concentrate the vector. The vector suspension was quickly frozen using liquid nitrogen, and cryopreserved at - 80°C.

### EXAMPLE 3: Preparation of hOct4/hSox2/hKlf4/hc-Myc Sustained Expression-Inducing Sendai Viral Vector Version 2

### (1) Preparation of Vector cDNA

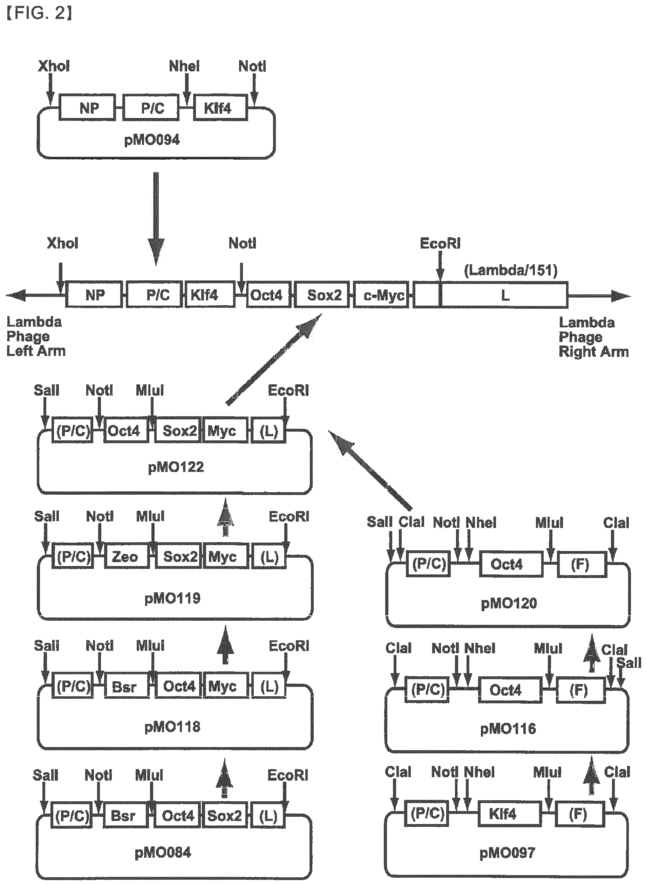

Human c-Myc gene was amplified by PCR from plasmid pJL1 comprising full-length human c-Myc cDNA using the following primers: 5'-ACTAGCTAGCTTAGACGCTGGATTTTTTTCGGGTAGTGG-3' (SEQ ID NO: 13 in the Sequence Listing; N-terminal side) and 5'-GTCCACCGGTCTTACGCACAAGAGTTCCGT-3' (SEQ ID NO: 14 in the Sequence Listing; C-terminal side). The ends of the resultant double-stranded DNA were cut with Nhe I and Age I. The thus obtained DNA was inserted between Nhe I-Age I sites of pMO084 prepared in Example 2 to thereby obtain plasmid pMO118 (FIG. 2).

Human Sox2 gene was amplified by PCR from pUC57-Sox2 using the following primers: 5'- AGTACCTAGGCGCATGTACAACATGATGGAGACGG-3' (SEQ ID NO: 15 in the Sequence Listing; N-terminal side) and 5'-GTCCGACGTCCTCACATGTGTGAGAGGGGCAGT-3' (SEQ ID NO: 16 in the Sequence Listing; C-terminal side). The ends of the resultant double-stranded DNA were cut with Avr II and Aat II. The thus obtained DNA was inserted between Avr II-Aat II sites of pMO118 to thereby obtain pMO119 (FIG. 2).

Human Oct4 gene was amplified by PCR from pUC57-Oct4 using the following primers: 5'-ACTAGCTAGCGGTTCCCCATGGCGGGACACCTGGCTTCGG-3' (SEQ ID NO: 17 in the Sequence Listing; N-terminal side) and 5'-GGTCCACGCGTTCAGTTTGAATGCATGGGAGAGCC-3' (SEQ ID NO: 18 in the Sequence Listing; C-terminal side). The ends of the resultant double-stranded DNA were cut with Nhe I and Mlu 1. The thus obtained DNA was inserted between Nhe I-Mlu I sites of pMO097 to thereby obtain pMO116. Subsequently, the orientation of the Cla I-Cla I fragment of pMO116 was reversed to thereby obtain pMO120. Then, the Sal I-Mlu I fragment of pMO119 was linked to the Sal I-Mlu I fragment of pMO120 to thereby obtain pMO122 (FIG 2).

After obtaining these plasmids, a DNA fragment containing T7 promoter sequence through SeV: 1-3655 was cut out from pMO094 (Example 2, FIG. 1) and a DNA fragment containing SeV: 3655-10480 was cut out from pMO122. These fragments were combined with a DNA fragment containing SeV: 10480-1538 + λDASHII right arm obtained by digesting λ/151 with EcoR I and cloned together to thereby prepare λ/SeVp (Mp+Klf4, ΔM::Oct4, ΔF::Sox2, ΔHN::c-Myc) (FIG. 2) (cDNA complementary to the full-length genome of hOct4/hSox2/hKlf4/hc-Myc sustained expression-inducing Sendai viral vector Version 2 is shown in SEQ ID NO: 19 in the Sequence Listing).

### (2) Preparation of hOct4/hSox2/hKlf4/hc-Myc Sustained Expression-Inducing Sendai Viral Vector Version 2

hOct4/hSox2/hKlf4/hc-Myc sustained expression-inducing Sendai viral vector Version 2 was prepared from the above-described cDNA complementary to the full-length genome of hOct4/hSox2/hKlf4/hc-Myc sustained expression-inducing Sendai viral vector Version 2 in the same manner as described in (2) in Example 2.

### Example 4: Preparation of hOct4/hSox2/hKlf4/hc-Myc Sustained Expression-Inducing Sendai Viral vector Version 3 capable of being Automatically Removed from iPS Cell

### (1) Preparation of Vector cDNA

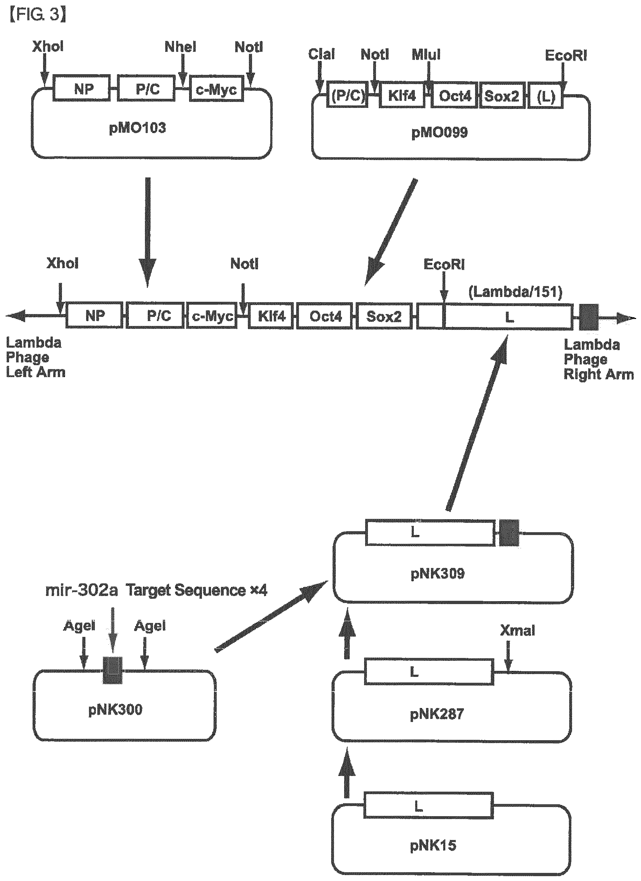

In order to clone a sequence formed by connecting four target sequences for mir-302a which is ES cell-specific of miRNA, two sets of oligo DNAs consisting of a set of 5'-CCGGTTATCACCAAAACATGGAAGCACTTACGATTCACCAAAACATGGAA GCACTTAGGTACC-3' (SEQ ID NO: 20 in the Sequence Listing) and 5'-TAAGTGCTTCCATGT TTTGGTGAATCGTAAGTGCTTCCATGTTTTGGTGATAA-3' (SEQ ID NO: 21 in the Sequence Listing) and a set of 5'-TCACCAAAACATGGAAGCACTTACGATTCACCAAAA CATGGAAGCACTTAA-3' (SEQ ID NO: 22 in the Sequence Listing) and 5'-CCGGTTAAGT GCTTCCATGTTTTGGTGAATCGTAAGTGCTTCCATGTTTTGGTGAGGTACC-3' (SEQ ID NO: 23 in the Sequence Listing) were annealed, and then ligated together. Ligated DNA was cloned into pGL4.12 (Promega Corp.) cut at Age I site to obtain pNK300 (FIG 3).

A plasmid vector pNK15 (SEQ ID NO: 24 in the Sequence Listing) was prepared by inserting the SeV strain Cl.151 genome cDNA (bases 9014 to 15384), a hairpin ribozyme sequence of a tobacco ringspot virus and a termination sequence of T7 RNA polymerase into pBluescript II SK(+) (Agilent Technologies, Inc.)). Then, using 5'-GACAGCTCGTAATCCC GGGTCCCTATCGTGC-3' (SEQ ID NO: 25 in the Sequence Listing (sense strand)) and 5'-GCACGATAGGGACCCGGGATTACGAGCTGTC-3' (SEQ ID NO: 26 in the Sequence Listing (antisense strand)) as an Xma I-recognition sequence insertion site-forming primer, an Xma I-recognition sequence was inserted into the plasmid vector pNK15 at a site just after SeV (15244) by a Quickchange Site-directed Mutagenesis II kit (Agilent Technologies, Inc.), to obtain pNK287. A fragment obtained by cutting the pNK300 at Age I site was inserted into the Xma I site of the pNK287 to generate pNK309 (FIG. 3).

T7 promoter sequence to SeV (1 to 3655 with c-Myc), and SeV (3655 to 10480 with Klf4/Oct4/Sox2), was cut out from pMO103 and pMO099 as described in Example 2, and the connected SeV (9014 to 15384)-hairpin ribozyme sequence-T7 RNA polymerase termination sequence was cut out from the pNK309. Then, these fragments were combined with a DNA fragment consisting of right and left arms of the λ DASH II, and the obtained combination was cloned to create λ/SeVp (Mp + myc, ΔM:: Klf4, ΔF:: Oct4, ΔHN:: Sox2, L + mir302T4) (FIG. 3) (a cDNA complementary to a full-length genome of a hOct4/hSox2/hKlf4/hc-Myc sustained expression-inducing Sendai viral vector Version 3 is described as SEQ ID NO: 27 in the Sequence Listing).

### (2) Preparation of hOct4/hSox2/hKlf4/hc-Myc Sustained Expression-Inducing Sendai Viral vector Version 3

In accordance with the process described in the Example 2(2), a hOct4/hSox2 /hKlf4/hc-Myc sustained expression-inducing Sendai viral vector Version 3 was prepared from the cDNA complementary to a full-length genome of the hOct4/hSox2/hKlf4/hc-Myc sustained expression-inducing Sendai viral vector Version 3.

### EXAMPLE 5: Preparation of hOct4/hSox2/hKlf4/hc-Myc Sustained Expression-Inducing Sendai Viral Vector Version 4 to Be Automatically Removed from iPS Cells

### (1) Preparation of Vector cDNA

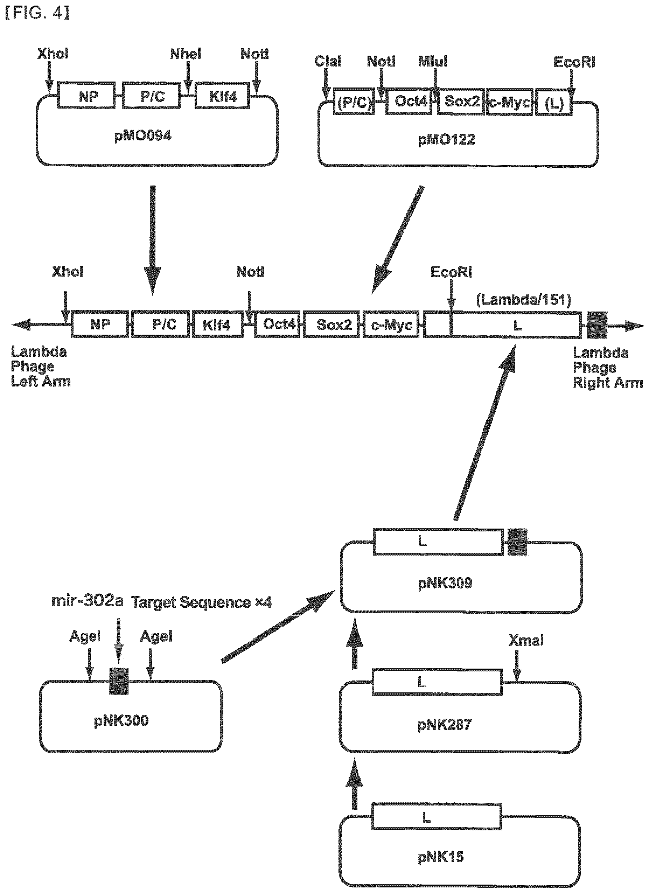

A DNA fragment containing T7 promoter sequence through SeV: 1-3655 was cut out from pMO094 prepared in Example 3; a DNA fragment containing SeV: 3655-10480 was cut out from pMO122; and a DNA fragment containing SeV: 9014-15384 - hairpin ribozyme sequence - T7 RNA polymerase termination sequence was cut out from pNK309 prepared in Example 4. These fragments were combined with DNA fragments of λDASHII right arm and left arm, and cloned together to thereby prepare λ/SeVp (Mp+Klf4, ΔM::Oct4, ΔF::Sox2, ΔHN::c-Myc, L+mir302T4) (FIG. 4) (cDNA complementary to the full-length genome of hOct4/hSox2/hKlf4/hc-Myc sustained expression-inducing Sendai viral vector Version 4 is described in SEQ ID NO: 28 in the Sequence Listing).

### (2) Preparation of hOct4/hSox2/hKlf4/hc-Myc Sustained Expression-Inducing Sendai Viral Vector Version 4

hOct4/hSox2/hKlf4/hc-Myc sustained expression-inducing Sendai viral vector Version 4 was prepared from the above-described cDNA complementary to the full-length genome of hOct4/hSox2/hKlf4/hc-Myc sustained expression-inducing Sendai viral vector Version 4 in the same manner as described in Example 2(2).

### Example 6: Evaluation on Temporal Change in Removal of RNA Genome of Sustained Expression-Type Sendai Viral vector from Cell using siRNA

It is expected that the RNA genomes of sustained expression-type Sendai Viral vectors described in Examples 2-5 are stably maintained in the transfected cells so that they will be reprogrammed by the product(s) of the expressed reprogramming gene(s). On the other hand, the sustained expression-type Sendai Viral vectors loaded with foreign reprogramming genes must be removed from the transfected cells in order to produce finally complete iPS cells. Therefore, in order to find out an effective siRNA-based technique for removing the vector genome from a cell stably transfected with the RNA genome of the sustained expression-type Sendai viral vector, the present inventors quantitatively analyzed the temporal change in the removal and confirmed that no vector genome remained in the cell after siRNA treatment.

As a marker of gene expression by the sustained expression-type Sendai viral vector, unstable firefly luciferase gene (Luc2CP, Promega Corp.) and Escherichia coli hygromycin B-resistant gene (HygB) were used. A luciferase activity reflects the copy number of the recombinant Sendai viral RNAs, and the number of hygromycin B-resistant cells reflects the number of cells transfected with the sustained expression-type Sendai viral vector.

A KO/HygB/EGFP/Luc2CP-loaded sustained expression-type Sendai viral vector containing a Luc2CP gene and a HygB gene was prepared by substituting the hOct4 gene, the hSox2 gene, the hKlf4 gene and the hc-Myc gene with the Kusabira Orange (KO) gene (Medical & Biological Laboratories, Co. Ltd.), HygB gene, Enhanced Green Fluorescent Protein (EGFP) gene, and Luc2CP gene, respectively, using the same methodology as that described for the cloning of hOct4/hSox2/hKlf4/hc-Myc sustained expression-inducing Sendai viral vector (see Example 2). Two types of short interfering RNAs (siRNAs) were used for suppressing expression of L gene (#1: sense strand 5'-GGUUCAGCAUCAAAUAUGAAG-3' (SEQ ID NO: 29 in the Sequence Listing) and antisense strand 5'-UCAUAUUUGAUGCUGAACCAU-3' (SEQ ID NO: 30 in the Sequence Listing). siRNA complimentary to sea-firefly luciferase gene (Rluc, Promega Corp.) served as negative control because it did not have any homologous with the Sendai viral vector genome.

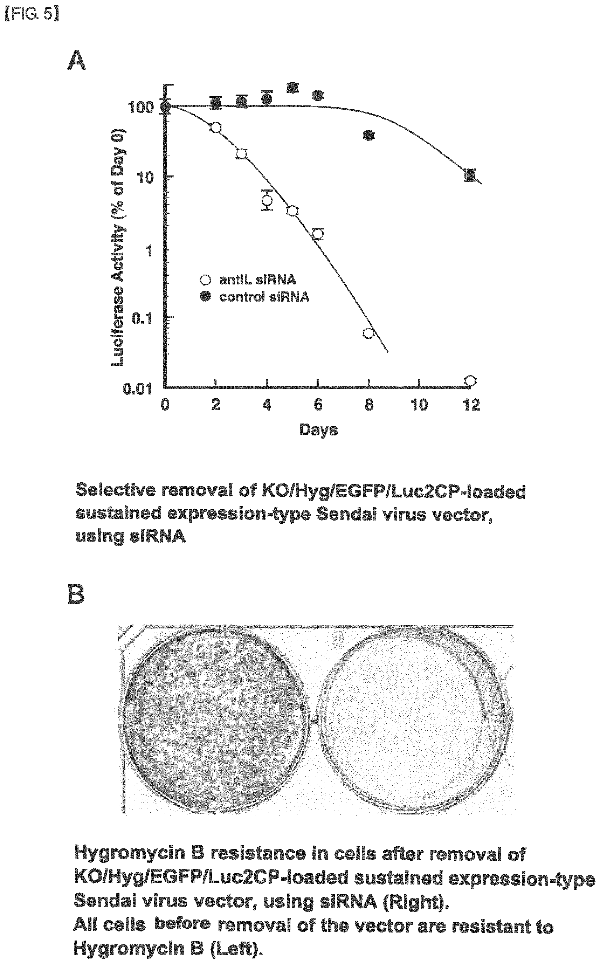

In order to check for the removal of the viral genome by the siRNA, a HeLa cell stably transduced with the genome of the sustained expression-type Sendai viral vector containing a Luc2CP gene and HygB gene was seeded into a 24-well plate at a concentration of 3 × 10⁴ cells/0.4 mL medium (MEM, 10% fetal bovine serum)/well. The siRNA was diluted with Opti-MEM to a final concentration of 40nM, and 1 µL of Lipofectamine RNAiMAX (Lifetechnologies, Inc.) was added the cell medium at room temperature for 20 minutes. Then, the siRNA was added to the above cells. Subsequently, the cells were collected at different times after transfection. On the 3rd and 6th days, the cells were subcultured under the above conditions, and the siRNA was added again using the above conditions. As a result, the luciferase activity as an index of an amount of the vector in the cell was lowered with time. On and after the 8th day, luciferase activity was no longer detectable (see FIG 5A).

Cells transfected with siRNA were passaged 3 times over a 4 week period in the absence of siRNA. Cells were then cultured in the presence of selective medium containing 200µg/mL of hygromycin B, and further cultured another week. As a result of the slection, no hygromycin B-resistance clone emerged, which demonstrates that none of the cells contained the sustained expression-type Sendai viral vector containing with the HygB gene (FIG. 5B).

### Example 7: Evaluation of the Gene Expression Patterns of Two Foreign Genes incorporated into the Sustained Expression-Type Sendai Viral vector

Previous experiments show that all four types of reprogramming genes need to be expressed simultaneously in a common cell, in order to produce an iPS cell. If the balance of expression intensity between the reprogramming genes is changed, the reprogramming efficiency decreases (Reference: Papapetrou, et al., Proc. Natl. Acad. Sci. USA, 106, 12759-12764, 2009), and a low-quality cell line having a similar configuration to an iPS cell but without pluripotency is likely to emerge (Reference: Chan, et al., Nat. Biotech., 27, 1034-1037, 2009). Thus, the method of producing iPS cells with high efficiency and excellent reproducibility needs to meet the following two requirements: 1) the four types of reprogramming genes must be introduced simultaneously into a common cell; and 2) the transduced reprogramming genes must be expressed simultaneously within each cell. To introduce the four types of reprogramming genes into a cell using the sustained expression-type Sendai viral vector, all of the reprogramming genes were cloned into a single vector, as shown in the Examples of the present invention. To determine if this *cis* configuration was more efficient at inducing iPS colonies than a *trans* configuration, each of the reprogramming genes was cloned into individual Sendai vectors. Virus produced from each of these vectors were then mixed and used to infect differentiated cells, as disclosed in the Patent Document 1 and Non-Patent Documents 12 and 21. Differences between the expression patterns of a foreign gene in the *cis* or *trans* configuration was then evaluated, by comparing expression patterns of two types of genes: the Kusabira Orange (KO) gene and Enhanced Green Fluorescent Protein (EGFP) gene present on each of the Sendai viral vectors.

The KO/HygB/EGFP/Luc2CP-loaded sustained expression-type Sendai viral vector described in Example 6 contains both the KO and EGFP genes. Further, for use as a vector loaded with only KO gene, a Zeo/KO/CLuc-loaded sustained expression-type Sendai viral vector was prepared by removing the hc-Myc gene from the hOct4/hSox2/hKlf4/hc-Myc sustained expression-inducing Sendai viral vector Version 1 as described in Example 2, and substituting the Klf4 gene, the Oct4 gene and Sox2 gene with zeocin-resistant (Zeo) gene, the KO gene and secreted luciferase (CLuc) gene, respectively For use as a vector loaded with only the EGFP gene, a Bsr/EGFP/gp91phox-loaded sustained expression-type Sendai viral vector was prepared by removing the hc-Myc gene from the hOct4/hSox2/hKlf4 sustained expression-indicing Sendai viral vector Version 1 as described in the Example 2, and substituting the Klf4 gene, Oct4 gene and Sox2 gene with Bsr gene, EGFP gene and chronic granulomatous disease-caused gene (gp91phox), respectively.

The monkey LLCMK₂ cell line was infected with the KO/HygB/EGFP/Luc2CP-loaded vector at a multiplicity of infection (m.o.i) of 5 vector particles/cell, and the resulting cells were selected with hygromycin B, to establish a cell pool LLCMK₂ (SeVdp/KO/HygB/EGFP/Luc2) containing the KO/HygB/EGFP/Luc2CP-loaded vector. In the same manner, the Zeo/KO/CLuc-loaded vector and the Bsr/EGFP/gp91phox-loaded vectors were mixed at a vector particle ratio of 1 : 1, and LLCMK₂ cells were infected with the mixed vectors at a m.o.i of 5 vector particles/cell, and the resulting cells were simultaneously selected with blasticidin S and Zeocin, to establish a cell pool LLCMK₂ (SeVdp/Zeo/KO/CLuc + SeVdp/Bsr/EGFP/gp91phox) having both vectors in each of the cells.

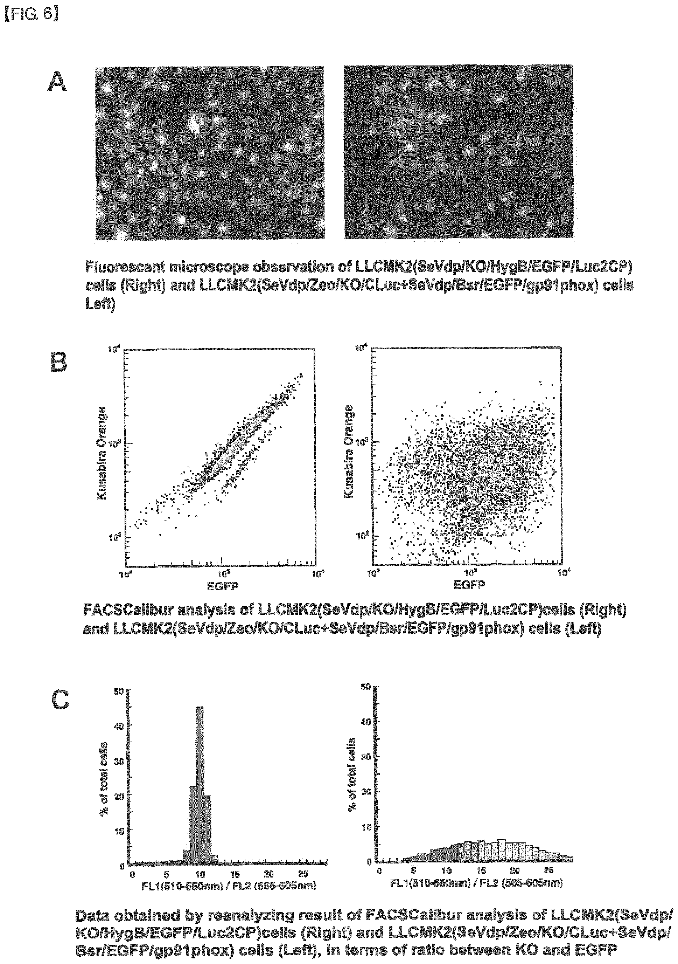

The two types of cell lines were then observed by fluorescent microscopy (Zeiss), and two images thereof were superimposed on each other, while assigning a red pseudocolor and a green pseudocolor to fluorescence generated by KO and fluorescence generated by EGFP, respectively. The image of LLCMK₂ (SeVdp/KO/HygB/EGFP/Luc2) cells became yellow which indicates that KO and EGPF are simultaneously expressed, whereas the image of the LLCMK₂ (SeVdp/Zeo/KO/CLuc + SeVdp/Bsr/EGFP/gp91phox) cells indicated a mixture of red/yellow/green- colored cells, which shows that a balance between the expression of KO and EGFP is significantly different in each cell (FIG 6A).

In order to quantitatively analyze the balance between the expressions of KO and EGFP, the above cells were analyzed by a Fluorescent-activated Cell Analyzer (BD FACSCalibur, Becton, Dickinson and Company). 10⁴ LLCMK₂ (SeVdp/KO/HygB/ EGFP/Luc2) cells and 10⁴ LLCMK₂ (SeVdp/Zeo/KO/CLuc + SeVdp/Bsr/ EGFP/gp91phox) cells were suspended in 2 mL of buffer to measure the fluorescence intensity (FL1) of EGFP and a fluorescence intensity (FL2) of KO. The analysis shows that the ratio between the fluorescence intensities of EGFP and KO in the LLCMK₂ (SeVdp/KO/HygB/EGFP/Luc2) is constant, whereas the ratio in the LLCMK₂ (SeVdp/Zeo/KO/CLuc + SeVdp/Bsr /EGFP/gp91phox) cells fluctuates significantly (FIG 6B). In an analysis of the ratio between FL1 and FL2, 50% or more of the LLCMK₂ (SeVdp/KO/HygB/EGFP/Luc2) cells had the same ratio, whereas the ratio in the LLCMK₂ (SeVdp/Zeo/KO/CLuc + SeVdp/Bsr /EGFP/gp91phox) was widely distributed in a broad range from 0 to 100% (FIG 6C).

The above results show that the function of simultaneously introducing two or more types of genes into each cell to induce gene expression at the same ratio can be achieved by the process of cloning the four types of reprogramming genes on a single common vector, as shown in the Examples of the present invention, but cannot be readily achieved when each of the four types of reprogramming genes are cloned into individual vectors that are mixed together prior to infection as disclosed in Patent Document 1 and Non-Patent Documents 12 and 21.

### EXAMPLE 8: Induction of Cells Expressing Human iPS Marker from Human Embryo-Derived Fibroblast Cells

### (1) Induction of Human iPS Marker-Expressing Cells

TIG3 cells, *i.e.* human embryo-derived fibroblast cells, were cultured in a 12-well plate at a density of 10 × 10⁵ cells/well. After one day, each of the hOct4/hSox2/hKlf4/hc-Myc sustained expression-inducing Sendai virus (prepared in Example 2) and the hOct4/hSox2/hKlf4/hc-Myc sustained expression-inducing Sendai virus Version 2 (prepared in Example 3) were added to the culture medium, and left at room temperature for 2 hours. The cells were then cultured in the presence of the recombinant Sendai viruses overnight at 37°C. The virus-infected cells were then plated on a feeder layer of mitomycin-treated MEFs and cultured in hES medium (DMEM/F12, 20% of Knockout Serum Replacement (KSR), 0.1 mM nonessential amino acids, 0.55 mM 2-ME, 10 ng/ml bFGF) or a primate ES cell culture medium (ReproCELL).

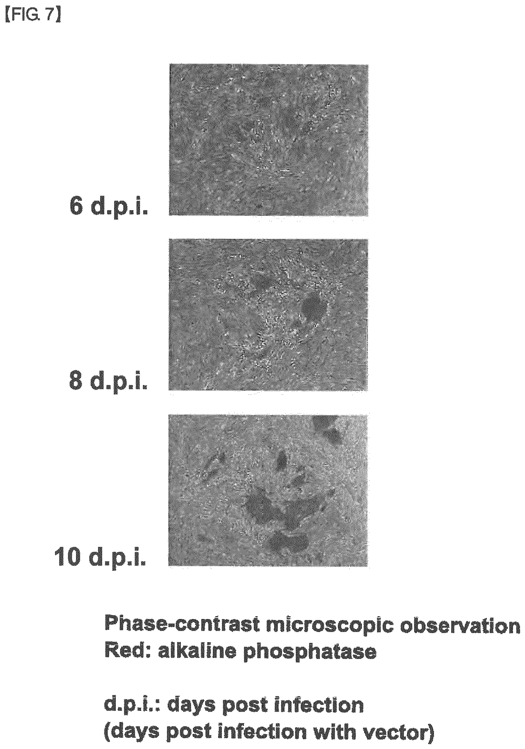

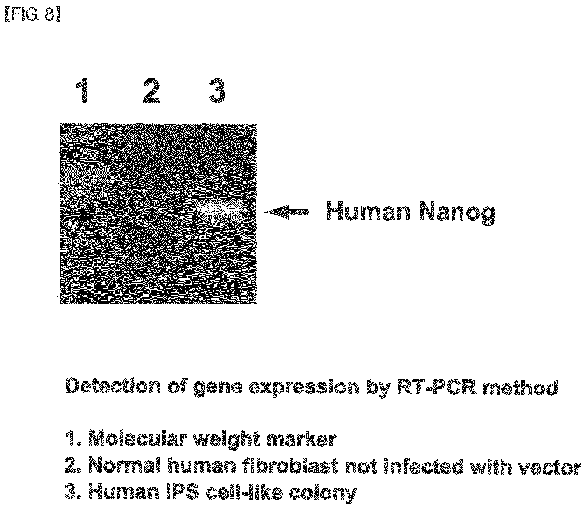

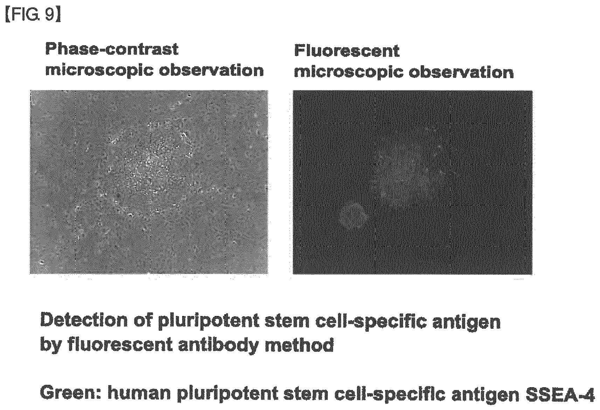

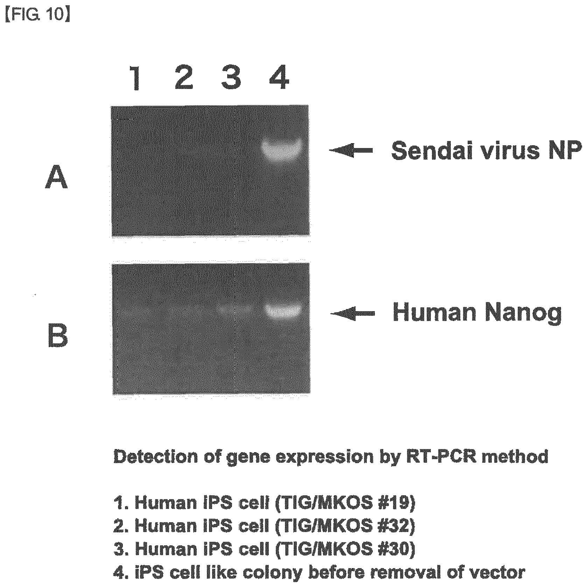

As shown in FIG 7, 10 days after the start of infection with the hOct4/hSox2/hKlf4/hc-Myc sustained expression-inducing Sendai virus, human ES-like cell colonies formed that expressed alkaline phosphatase (see (b) described hereinsfter). RT-PCR anlaysis (see (c) described hereinafter), confirmed that human Nanog expression is induced in human iPS cells that are capable of forming colonies (see FIG. 8). Using a fluorescent antibody method (see (a) described hereinafter), these colonies were also shown to express SSEA-4 antigen, a marker characteristic of embryonic stem cells and iPS cells (FIG. 9). Substantially the same results were obtained using the hOct4/hSox2/ hKlf4/hc-Myc sustained expression-inducing Sendai virus Version 2.

### (2) Test for Induction Efficiency of Human iPS Marker-Expressing Cells

A fluorescent antibody method was used to determine the amount of NP protein present in cells infected with the sustained expression-type Sendai virus. The amount of NP protein correlates with the infection rate of the recombinant Sendai virus. The number of cells in an alkaline phosphatase activity-positive colony was then corrected for the rate of infection which allowed the calculation of the induction efficiency of human iPS marker-expressing cells. The results are shown in Table 1.

**TABLE 1: Frequency of alkaline phosphatase-expressing cells in human embryo fibroblast cells infected with the hOct4, hSox2, hKlf4, and hc-Myc sustained expression-inducing Sendai virus Version 1 as a function of time after the initiation of infection.**

| Time (days) after infection | Frequency with respect to all cells (%) | Frequency with respect to infected cells (%) |
|---|---|---|
| 6 | 2.7 | 10.2 |
| 8 | 3.7 | 13.9 |
| 10 | 4.4 | 16.8 |

As can be seen from the results in Table 1, it became evident that the cells expressing human iPS markers can be induced with significantly higher efficiency than previous reports of iPS cell generation in which hOct4, hSox2, hKlf4 and hc-Myc were introduced into the host cell using a retroviral vector or by using other vector systems. Substantially the same results were obtained after infection with hOct4/ hSox2/hKlf4/hc-Myc sustained expression-inducing Sendai virus Version 2.

### EXAMPLE 9: Production of Human iPS Cells by Removing the RNA Genome of Sustained Expression-Type Sendai Viral vector

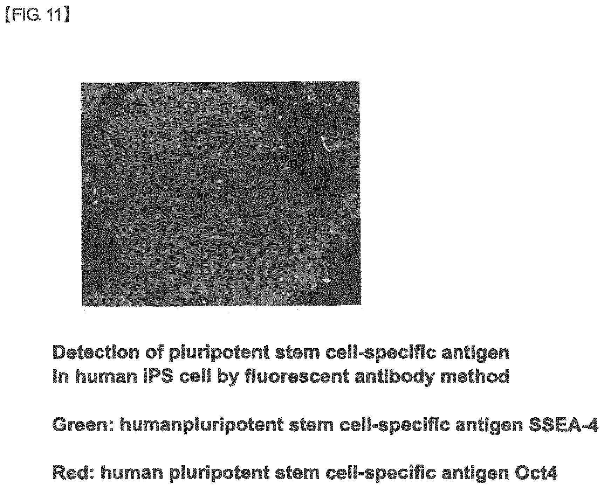

The human iPS marker-expressing cells obtained in Example 8 were treated with siRNA for targeting the L gene as described in Example 6 and successfully cultured for long periods of time. One month after infection with the vector, fluorescent antibody staining of Sendai virus NP protein confirmed that no vector RNA genome remained in the cells as described in (a) hereinafter. RT-PCR analysis of NP gene expression as described in (c) hereinafter further confirmed the absence of viral vector sequences in these cells (FIG. 10A). In addition, RT-PCR analysis of Nanog mRNA (see (c) described hereinafter), demonstrated that endogenous Nanog expression persists in iPS cells even after the cells no longer contain any detectable viral vector sequences (FIG. 10B). The same is true for the stem cell markers human SSEA-4 and Oct-4 (FIG. 11) detected using appropriate fluorescent antibodies (see (a) described hereinafter) further indicating that these endogenous stem cell markers do not require the persistent expression of the reprogramming genes in order to maintain the stem cell phenotype.

### EXAMPLE 10: Formation of Teratoma from Human iPS Cells

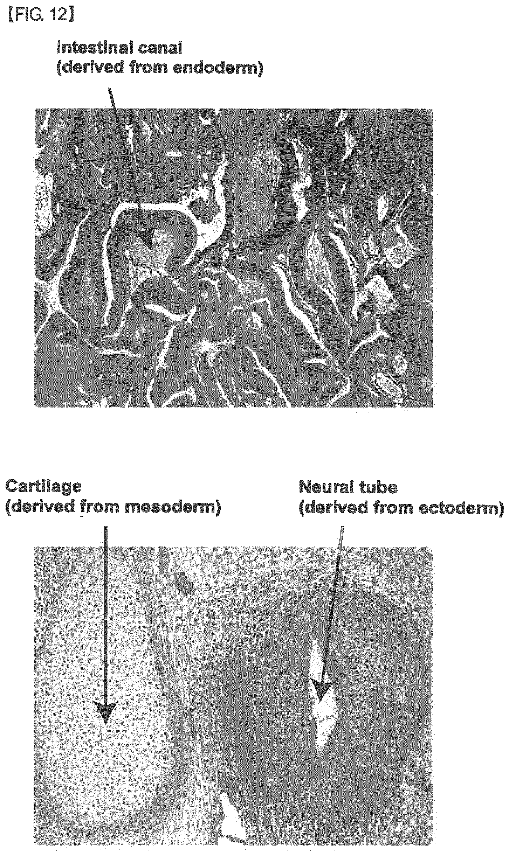

The human iPS cells obtained in Example 9 were adjusted to a concentration of 1.0 × 10⁶ cells/40 µL Hepes Buffered Saline Solution (HBSS)/mouse. A testis of a mouse (C.B17/Icr-scidJcl) anesthesized with Nembutal and isoflurane, was inoculated with iPS cells. After about 8 weeks, a visually identifiable teratoma formed. After 60 days post-inoculation, the teratoma was excised and fixed in Bouin's fixative solution (75% of saturated picric acid, 12% of formalin, 3% of acetic acid), and dehydrated by a treatment with 70% ethanol solution (1 hour), 90% ethanol solution (1 hour), 100% ethanol solution (1 hour, twice), 50% ethanol solution, 50% 2-butanol solution (1 hour) and 100% 2-butanol solution (30 minutes, twice). The specimen was then fixed in paraffin. Sections of 6 µm thickness were then prepared using a microtome, the section were deparaffinized, and subjected to HE staining. Differentiation to all of three germ layers was observed within each teratocarcinoma analyzed (FIG 12).

### Example 11: Induction of iPS cells using Sustained Expression-Type Sendai Viral vectors each loaded with a Reprogramming Gene

iPS cell production efficiency with Sendai vector comprising all four types of reprogramming genes on a single common vector to produce iPS cells, as shown in the Examples of the present invention, was then compared to iPS induction by infection with Sendai virus containing only one of the reprogramming genes, as disclosed in Patent Document 1 and Non-Patent Documents 12 and 21. The hOct4/hSox2/hKlf4/hc-Myc sustained expression-inducing Sendai viral vector Version 1 containing all four types of reprogramming genes was compared with the hOct4/hSox2/hKlf4 sustained expression-inducing Sendai viral vector comprising three reprogramming genes (as shown in the Example 2), and a Zeo/KO/hc-Myc sustained expression-inducing Sendai viral vector containing just c-Myc. The Zeo/KO/hc-Myc sustained expression-inducing Sendai viral vector was prepared by substituting the Oct4 gene, the Sox2 gene and the Klf4 gene of the hOct4/hSox2/hKlf4 sustained expression-inducing Sendai viral vector prepared in Example 3 with the Zeo gene, KO gene and c-Myc gene, respectively.

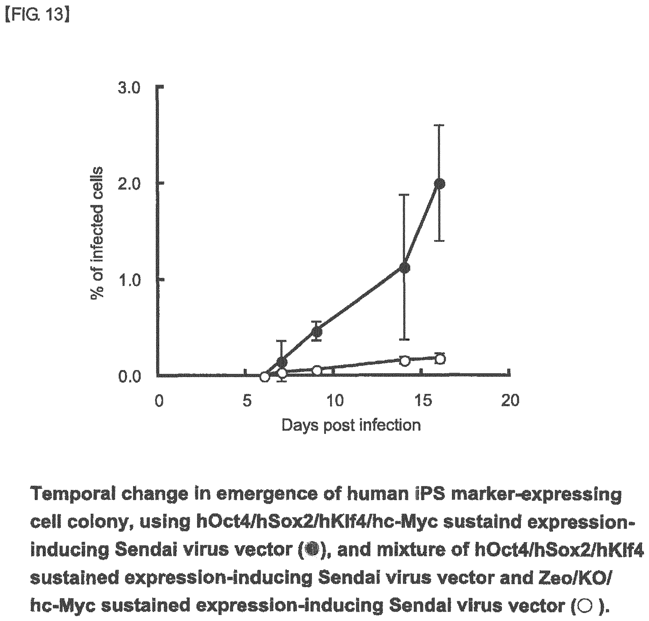

According to the Example 8, cells were infected with the hOct4/hSox2/hKlf4/hc-Myc sustained expression-inducing Sendai virus or a mixture of hOct4/hSox2/hKlf4 sustained expression-inducing Sendai virus with the Zeo/KO/hc-Myc sustained expression-inducing Sendai virus at a vector particle ratio of 1 : 1. Emergence of iPS cell colonies was checked by an index based on emergence of an alkaline phosphatase-positive cell colony. As a result, it could be shown that cloning the four types of reprogramming genes on the single common vector produces iPS cells with a cell production efficiency far greater than that in the process in which each of the four types of reprogramming genes is loaded on a respective one of two vectors, separately (FIG. 13).

### Example 12: Induction of iPS Cells using hOct4/hSox2/hKlf4/hc-Myc Sustained Expression-Inducing Sendai virus Version 3

TIG3 cells was seeded on a 12-well plate at a density of 1.0 × 10⁵ cells/well. On the next day, the hOct4/hSox2/hKlf4/hc-Myc sustained expression-inducing Sendai viral vector prepared in Example 3, or the hOct4/hSox2/hKlf4/hc-Myc sustained expression-inducing Sendai viral vector Version 3 prepared in Example 4, was added to the medium to induce human iPS cells according to Example 8.

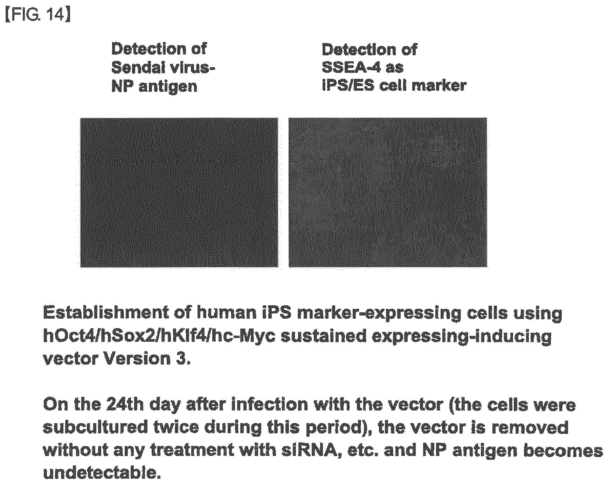

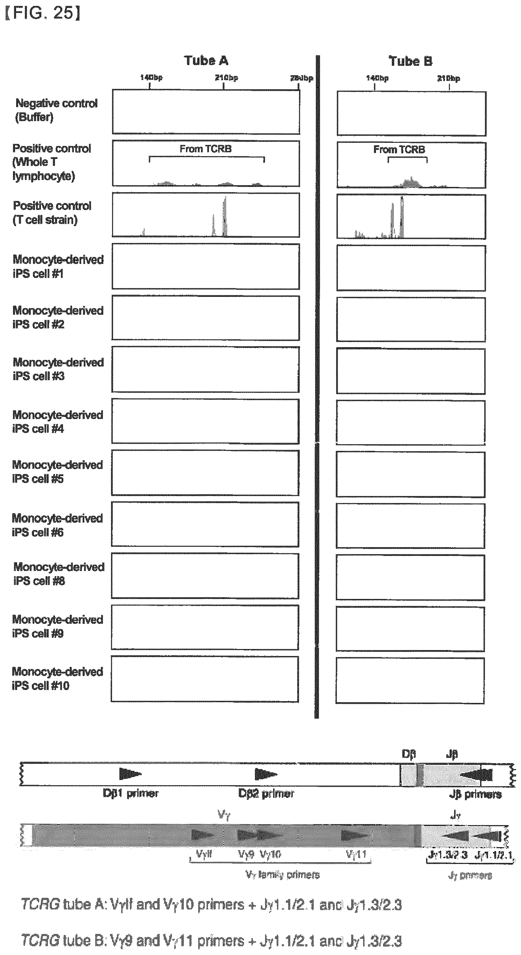

Colonies was subcultured twice. Then, on the 24th day after infection, colonies were fluorescently stained using an antibody against NP protein As shown in FIG 25A, colonies induced with hOct4/hSox2/hKlf4/hc-Myc sustained expression-inducing Sendai viral vector Version 3 did not contain any vector (FIG 14) whereas expressed iPS/ES marker SSEA-4 antigen.

The above result clearly shows that, when human iPS cells are induced with the hOct4/hSox2/hKlf4/hc-Myc sustained expression-inducing Sendai virus Version 3, the vector is automatically removed by the microRNA, mir-302a, expressed in the induced iPS cell. The hOct4/hSox2/hKlf4/hc-Myc sustained expression-inducing Sendai virus Version 4 exhibited the same effect.

### Example 13: Establishment of iPS cells from Human Peripheral-Blood Mononuclear Cells

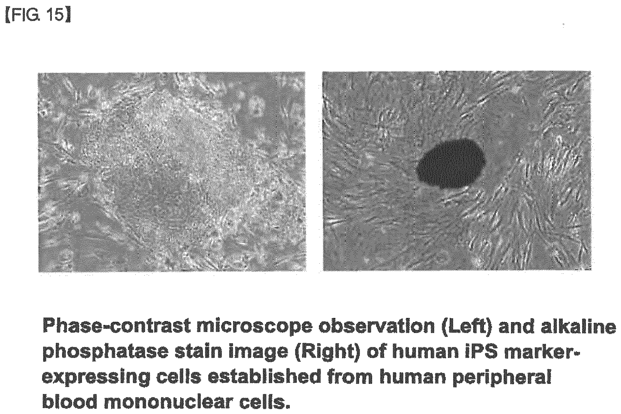

20 mL of adult blood was diluted with 20 mL of PBS (-), and layered on 6 mL of Lymphoprep. The blood was then centrifuged at 1.800 r.p.m. for 30 minutes to separate an upper layer of platelets, an intermediate layer including mononuclear cells and a lower layer including red blood cells. The intermediate layer was washed with PBS (-) to obtain human peripheral-blood mononuclear cells. In accordance with the technique described in Example 8, the cells were infected with hOct4/hSox2/hKlf4/hc-Myc sustained expression-inducing Sendai virus, and then cultured. iPS cells positive for alkaline phosphatase and having a morphology similar to that of a human ES cell formed (FIG. 15). Cell colonies were not detected in a negative control comprising cells that were not infected with the Sendai vector.

### EXAMPLE 14: Purification of Monocytes from Human Peripheral Blood

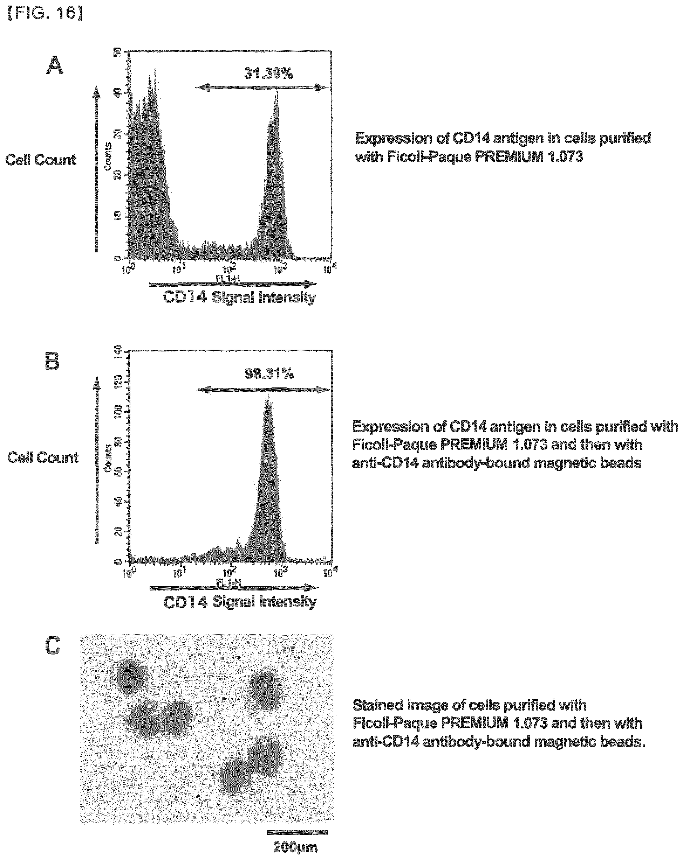

Peripheral blood (38 mL) from an adult (age 54, male) was diluted with PBS(-) (42 mL) to make a total volume of 80 mL. 8 mL of the diluted peripheral blood was layered over 7 mL of Ficoli-Paque PREMIUM 1.073 (GE Healthcare) and centrifuged at 1800 r.p.m. for 30 minutes. Mononuclear cells comprising monocytes were recovered from the intermediate layer between Ficoll layer and the upper layer. To 2.5 mL of this fraction, 12 mL of PBS(-), 2% fetal bovine serum and 1 mM EDTA were added. The resultant mixture was centrifuged at 1000 r.p.m. for 10 minutes to remove platelets, and mononuclear cells were recovered as pellet. Further, CD 14 (monocyte specific antigen) positive cells were purified from the resultant mononuclear cells magnetically using anti-CD14 antibody-bound magnetic beads (Miltenyi Biotec). The thus purified cells were stained with anti-CD14-FITC (DAKO) and subjected to flowcytometry to assay their purity. The purity after purification with Ficoli-Paque was 31% (FIG 16A). The purity increased to 98% or more after further purification with anti-CD14 antibody-bound magnetic beads (FIG. 16B). When the finally purified cells were observed with Wright's stain, almost all of the cells retained the typical monocytic shape (FIG. 16C). Through the above-described tests, a total of 6 x 10⁶ monocytes with 98% or more purity were recovered.

### EXAMPLE 15: Induction of iPS Marker-Expressing Cells from Human Peripheral Blood-Derived Monocytes

### (1) Induction of Human iPS Marker-Expressing Cells

To 3 x 10⁵ human monocytes isolated in Example 14, the hOct4/hSox2/hKlf4/hc-Myc sustained expression-inducing Sendai viral vector (Version 1, 2, 3 or 4) prepared in Example 2, 3, 4 or 5 was added to give a total volume of 200 µL and allowed to infect at room temperature for 2 hours. As a comparison control, a sustained expression-inducing Sendai viral vector not loaded with reprogramming genes was used. After the infection, 500 µL of medium (RPMI 1640, 10% bovine serum) was added thereto, followed by low speed centrifugation to remove the vector. The infected monocytes were suspended in a medium for human ES cells (ReproCELL) and seeded at 1 x 10⁵ cells/well/500 µL on 12-well plates in which feeder cells (mouse embryo-derived fibroblast cells pre-treated with mitomycin C) had been cultured at a density of 1.8 x 10⁵ cells/well. Then, the cells were cultured at 37°C under 5% CO₂ gas. Culture medium was exchanged every other day.

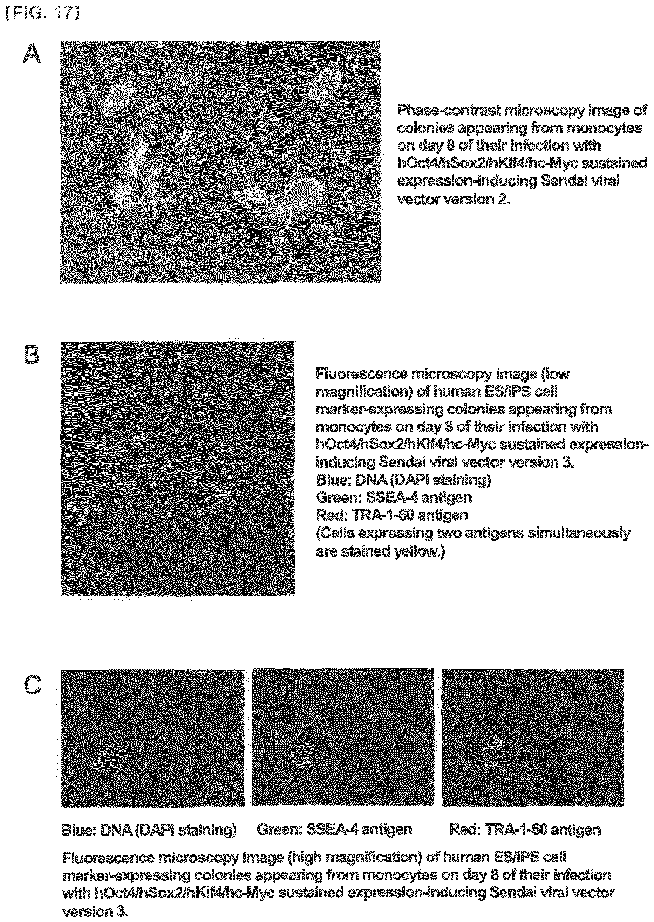

In human monocytes infected with the hOct4/hSox2/hKlf4/hc-Myc sustained expression-inducing Sendai viral vector, colonies of aggregating and growing cells were observed from day 5 to day 8 of culture (FIG 17A). Such cell clusters did not appear in monocytes infected with the comparison control (i.e., sustained expression-inducing Sendai viral vector not loaded with reprogramming genes). Cells constituting these cell clusters were expressing SSEA-4 antigen and TRA-1-60 antigen, both being human iPS cell markers (FIGS. 17B and 17C).

### (2) Assay of Efficiency of Colony Induction from Human Peripheral Blood-Derived Monocytes

On day 8 of infection with the hOct4/hSox2/hKlf4/hc-Myc sustained expression-inducing Sendai viral vector (Version 1, 2, 3 or 4), the number of colonies which had the shape shown in FIG 17A were measured and divided by the number of cells seeded (1 x 10⁵) to thereby calculate the colony induction efficiency. The results are shown in Table 2.

**TABLE 2: Observation of Colony Appearance Frequency in Human Peripheral Blood-Derived Monocytes Infected with hOct4/hSox2/hKlf4/hc-Myc Sustained Expression-Inducing Sendai Viral Vector (Version 1, 2, 3 or 4)**

| Type of hOct4/hSox2/hKlf4/hc-Myc sustained expression-inducing Sendai viral vector | Number of colonies | Colony induction efficiency (%) |
|---|---|---|
| Version 1 | 381 | 0.381 |
| Version 2.1 | 342 | 0.342 |
| Version 3 | 636 | 0.636 |
| Version 4 | 85 | 0.085 |

From the results shown in Table 2, it was believed that approximately 0.1 to 0.6% of the cells (with some difference depending on the vector used) formed colonies which may potentially become iPS cells in the future.

### EXAMPLE 16: Preparation of Human iPS Cells by Removal of the RNA Genome of Sustained Expression-Inducing Sendai Viral Vector

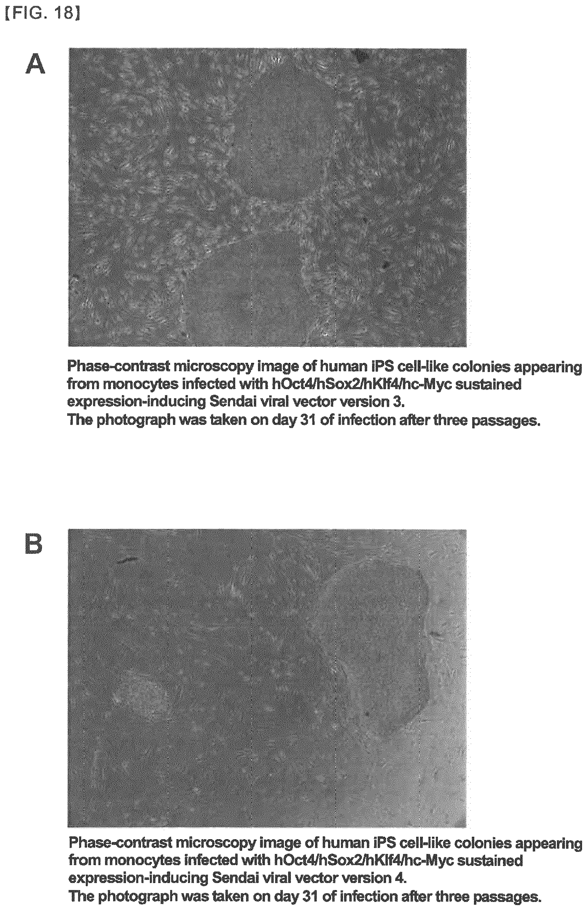

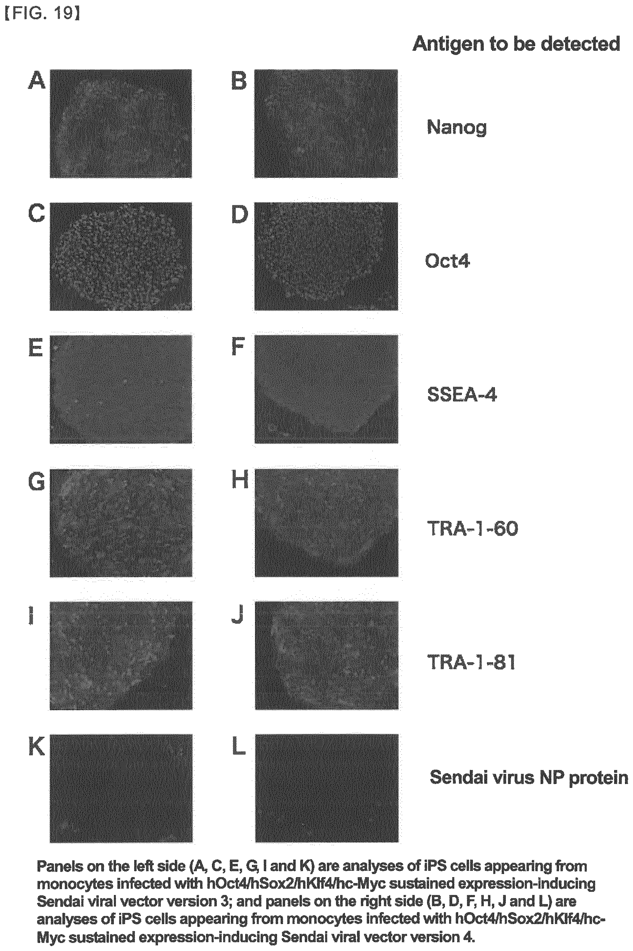

The human iPS marker-expressing cells that appeared in Example 15 were dissociated with trypsin and subcultured under the same culture conditions. At the same time, anti-L gene siRNA was added to the medium 3 times in total in the same manner as shown in Example 6 to thereby remove the Sendai viral vector. On day 15 of infection, the cells were subcultured using a dissociation solution for human ES cells and subjected to an additional two treatments with siRNA. On day 31 of infection, colonies with a flat shape similar to the shape of typical human ES/iPS cells appeared (FIGS. 18A and 18B). These colonies expressed Nanog (FIGS. 19A and 19B), Oct4 (FIGS. 19C and 19D), SSEA-4 antigen (FIGS. 19E and 19F), TRA-1-60 antigen (FIGS. 19G and 19H) and TRA-1-81 antigen (FIGS. 19I and 19J), all being human iPS cell markers, but they did not express the NP antigen of Sendai virus (FIGS. 19K and 19L). Thus, these colonies were confirmed to be Sendai viral vector-free human iPS cells.

### EXAMPLE 17: Rearrangement Analysis of T Cell Receptor Gene in Human Monocyte-Derived iPS Cells

In the preparation of human peripheral blood monocyte-derived iPS cells described in Examples 14 to 16, the monocytes used for the preparation had a purity of 98% or more, suggesting an extremely high possiblility that the iPS cells shown in Example 16 were derived from monocytes. However, mononuclear cells before purification with anti-CD 14 antibody bound magnetic beads contain lymphocytes (T cells and B cells). In particular, it is already known that iPS cells can be prepared from T cells (Loh, et al, Cell Stem Cell, 7, 15-19, 2010; Staerk, et al., Cell Stem Cell, 7, 20-24, 2010; Seki, et al., Cell Stem Cell, 7, 11-14, 2010), so it was examined whether the iPS cells shown in Example 16 was derived from T cells or B cells that the cell material used contained at a probability of 2% or less.

First, it is known that human peripheral blood B cells is not capable of being infected with Sendai virus (Nakanishi, et al., J. Cont. Rel., 54, 61-68, 1998) and this denies the possibility that the iPS cells shown in Example 16 was derived from B cells. In order to examine whether the iPS cells shown in Example 16 were derived from T cells, rearrangement of T cell receptor gene was investigated. This technique has been established in the clinical field as a method to diagnose whether a leukemia cell of interest is derived from T cells.

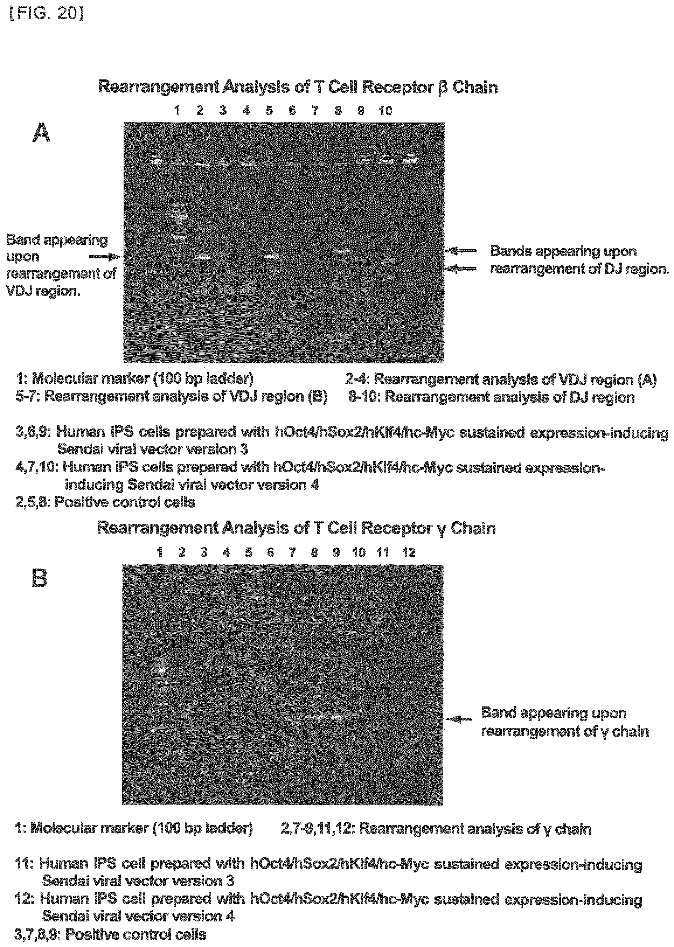

The genome DNA of human iPS cells was purified with DNeasy Blood & Tissue kit (QIAGEN), and 40 ng (FIG. 20A) or 20 ng (FIG. 20B) of the purified DNA was used for the analysis. The rearrangement of T cell receptor β chain gene was analyzed by PCR according to the method described in van Dongen, et al., Leukemia, 17, 2257-2317, 2003. The rearrangement of T cell receptor γ chain gene was analyzed by PCR according to the method described in Benhattar, et al, Diagn. Mol. Pathol., 4, 108-112, 1995. The results are shown in FIG. 20.

When rearrangement has occurred in T cell receptor β chain gene, a distinct DNA band is detected at approximately 300 bp or 180 bp. In the genome DNA of the T cell-derived iPS cells used as a comparison control, rearrangement occurred and a distinct band was detected. On the other hand, no corresponding band was detected in the genome DNA of the two samples of iPS cells shown in Example 16 (FIG. 20A). Likewise, when rearrangement has occurred in T cell receptor γ chain gene, a distinct DNA band is detected at approximately 200 bp. In the genome DNA of the T cell-derived iPS cells used as a comparison control, rearrangement occurred and a distinct band was detected. On the other hand, no corresponding band was detected in the genome DNA of the two samples of iPS cells shown in Example 16 (FIG. 20B).

These results demonstrated that the iPS cells shown in Example 16 were not derived from either T cells or B cells, but derived from monocytes.

Ascertaining means used in Examples 8-16 are as follows:

### (a) Verification of Gene Expression by Indirect Fluorescent Antibody Method

Expressions of human Oct4, human Sox2, human Klf4, human c-Myc, mouse SSEA-1, human SSEA-4 and Sendai virus NP gene in each cell were verified using antibodies to each of the antigens. A primary antibody and a dilution rate used herein are as follows. The human Oct4: rabbit anti-Oct4 polyclonal antibody (Abcam Inc.) [× 100]; the human Sox2: rabbit anti-Sox2 polyclonal antibody (Abcam Inc.) [× 100]; the human Klf4: rabbit anti-Klf4 polyclonal antibody (CeMines Inc.) [× 100]; the human c-Myc: rabbit anti-c-myc polyclonal antibody (Santa Cruz Biotechnology Inc.) [× 100]; the SSEA-1: mouse anti-SSEA-1 monoclonal antibody (Santa Cruz Biotechnology Inc.) [× 200]; the SSEA-4: mouse anti-SSEA-4 monoclonal antibody (Santa Cruz Biotechnology Inc.) [× 200]; and the Sendai virus NP: mouse anti-NP monoclonal antibody [× 200] or rabbit anti-NP polyclonal antibody [× 1000].

### (b) Alkaline Phosphatase Staining

Culture medium was first removed, and the cells were washed with PBS. Then, Vector Red Alkaline Phosphatase Kit I (Vector Laboratories Inc.) was added to the cells, and left to react at room temperatures for 20 to 30 minutes. Cells having alkaline phosphatase activity stained red.

### (c) Verification of the Expression of Mouse Nanog, Mouse Oct4, Human Nanog and Sendai Virus NP Gene by Reverse Transcription-Polymerase Chain Reaction (RT-PCR) Method

Total RNA was extracted from iPS cells using ISOGEN (Nippon Gene Co. Ltd.). cDNA was synthesized using random primer according to instructions in the SuperScript III First strand synthesis system (Life technologies, Inc.). Target cDNA was then amplified by PCR using the following primers. The human Nanog: 5'-AGCATCCGACTGTAAA GAAT-3' (SEQ ID NO: 31 in the Sequence Listing (sense strand)), 5'-CCTCTCCACA GTTATAGAAG-3' (SEQ ID NO: 32 in the Sequence Listing (antisense strand)); SeV NP: 5'-AGACCCTAAGAGGACGAAGA-3' (SEQ ID NO: 33 in the Sequence Listing (sense strand)), 5'-ACTCCCATGGCGTAACTCCATAGTG-3' (SEQ ID NO: 34 in the Sequence Listing (antisense strand)).

### EXAMPLE 18: Formation of Teratoma from Human Peripheral Blood Monocyte-Derived iPS Cells

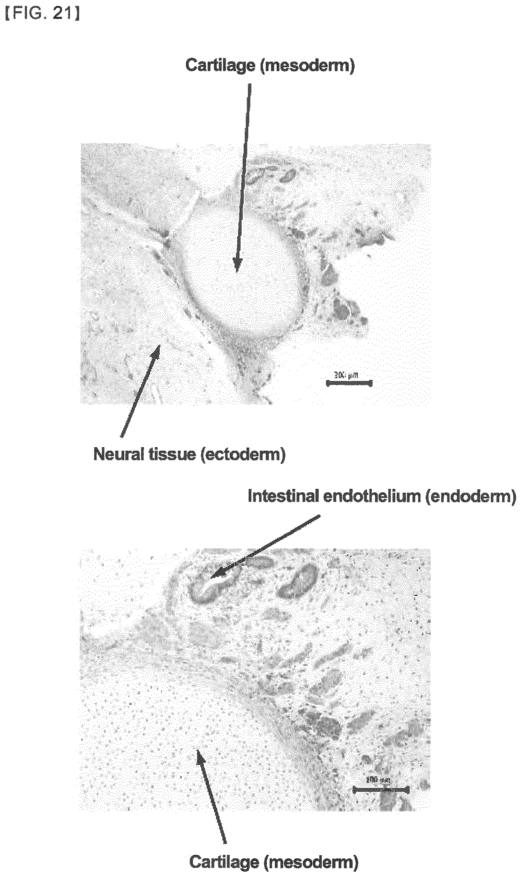

The human peripheral blood monocyte-derived iPS cells obtained in Example 16 were adjusted to a concentration of 1.0 x 10⁶ cells/40 µL Hepes Buffered Saline Solution (HBSS)/mouse. The testis of a mouse (C.B17/Icr-scidJcl) anesthetized with Nembutal and isoflurane was exposed, inoculated with the adjusted iPS cells, and sutured. About 8 weeks after the inoculation, a visually identifiable teratoma formed, and 60 days after inoculation, the teratoma was excised and fixed in Bouin's fixative solution (75% of saturated picric acid, 12% formalin, 3% acetic acid), and dehydrated by treating with 70% ethanol (1 hour), 90% ethanol (1 hour), 100% ethanol (1 hour, twice), 50% ethanol: 50% 2-butanol solution (1 hour) and 100% 2-butanol (30 minutes, twice). The specimen was fixed in paraffin and 6 µm-thick sections were then prepared using a microtome. The sections were deparaffinized and subjected to HE staining. As a result, differentiation to all of three germ layers was observed. Thus, that human peripheral blood monocyte-derived iPS cells were confirmed to have pluripotency (FIG. 21).

### EXAMPLE 19: Examination of the Capacity of Human Peripheral Blood Monocyte-Derived iPS Cells to Redifferentiate to Blood Cells

It is known that human iPS cells, often retaining the epigenetic characters of the somatic cell used for their preparation, tend to easily redifferentiate to cells of the same tissue as that of the original somatic cell. If human peripheral blood monocyte-derived iPS cells have a tendency to easily redifferentiate to hematopoietic progenitor cells with intact genome, such iPS cells are extremely useful in regenerative therapies, *in vitro* preparation of platelets, and so on. Then, using a system allowing ES cells to differentiate to blood cells *in vitro* (Takayama, et al., Blood, 111, 5298-5306 (2008)), human peripheral blood monocyte-derived iPS cells and human fibroblast cell-derived iPS cells were compared with respect to their tendency to differentiate to blood cells.

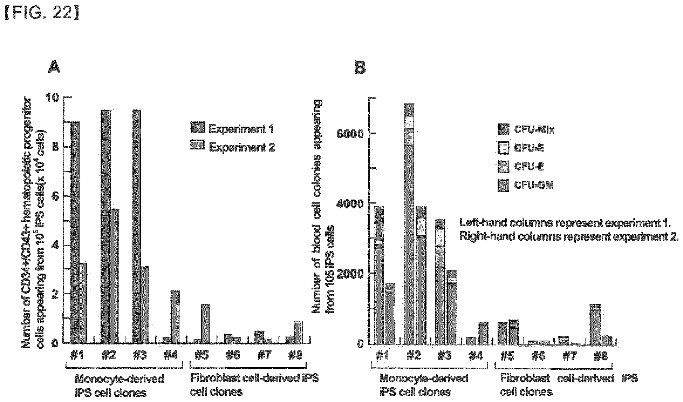

The human peripheral blood monocyte-derived iPS cells obtained in Example 16 and the human fibroblast cell-derived iPS cells obtained in Example 9 were allowed to redifferentiate to blood cells according to the method described in Takayama, et al., Blood, 111, 5298-5306 (2008). Briefly, iPS cells prepared in small clusters of about 100 cells were overlayered on mouse mesenchymal stem cell strain C3H10T1/2 (obtained from RIKEN BioResource Center) (in a 100 mm dish) that had been irradiated with γ rays (50 Gy) immediately before use to be deprived of proliferative capacity. The cells were cultured in a differentiation medium (Iscove modified DMEM, 10 µg/mL human insulin, 5.5 µg/mL human transferring, 5 ng/mL sodium selenite, 2 mM L-glutamine, 0.45 mM monothioglycerol, 50 µg/mL ascorbic acid, 15% FCS, VEGF 20 ng/mL) for two weeks. After two weeks, bag-like structures called iPS-sac were isolated, and the number of CD34/CD43 positive (hematopoietic progenitor cell marker positive) cells contained therein was counted by flowcytometry (FIG. 22A). The results are shown in terms of the number of CD34/CD43 positive cells appearing per 10⁵ human iPS cells.

Of the four human peripheral blood monocyte-derived iPS cell clones, three clones showed a significantly higher capacity of differentiation to hematopoietic progenitor cells than human fibroblast cell-derived iPS cells and one clone showed a comparable differentiation capacity to that of human fibroblast cell-derived iPS cells. These results revealed that human peripheral blood monocyte-derived iPS cells redifferentiated to human hematopoietic progenitor cells more easily than human fibroblast cell-derived iPS cells, although both types of iPS cells were similarly prepared using the hOct4/hSox2/hKlf4/hc-Myc sustained expression-inducing Sendai viral vector.

Further, 2 x 10⁴ cells contained in the iPS-sac were re-seeded on γ ray-irradiated C3H10T1/2 cells (in a 6-well plate) and cultured in a differentiation medium containing cytokine cocktail (containing human IL-6, IL-11 and SCF; Pharmacia & Upjohn) for 3 weeks. The medium was exchanged once every 3 days. After 3 weeks, colonies of blood cells appearing therein were identified by their shapes, and the numbers of those colonies were quantitatively determined (FIG. 22B). The results are shown in terms of the numbers of colony forming unit-granulocyte/macrophage (CFU-GM), colony forming unit-erythroid (CFU-E), burst forming unit-erythroid (BFU-E) and colony forming unit-mix (CFU-Mix) appearing per 10⁵ human iPS cells, and the total of their numbers. Of the four human peripheral blood monocyte-derived iPS cell clones, three had a higher activity for producing differentiated blood cell colonies than human fibroblast cell-derived iPS cells and one clone showed a comparable activity to that of human fibroblast cell-derived iPS cells. From these results, it was confirmed that human hematopoietic progenitor cells derived from human peripheral blood monocyte-derived iPS cells have a normal capacity to differentiate to macrophages, granulocytes and erythroids.

### EXAMPLE 20: Gene Expression in Human Peripheral Blood Monocyte-Derived iPS Cells Established with hOct4/hSox2/hKlf4/hc-Myc Sustained Expression-Inducing Sendai Viral Vector as Compared with Gene Expression in Human Fibroblast Cell-Derived iPS Cells and Human ES Cells

### (1) Preparation of RNA Samples for Analysis

The human peripheral blood monocyte-derived iPS cells obtained in Example 16 and the human fibroblast cell-derived iPS cells obtained in Example 9 were cultured in MEF conditioned medium on Matrigel (Becton, Dickinson and Company) without using feeder cells, and each type of cells was recovered in an amount of 1.0 x 10⁶ cells. From the thus recovered cells, total cellular RNA was extracted with ISOGEN (Nippon Gene Co., Ltd.). As comparison controls, human normal fibroblast cells, human ES cells and standard human iPS cell strain 201B7 (provided by Dr. Shinya Yamanaka, Kyoto University; as prepared by introducing hOct4, hSox2, hK1f4 and hc-Myc genes with a retrovirus vector) were cultured and RNA extracted in the same manner.

### (2) Analysis of Gene Expression

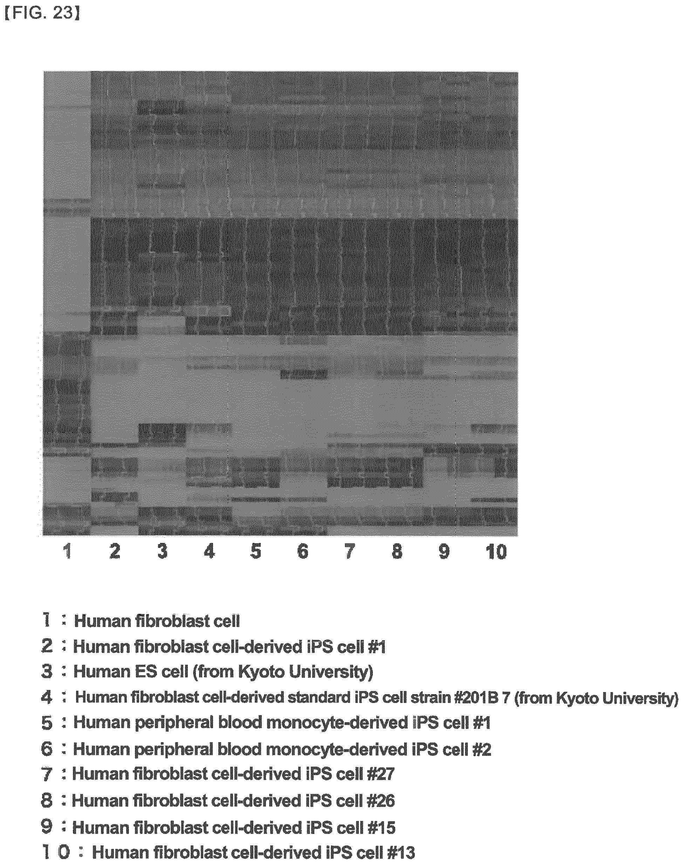

Total cellular RNA (0.5 µg) was labeled with Cy3 using Quick Amp Labeling Kit (Agilent Technologies, Inc.). The labeled RNA was hybridized to Whole Human Genome (4x44k) DNA array (Agilent Technologies, Inc.) using Gene Expression Hybridization Kit (Agilent Technologies, Inc.), and signals were obtained with an Agilent DNA microarray scanner. The thus obtained signals were analyzed with GeneSpringGX10 software (Agilent Technologies, Inc.), and gene expressions in individual cell clones were analyzed by a display method called Heat Map in which the intensity of gene expression is indicated by a gradient from red to green (FIG. 23). As a result, human peripheral blood monocyte-derived iPS cells established with the hOct4/hSox2/hKlf4/hc-Myc sustained expression-inducing Sendai viral vector showed a gene expression pattern almost comparable to the corresponding patterns for human fibroblast cell-derived iPS cells prepared in the same manner, standard human iPS cell strain 201B7, and human ES cells.

### EXAMPLE 21: Analysis of Rearrangement of T Cell Receptor Gene in the Genome DNA of Human Peripheral Blood Monocyte-Derived iPS Cells Established with hOct4/hSox2/hKlf4/hc-Myc Sustained Expression-Inducing Sendai Viral Vector

In order to confirm that the human peripheral blood monocyte-derived iPS cells obtained in Example 16 was not derived from T lymphocytes present in a small amount in the monocyte fraction used for their preparation, rearrangement of T cell receptor gene was examined. In differentiated T cells, rearrangement of T cell receptor gene has necessarily occurred and such T cells have either T cell receptor consisting of α/β chains or T cell receptor consisting of γ/δ chains. In T cells having T cell receptor consisting of α/β chains, rearrangement of β chain gene is necessarily the first to occur. Therefore, this gene was analyzed. In T cells having T cell receptor consisting of γ/δ chains, both γchain gene and δ chain gene have been rearranged. Therefore, both genes were analyzed.

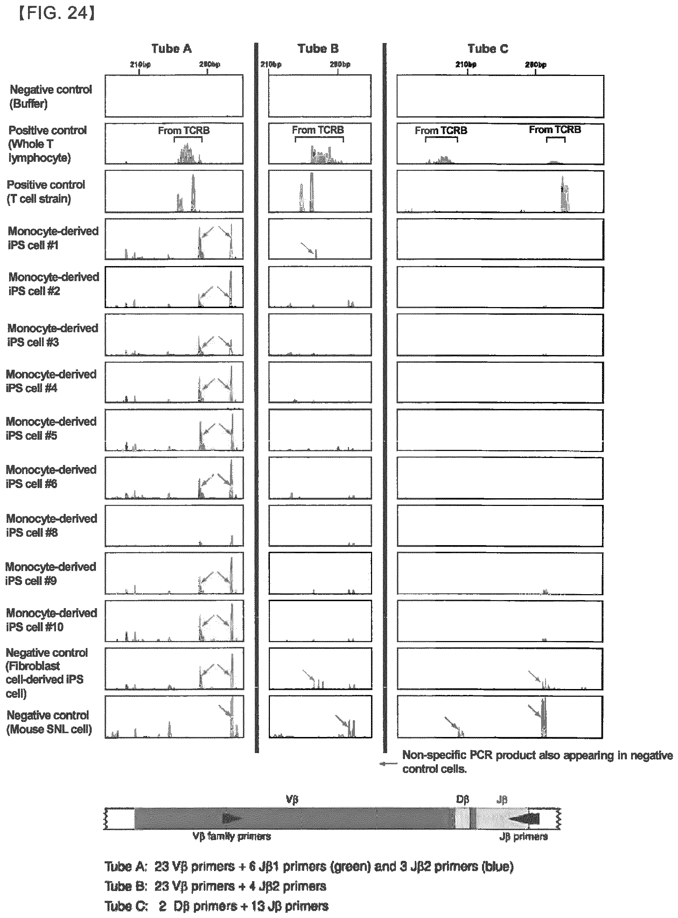

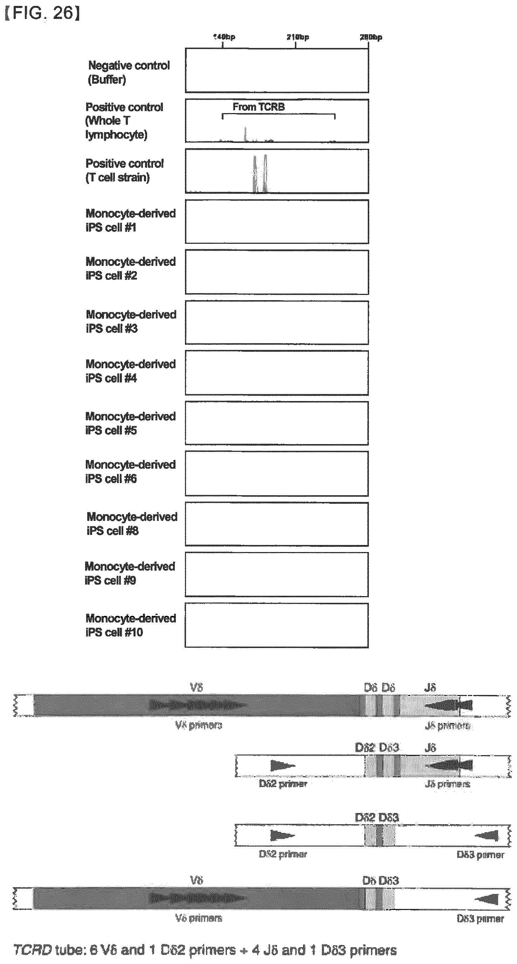

The genome DNA of human iPS cells was purified with DNeasy Blood & Tissue kit (QIAGEN), and 0.5 µg of the purified DNA was used for analysis. As positive controls, the peripheral blood whole T cell-derived genome DNA attached to the analysis kit and the genome DNA derived from a cell strain of T cells in which occurrence of rearrangement of each T cell receptor gene had been confirmed were used. As negative controls, the genome DNA of fibroblast-derived human iPS cells and the genome DNA of mouse feeder cells were used. Rearrangement of β chain gene and γ chain gene was detected with TCRB+TCRG T-Cell Clonality Assay for ABI Fluorescence Detection (InvivoScribe Technologies); and rearrangement of δ chain gene was detected with TCRD Clonality Assay for ABI Fluorescence Detection (InvivoScribe Technologies). The sizes of PCR products were analyzed with 3130 Genetic Analyzer (Applied Biosystem). In any of the genome DNAs, a specific PCR product can be detected if rearrangement has occurred in T cell receptor gene contained in the genome DNA. The results of rearrangement of β chain gene are shown in FIG. 24; the results of rearrangement of γ chain gene are shown in FIG. 25; and the results of rearrangement of δ chain gene are shown in FIG. 26. In any of the above results, no PCR product specific to rearrangement of T cell receptor gene was recognized in the genome DNA of human peripheral blood monocyte-derived iPS cells. Thus, it was confirmed that these iPS cells were not derived from T lymphocytes present in a small amount in the monocyte fraction used for their preparation.

### EXAMPLE 22: Analysis of Rearrangement of Immunoglobulin Heavy Chain Gene in the Genome DNA of Human Peripheral Blood Monocyte-Derived iPS Cells Established with hOct4/hSox2/hKlf4/hc-Myc Sustained Expression-Inducing Sendai Viral Vector

In order to confirm that the human peripheral blood monocyte-derived iPS cells obtained in Example 16 was not derived from B lymphocytes present in a small amount in the monocyte fraction used for their preparation, the characteristic occurrence of examination was performed to check for rearrangement of immunoglobulin heavy chain gene in differentiated B cells.

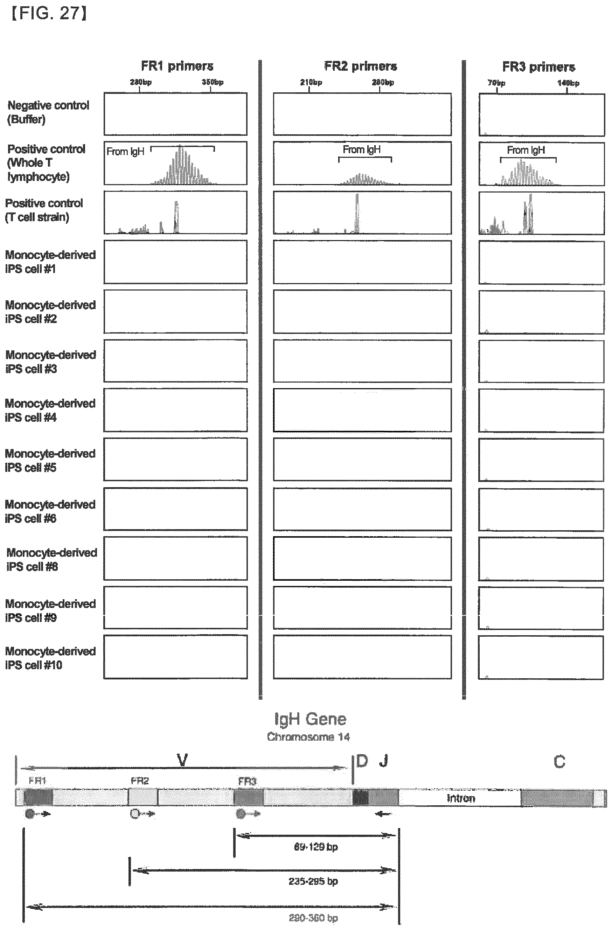

The genome DNA of human iPS cells was purified with DNeasy Blood & Tissue kit (QIAGEN), and 0.5 µg of the purified DNA was used for analysis. As positive controls, the peripheral blood whole B cell-derived genome DNA attached to the analysis kit and the genome DNA derived from a cell strain of B cells in which occurrence of rearrangement had been confirmed were used. As negative controls, the genome DNA of fibroblast-derived human iPS cells and the genome DNA of mouse feeder cells were used. Rearrangement of immunoglobulin heavy chain gene was detected with IGH Gene Rearrangement Assay for ABI Fluorescence Detection (InvivoScribe Technologies). The sizes of PCR products were analyzed with 3130 Genetic Analyzer (Applied Biosystem). In any of the genome DNAs, a specific PCR product can be detected when rearrangement has occurred in immunoglobulin heavy chain gene contained in the genome DNA. The results of analysis are shown in FIG. 27. No PCR product specific to rearrangement of immunoglobulin heavy chain gene was observed in any of the samples of the genome DNA of human peripheral blood monocyte-derived iPS cells. Thus, it was confirmed that these iPS cells were not derived from B lymphocytes present in the monocyte fraction used for their preparation.

### INDUSTRIAL APPLICABILITY

The present invention is useful for regenerative therapy and drug development technologies.

## Claims

1. A method of producing an induced pluripotent stem cell, comprising the steps of infecting a peripheral blood-derived mononuclear cell with a reprogramming gene-loaded Sendai virus vector to reprogram the mononuclear cell; and then artificially removing the reprogramming gene-loaded Sendai virus vector from the reprogrammed cell, wherein the reprogramming gene-loaded Sendai virus vector has Sendai virus genes and a reprogramming gene(s) on the same genome, the Sendai virus genes comprising NP gene, P/C gene, M gene, F gene, HN gene and L gene, provided that M gene, F gene and HN gene are all lacking in their respective functions and that the L protein expressed by the L gene of the reprogramming gene-loaded Sendai virus vector has a valine as an amino acid residue at position 1618 that enables sustained gene expression, wherein the peripheral blood-derived mononuclear cell is a monocyte

2. The method of claim 1, wherein the reprogramming gene-loaded Sendai virus vector is removed from the reprogrammed cell by siRNA.

3. The method of claim 1, wherein the reprogramming gene-loaded Sendai virus vector comprises, on its genome, a target sequence for a microRNA expressed in a pluripotent stem cell.

4. The method of claim 1, wherein the peripheral blood-derived mononuclear cell is derived from human.

5. An induced pluripotent stem cell obtainable by the method of any one of claims 1-4.

## Patentansprüche

1. Verfahren zur Herstellung einer induzierten pluripotenten Stammzelle, umfassend die Schritte des Infizierens einer aus Peripherblut abgeleiteten mononukleären Zelle mit einem mit umprogrammierendem Gen beladenen Sendai-Virus-Vektor zum Umprogrammieren der mononukleären Zelle; und dann des künstlichen Entfernens des mit umprogrammierendem Gen beladenen Sendai-Virus-Vektors von der umprogrammierten Zelle, wobei der mit umprogrammierendem Gen beladenen Sendai-Virus-Vektor Sendai-Virus-Gene und ein reprogrammierendes Gen bzw. reprogrammierende Gene an demselben Genom aufweist, wobei die Sendai-Virus-Gene NP-Gen, P/C-Gen, M-Gen, F-Gen, HN-Gen und L-Gen umfassen, vorausgesetzt, dass dem M-Gen, F-Gen und HN-Gen allen ihre jeweiligen Funktionen fehlen und dass das L-Protein, das durch das L-Gen des mit umprogrammierendem Gen beladenen Sendai-Virus-Vektors exprimiert wird, ein Valin als Aminosäurerest an Position 1618 aufweist, das eine anhaltende Genexpression ermöglicht, wobei die aus Peripherblut abgeleitete mononukleäre Zelle ein Monozyt ist.

2. Verfahren nach Anspruch 1, wobei der mit umprogrammierendem Gen beladene Sendai-Virus-Vektor von der umprogrammierten Zelle durch siRNA entfernt wird.

3. Verfahren nach Anspruch 1, wobei der mit umprogrammierendem Gen beladene Sendai-Virus-Vektor an seinem Genom eine Targetsequenz für eine in einer a pluripotenten Stammzelle exprimierten Mikro-RNA umfasst.

4. Verfahren nach Anspruch 1, wobei die aus Peripherblut abgeleitete mononukleäre Zelle vom Menschen abgeleitet ist.

5. Induzierte pluripotente Stammzelle, die durch das Verfahren nach einem der Ansprüche 1-4 erhältlich ist.

## Revendications

1. Procédé de production d'une cellule souche pluripotente induite, comprenant les étapes d'infection d'une cellule mononucléaire dérivée du sang périphérique avec un vecteur du virus de Sendai chargé d'un gène de reprogrammation pour reprogrammer la cellule mononucléaire; et ensuite l'élimination artificiellement du vecteur du virus de Sendai chargé d'un gène de reprogrammation à partir de la cellule reprogrammée, le vecteur du virus de Sendai chargé d'un gène de reprogrammation possédant les gènes du virus de Sendai et un(des) gène(s) de reprogrammation sur le même génome, les gènes du virus de Sendai comprenant le gène NP, le gène P/C, le gène M, le gène F, le gène HN et le gène L, à condition que le gène M, le gène F et le gène HN manquent tous de leurs fonctions respectives et que la protéine L exprimée par le gène L du vecteur du virus de Sendai chargé d'un gène de reprogrammation possède une valine comme résidu d'acide aminé à la position 1618 qui permet l'expression génétique durable, où la cellule mononucléaire dérivée du sang périphérique est un monocyte.

2. Procédé selon la revendication 1, dans lequel le vecteur du virus de Sendai chargé d'un gène de reprogrammation est retiré de la cellule reprogrammée par de l'ARNsi.

3. Procédé selon la revendication 1, dans lequel le vecteur du virus de Sendai chargé d'un gène de reprogrammation comprend, sur son génome, une séquence cible pour un ARNmicro exprimée dans une cellule souche pluripotente.

4. Procédé selon la revendication 1, dans lequel la cellule mononucléaire dérivée du sang périphérique est dérivée de l'être humain.

5. Cellule souche pluripotente induite pouvant être obtenue grâce au procédé selon l'une quelconque des revendications 1 à 4.


### SEQUENCE LISTING

<110> National Institute of Advance Industrial Science and Thechnology Japan Biological Informatics Consortium
<120> A method of preparing pluripotent stem cells from peripheral blood
   mononuclear cells
<130> FP-162PCT
<150> JP P2010-250993
   <151> 2010-11-09
<160> 34
<170> PatentIn version 3.1
<210> 1
   <211> 2652
   <212> DNA
   <213> Artificial
<220>
   <223> Codon-optimaized T7 RNA polymerase
<400> 1
   atgaacacca tcaacattgc caaaaacgat ttcagcgaca ttgagctggc cgccatcccc      60
   ttcaataccc tggccgatca ctacggggag cggctggcca gggagcagct ggccctggag     120
   cacgagtctt acgagatggg cgaggcccgg ttccggaaaa tgtttgaacg ccagctgaaa     180
   gccggagaag tggccgataa cgccgccgcc aagcctctga ttaccaccct gctgcccaag     240
   atgattgccc ggattaacga ttggttcgaa gaggtgaaag ccaagagggg caagagacct     300
   accgcctttc agtttctgca ggaaatcaaa cctgaagccg tggcctacat caccattaag     360
   accacactgg cctgcctgac cagcgccgac aacaccacag tgcaggccgt ggccagcgcc     420
   atcggcagag ccatcgagga cgaagccagg ttcgggagga tcagggatct ggaggccaag     480
   cacttcaaga aaaatgtgga agagcagctg aataagcggg tgggccacgt gtacaagaag     540
   gccttcatgc aggtggtgga agccgatatg ctgagcaaag gcctgctggg cggagaagcc     600
   tggagctctt ggcacaagga agatagcatt cacgtgggcg tgagatgtat tgaaatgctg     660
   attgagagca cagggatggt gtccctgcac cggcagaacg ccggagtggt gggccaggat     720
   agcgagacca tcgagctggc ccccgaatat gccgaggcca ttgccacaag agccggggcc     780
   ctggccggga tctctccaat gttccagcca tgcgtggtgc ctccaaagcc atggacaggc     840
   atcaccggag ggggctactg ggccaatggg cgcaggcctc tggccctggt gaggacccac     900
   agcaaaaagg ccctgatgcg ctacgaggac gtgtacatgc ctgaggtgta caaagccatc     960
   aacattgccc agaacaccgc ctggaagatc aacaagaaag tgctggccgt ggccaatgtg    1020
   attaccaagt ggaagcactg tccagtggaa gacatccctg ccatcgagcg cgaggaactg    1080
   cctatgaagc ccgaggacat tgatatgaac cccgaagccc tgacagcctg gaagagagcc    1140
   gccgccgccg tgtaccgcaa agatcgcgcc cggaagtcta ggagaatttc cctggagttc 1200
   atgctggagc aggccaataa gttcgccaac cacaaggcca tctggttccc ctacaatatg 1260
   gattggcgcg gccgggtgta tgccgtgtcc atgttcaatc cccagggcaa cgacatgacc 1320
   aaaggcctgc tgacactggc caagggcaag cccatcggca aggaaggata ttattggctg 1380
   aagatccacg gcgccaattg tgccggggtg gacaaagtgc cttttcctga aaggatcaag 1440
   ttcatcgagg agaaccacga gaacatcatg gcctgtgcca aatctcccct ggagaacacc 1500
   tggtgggccg aacaggactc tcctttctgc tttctggcct tttgtttcga gtacgccggg 1560
   gtgcagcacc acggcctgtc ctacaattgc tctctgcctc tggcctttga cggctcttgc 1620
   tccgggattc agcactttag cgccatgctg cgggacgaag tgggcggaag ggccgtgaat 1680
   ctgctgccct ccgaaaccgt gcaggatatc tacggcatcg tggccaagaa agtgaatgaa 1740
   atcctgcagg ccgatgccat caacgggaca gataacgaag tggtgaccgt gacagacgag 1800
   aatacaggcg agattagcga aaaagtgaaa ctggggacca aggccctggc cggccagtgg 1860
   ctggcccacg gcgtgacaag gtctgtgacc aagcgcagcg tgatgaccct ggcctacggc 1920
   tccaaagagt tcgggttcag acagcaggtg ctggaagaca caatccagcc tgccatcgac 1980
   agcggaaagg ggcccatgtt cacccagcca aaccaggccg ccggctatat ggccaagctg 2040
   atctgggaaa gcgtgtctgt gacagtggtg gccgccgtgg aggccatgaa ttggctgaag 2100
   agcgccgcca agctgctggc cgccgaagtg aaagacaaga agacaggaga gattctgagg 2160
   aagaggtgcg ccgtgcactg ggtgacccca gatggattcc ccgtgtggca ggagtacaag 2220
   aaaccaatcc agaccaggct gaatctgatg ttcctgggcc agtttcgcct gcagccaaca 2280
   attaacacca acaaggattc cgagattgat gcccacaagc aggaatctgg catcgccccc 2340
   aactttgtgc actctcagga tgggtctcac ctgaggaaga ccgtggtgtg ggcccacgaa 2400
   aaatatggaa ttgagtcctt tgccctgatt cacgattcct ttggcacaat ccctgccgac 2460
   gccgccaacc tgttcaaggc cgtgagagaa accatggtgg atacctacga atcttgcgat 2520
   gtgctggccg atttctacga ccagttcgcc gatcagctgc acgagtccca gctggacaag 2580
   atgcccgccc tgcccgccaa aggcaacctg aacctgcggg atattctgga gagcgatttt 2640
   gccttcgcct aa 2652
<210> 2
   <211> 2129
   <212> DNA
   <213> Artificial
<220>
   <223> Optimized human Oct4,human Sox2 and a part of Sendai virus genome cDNA
<400> 2
   cctaggttcc ccatggcggg acacctggct tcggatttcg ccttctcgcc ccctccaggt 60
   ggtggaggtg atgggccagg ggggccggag ccgggctggg ttgatcctcg gacctggcta 120
   agcttccaag gccctcctgg agggccagga atcgggccgg gggttgggcc aggctctgag 180
   gtgtggggga ttcccccatg ccccccgccg tatgagttct gtggggggat ggcgtactgt 240
   gggccccagg ttggagtggg gctagtgccc caaggcggct tggagacctc tcagcctgag 300
   ggcgaagcag gagtcggggt ggagagcaac tccgatgggg cctccccgga gccctgcacc 360
   gtcacccctg gtgccgtgaa gctggagaag gagaagctgg agcaaaaccc ggaggagtcc 420
   caggacatca aagctctgca gaaagaactc gagcaatttg ccaagctcct gaagcagaag 480
   aggatcaccc tgggatatac acaggccgat gtggggctca ccctgggggt tctatttggg 540
   aaggtattca gccaaacgac catctgccgc tttgaggctc tgcagcttag cttcaagaac 600
   atgtgtaagc tgcggccctt gctgcagaag tgggtggagg aagctgacaa caatgaaaat 660
   cttcaggaga tatgcaaagc agaaaccctc gtgcaggccc gaaagagaaa gcgaaccagt 720
   atcgagaacc gagtgagagg caacctggag aatttgttcc tgcagtgccc gaaacccaca 780
   ctgcagcaga tcagccacat cgcccagcag cttgggctcg agaaggatgt ggtccgagtg 840
   tggttctgta accggcgcca gaagggcaag cgatcaagca gcgactatgc acaacgagag 900
   gattttgagg ctgctgggtc tcctttctca gggggaccag tgtcctttcc tctggcccca 960
   gggccccatt ttggtacccc aggctatggg agccctcact tcactgcact gtactcctcg 1020
   gtccctttcc ctgaggggga agcctttccc cctgtctccg tcaccactct gggctctccc 1080
   atgcattcaa actgaggacg tcagatctgt atataataag aaaaacttag ggtgaaagtg 1140
   aggttgcgcg gtattttagc tagcccgcat gtacaacatg atggagacgg agctgaagcc 1200
   gccgggcccg cagcaaactt cggggggcgg cggcggcaac tccaccgcgg cggcggccgg 1260
   cggcaaccag aaaaacagcc cggaccgcgt caagcggccc atgaatgcct tcatggtgtg 1320
   gtcccgcggg cagcggcgca agatggccca ggagaacccc aagatgcaca actcggagat 1380
   cagcaagcgc ctgggcgccg agtggaaact tttgtcggag acggagaagc ggccgttcat 1440
   cgacgaggct aagcggctgc gagcgctgca catgaaggag cacccggatt ataaataccg 1500
   gccccggcgg aaaaccaaga cgctcatgaa gaaggataag tacacgctgc ccggcgggct 1560
   gctggccccc ggcggcaata gcatggcgag cggggtcggg gtgggcgccg gcctgggcgc 1620
   gggcgtgaac cagcgcatgg acagttacgc gcacatgaac ggctggagca acggcagcta 1680
   cagcatgatg caggaccagc tgggctaccc gcagcacccg ggcctcaatg cgcacggcgc 1740
   agcgcagatg cagcccatgc accgctacga cgtgagcgcc ctgcagtaca actccatgac 1800
   cagctcgcag acctacatga acggctcgcc cacctacagc atgtcctact cgcagcaggg 1860
   cacccctggc atggctcttg gctccatggg ttcggtggtc aagtccgagg ccagctccag 1920
   cccccctgtg gttacctctt cctcccactc cagggcgccc tgccaggccg gggacctccg 1980
   ggacatgatc agcatgtatc tccccggcgc cgaggtgccg gaacccgccg cccccagcag 2040
   acttcacatg tcccagcact accagagcgg cccggtgccc ggcacggcca ttaacggcac 2100
   actgcccctc tcacacatgt gagaccggt 2129
<210> 3
   <211> 5851
   <212> DNA
   <213> Artificial
<220>
   <223> pMO078
<400> 3
<210> 4
   <211> 1478
   <212> DNA
   <213> Artificial
<220>
   <223> Optimized human Klf4 and a part of Sendai virus genome cDNA
<400> 4
   gctagcacct aggtctgaca tggctgtcag cgacgcgctg ctcccatctt tctccacgtt      60
   cgcgtctggc ccggcgggaa gggagaagac actgcgtcaa gcaggtgccc cgaataaccg     120
   ctggcgggag gagctctccc acatgaagcg acttccccca gtgcttcccg gccgccccta     180
   tgacctggcg gcggcgaccg tggccacaga cctggagagc ggcggagccg gtgcggcttg     240
   cggcggtagc aacctggcgc ccctacctcg gagagagacc gaggagttca acgatctcct     300
   ggacctggac tttattctct ccaattcgct gacccatcct ccggagtcag tggccgccac     360
   cgtgtcctcg tcagcgtcag cctcctcttc gtcgtcgccg tcgagcagcg gccctgccag     420
   cgcgccctcc acctgcagct tcacctatcc gatccgggcc gggaacgacc cgggcgtggc     480
   gccgggcggc acgggcggag gcctcctcta tggcagggag tccgctcccc ctccgacggc     540
   tcccttcaac ctggcggaca tcaacgacgt gagcccctcg ggcggcttcg tggccgagct     600
   cctgcggcca gaattggacc cggtgtacat tccgccgcag cagccgcagc cgccaggtgg     660
   cgggctgatg ggcaagttcg tgctgaaggc gtcgctgagc gcccctggca gcgagtacgg     720
   cagcccgtcg gtcatcagcg tcagcaaagg cagccctgac ggcagccacc cggtggtggt     780
   ggcgccctac aacggcgggc cgccgcgcac gtgccccaag atcaagcagg aggcggtctc     840
   ttcgtgcacc cacttgggcg ctggaccccc tctcagcaat ggccaccggc cggctgcaca     900
   cgacttcccc ctggggcggc agctccccag caggactacc ccgaccctgg gtcttgagga     960
   agtgctgagc agcagggact gtcaccctgc cctgccgctt cctcccggct tccatcccca    1020
   cccggggccc aattacccat ccttcctgcc cgatcagatg cagccgcaag tcccgccgct    1080
   ccattaccaa gagctcatgc cacccggttc ctgcatgcca gaggagccca agccaaagag    1140
   gggaagacga tcgtggcccc ggaaaaggac cgccacccac acttgtgatt acgcgggctg    1200
   cggcaaaacc tacacaaaga gttcccatct caaggcacac ctgcgaaccc acacaggtga    1260
   gaaaccttac cactgtgact gggacggctg tggatggaaa ttcgcccgct cagatgaact    1320
   gaccaggcac taccgtaaac acacggggca ccgcccgttc cagtgccaaa aatgcgaccg    1380
   agcattttcc aggtcggacc acctcgcctt acacatgaag aggcattttt aaagacgtcg    1440
   attaagaaaa acttagggtg aaagttcatc gcggccgc                            1478
<210> 5
   <211> 38
   <212> DNA
   <213> Artificial
<220>
   <223> Primer
<400> 5
   actagctagc agtctgacat ggctgtcagc gacgcgct 38
<210> 6
   <211> 32
   <212> DNA
   <213> Artificial
<220>
   <223> Primer
<400> 6
   ggtccacgcg tttaaaaatg cctcttcatg tg 32
<210> 7
   <211> 1845
   <212> DNA
   <213> Artificial
<220>
   <223> pMO026
<400> 7
   gaattcgagt catcccgaga cgcgagttat gtgtttgcaa gacgtgccct aaagtctgca 60
   aactatgcag agatgacatt caatgtatgc ggcctgatcc tttctgccga gaaatcttcc 120
   gctcgtaagg tagatgagaa caaacaactg ctcaaacaga tccaagagag cgtggaatca 180
   ttccgggata tttacaagag attctctgag tatcagaaag aacagaactc attgctgatg     240
   tccaacctat ctacacttca tatcatcaca gatagaggtg gcaagactga caacacagac     300
   tcccttacaa ggtccccctc cgtttttgca aaatcaaaag agaacaagac taaggctacc     360
   aggtttgacc catctatgga gaccttagaa gatatgaagt acaaaccgga cctaatccga     420
   gaggatgaat ttagagatga gatccgcaac ccggtgtacc aagagaggga cacagaaccc     480
   agggcctcaa acgcatcacg tctcttcccc tccaaagaga agcccacaat gcactctctc     540
   aggctcgtca tagagagcag tcccctaagc agagctgaga aagcagcata tgtgaaatca     600
   ttatccaagt gcaagacaga ccaagaggtt aaggcagtca tggaactcgt agaagaggac     660
   atagagtcac tgaccaacta gatcccgggt gaggcatccc accatcctca gtcacagaga     720
   gacccaatct accatcagca tcagccagta aagattaaga aaaacttagg gtgaaagaaa     780
   tgcggccgct tgctagcaga atatatgaaa acatttaaca tttctcaaca agatctagaa     840
   ttagtagaag tagcgacaga gaagattaca atgctttatg aggataataa acatcatgtg     900
   ggagcggcaa ttcgtacgaa aacaggagaa atcatttcgg cagtacatat tgaagcgtat     960
   ataggacgag taactgtttg tgcagaagcc attgccattg gtagtgcagt ttcgaatgga    1020
   caaaaggatt ttgacacgat tgtagctgtt agacaccctt attctgacga agtagataga    1080
   agtattcgag tggtaagtcc ttgtggtatg tgtagggagt tgatttcaga ctatgcacca    1140
   gattgttttg tgttaataga aatgaatggc aagttagtca aaactacgat tgaagaactc    1200
   attccactca aatatacccg aaattaaaac gcgtcagaga cctgcaacaa tgtctcaagc    1260
   agacaccacc tggcagtcgg agccaccggg tcactccttg tcttaaataa gaaaaactta    1320
   gggataaagt cccttgtgag tgcttggttg caaaactctc cccttgggaa acatgacagc    1380
   atatatccgg aggtcacagt gcatctcaac atcactactg gttgttctca ccacattggt    1440
   ctcgtgtcag attcccaggg atatgctctc taacataggg gtcatagtcg atgaagggaa    1500
   atcactgaag atagctgggt cccacgaatc gaggtacata gtactgagtc tagttccggg    1560
   ggtagacctt gagaatggat gcggaacagc tcaggttatc cagtacaaga gcctactgaa    1620
   caggctgtta atcccattga gggatgcctt agatcttcag gaggctctga taactgtcac    1680
   caatgatacg acacaaaatg ccggtgttcc acagttgaga ttcttcggtg ctgtgattgg    1740
   tactatcgca cttggagtgg cgacatcagc acagatcacc acagggattg cactagccga    1800
   agcgagggag gccaaaagag acatagcgct catcaaggaa tcgat                    1845
<210> 8
   <211> 3696
   <212> DNA
   <213> Artificial
<220>
   <223> A part of Sendai virus genome cDNA : Xhol-T7pro-SevcDNA-Nhel-Not1
<400> 8
   ctcgagtaat acgactcact atagggacca aacaagagaa gaaacatgta tggaatatat      60
   aatgaagtta gacaggattt tagggtcaaa gtatccaccc tgaggagcag gttccagatc     120
   cttttctttg ctgccaaagt tcacgatggc cgggttgttg agcaccttcg atacatttag     180
   ctctaggagg agcgaaagta ttaataagtc gggaggaggt gctgttatcc ccggccagag     240
   gagcacagtc tcagtgttca tactaggccc aagtgtgact gatgatgcag acaagttatt     300
   cattgcaaca accttcctag ctcactcatt ggacacagat aagcagcact ctcagagagg     360
   agggttcctc gtctctctgc ttgccatggc ttacagtagt ccagaattgt acttgacaac     420
   aaacggagta aacgccgatg tcaaatatgt gatctacaac atagagaaag accctaagag     480
   gacgaagaca gacggattca ttgtgaagac gagagatatg gaatatgaga ggaccacaga     540
   atggctgttt ggacctatgg tcaacaagag cccactcttc cagggtcaac gggatgctgc     600
   agaccctgac acactccttc aaatctatgg gtatcctgca tgcctaggag caataattgt     660
   ccaagtctgg attgtgctgg tgaaggccat cacaagcagc gccggcttaa ggaaagggtt     720
   cttcaacagg ttagaggcgt tcagacaaga cggcaccgtg aaaggtgcct tagttttcac     780
   tggggagaca gttgagggga taggctcggt tatgagatct cagcaaagcc ttgtatctct     840
   catggttgag acccttgtga ctatgaatac tgcaagatct gatctcacca cattagagaa     900
   gaacatccag atcgttggga actacatccg agatgcaggg ctggcttcct tcatgaacac     960
   tattaaatat ggggtggaga caaagatggc agctctaacg ttgtcaaacc tgaggcccga    1020
   tattaataag cttagaagcc tcatagacac ctacctgtca aaaggcccca gagctccctt    1080
   tatctgtatc ctcaaggacc ctgttcatgg tgaatttgct ccaggcaatt atcctgcact    1140
   atggagttac gccatgggag tcgccgtcgt acagaacaag tcaatgcagc agtacgtcac    1200
   agggaggaca taccttgata tggaaatgtt cttactagga caagccgtgg caaaggatgc    1260
   tgaatcgaag atcagcagtg ccttggaaga tgagttagga gtgacggata cagccaagga    1320
   gaggctcaga catcatctgg caaacttgtc cggtggggat ggtgcttacc acaaaccaac    1380
   aggcggtggt gcaattgagg tagctctaga caatgccgat atcgacctgg aaacagaagc    1440
   tcatgcggac caggacgcta ggggttgggg tggagatagt ggtgaaagat gggcacgtca    1500
   ggtgagtggt ggccactttg tcacactaca tggggctgaa cggttagagg aggaaaccaa    1560
   tgatgaggat gtatcagaca tagagagaag aatagccatg agactcgcag agagacggca    1620
   agaggattct gcaacccatg gagatgaagg ccgcaataac ggtgttgatc acgaagaaga    1680
   tgacgatgcc gcagcagcag ctgggatagg aggaatctag gatcatacga ggcctcaagg    1740
   tacttgatcc gcagtaagaa aaacttaggg tgaaagttca tccaccgatc ggctcaggca 1800
   aggccacacc caaccccacc gaccacaccc agcagtcgag acagccacgg cttcggctac 1860
   acttaccgca tggatcaaga tgccttcatt cttaaagaag attctgaagt tgagaggaag 1920
   gcgccaggag gacgagagtc gctctcggat gttatcggat tcctcgatgc tgtcctgtcg 1980
   aatgaaccaa ctgacatcgg aggggacaga agctggctcc acaacaccat caacactccc 2040
   caaggaccag gctctgctca tagagccaaa agtgagggcg aaggagaagt ctcaacaccg 2100
   tcgacccaag ataatcgatc aggtgaggag agtagagtct ctgggagaac aagcaagcca 2160
   gaggcagaag cacatgctgg aaaccttgat aaacaaaata tacactgggc ctttagggga 2220
   agaactggta caaactctgt atctcaggat ctggacgatg gaggagactc cggaatcctt 2280
   gaaaatcctc caaatgagag aggatatccg agatcaggta ttgaagatga aaacagagag 2340
   atggctgcgc accctgataa gaggggagaa gaccaagctg aaggacttcc agaagaggta 2400
   cgaggaggta catccctacc tgatgaagga gaaggtggag caagtaataa tggaagaagc 2460
   atggagcctg gcagctcaca tagtgcaaga gtaactgggg tcctggtgat tcctagcccc 2520
   gaacttgaag aggctgtgct acggaggaac aaaagaagac ctaccaacag tgggtccaaa 2580
   cctcttactc cagcaaccgt gcctggcacc cggtccccac cgctgaatcg ttacaacagc 2640
   acagggtcac caccaggaaa acccccatct acacaggatg agcacatcaa ctctggggac 2700
   acccccgccg tcagggtcaa agaccggaaa ccatcaatag ggactcgctc tgtctcagat 2760
   tgtccagcca acggccgccc aatccatccg ggtatagaga ccgactcaac aaaaaagggc 2820
   ataggagaga acacatcatc tatgaaagat atggctacat tgttgacgag tcttggtgta 2880
   atccagtctg ctcaagaatt cgagtcatcc cgagacgcga gttatgtgtt tgcaagacgt 2940
   gccctaaagt ctgcaaacta tgcagagatg acattcaatg tatgcggcct gatcctttct 3000
   gccgagaaat cttccgctcg taaggtagat gagaacaaac aactgctcaa acagatccaa 3060
   gagagcgtgg aatcattccg ggatatttac aagagattct ctgagtatca gaaagaacag 3120
   aactcattgc tgatgtccaa cctatctaca cttcatatca tcacagatag aggtggcaag 3180
   actgacaaca cagactccct tacaaggtcc ccctccgttt ttgcaaaatc aaaagagaac 3240
   aagactaagg ctaccaggtt tgacccatct atggagacct tagaagatat gaagtacaaa 3300
   ccggacctaa tccgagagga tgaatttaga gatgagatcc gcaacccggt gtaccaagag 3360
   agggacacag aacccagggc ctcaaacgca tcacgtctct tcccctccaa agagaagccc 3420
   acaatgcact ctctcaggct cgtcatagag agcagtcccc taagcagagc tgagaaagca 3480
   gcatatgtga aatcattatc caagtgcaag acagaccaag aggttaaggc agtcatggaa 3540
   ctcgtagaag aggacataga gtcactgacc aactagatcc cgggtgaggc atcccaccat 3600
   cctcagtcac agagagaccc aatctaccat cagcatcagc cagtaaagat taagaaaaac 3660
   ttagggtgaa agaaatttca ccgctagcgc ggccgc 3696
<210> 9
   <211> 5160
   <212> DNA
   <213> Artificial
<220>
   <223> pMO094(+E)Mp;Klf4 Xhol-
<400> 9
   ctcgagtaat acgactcact atagggacca aacaagagaa gaaacatgta tggaatatat     60
   aatgaagttt aagaaaaact tagggtcaaa gtatccaccc tgaggagcag gttccagatc    120
   cttttctttg ctgccaaagt tcacgatggc cgggttgttg agcaccttcg atacatttag    180
   ctctaggagg agcgaaagta ttaataagtc gggaggaggt gctgttatcc ccggccagag    240
   gagcacagtc tcagtgttca tactaggccc aagtgtgact gatgatgcag acaagttatt    300
   cattgcaaca accttcctag ctcactcatt ggacacagat aagcagcact ctcagagagg    360
   agggttcctc gtctctctgc ttgccatggc ttacagtagt ccagaattgt acttgacaac    420
   aaacggagta aacgccgatg tcaaatatgt gatctacaac atagagaaag accctaagag    480
   gacgaagaca gacggattca ttgtgaagac gagagatatg gaatatgaga ggaccacaga    540
   atggctgttt ggacctatgg tcaacaagag cccactcttc cagggtcaac gggatgctgc    600
   agaccctgac acactccttc aaatctatgg gtatcctgca tgcctaggag caataattgt    660
   ccaagtctgg attgtgctgg tgaaggccat cacaagcagc gccggcttaa ggaaagggtt    720
   cttcaacagg ttagaggcgt tcagacaaga cggcaccgtg aaaggtgcct tagttttcac    780
   tggggagaca gttgagggga taggctcggt tatgagatct cagcaaagcc ttgtatctct    840
   catggttgag acccttgtga ctatgaatac tgcaagatct gatctcacca cattagagaa    900
   gaacatccag atcgttggga actacatccg agatgcaggg ctggcttcct tcatgaacac    960
   tattaaatat ggggtggaga caaagatggc agctctaacg ttgtcaaacc tgaggcccga   1020
   tattaataag cttagaagcc tcatagacac ctacctgtca aaaggcccca gagctccctt   1080
   tatctgtatc ctcaaggacc ctgttcatgg tgaatttgct ccaggcaatt atcctgcact   1140
   atggagttac gccatgggag tcgccgtcgt acagaacaag tcaatgcagc agtacgtcac   1200
   agggaggaca taccttgata tggaaatgtt cttactagga caagccgtgg caaaggatgc   1260
   tgaatcgaag atcagcagtg ccttggaaga tgagttagga gtgacggata cagccaagga   1320
   gaggctcaga catcatctgg caaacttgtc cggtggggat ggtgcttacc acaaaccaac   1380
   aggcggtggt gcaattgagg tagctctaga caatgccgat atcgacctgg aaacagaagc   1440
   tcatgcggac caggacgcta ggggttgggg tggagatagt ggtgaaagat gggcacgtca   1500
   ggtgagtggt ggccactttg tcacactaca tggggctgaa cggttagagg aggaaaccaa 1560
   tgatgaggat gtatcagaca tagagagaag aatagccatg agactcgcag agagacggca 1620
   agaggattct gcaacccatg gagatgaagg ccgcaataac ggtgttgatc acgaagaaga 1680
   tgacgatgcc gcagcagcag ctgggatagg aggaatctag gatcatacga ggcctcaagg 1740
   tacttgatcc gcagtaagaa aaacttaggg tgaaagttca tccaccgatc ggctcaggca 1800
   aggccacacc caaccccacc gaccacaccc agcagtcgag acagccacgg cttcggctac 1860
   acttaccgca tggatcaaga tgccttcatt cttaaagaag attctgaagt tgagaggaag 1920
   gcgccaggag gacgagagtc gctctcggat gttatcggat tcctcgatgc tgtcctgtcg 1980
   aatgaaccaa ctgacatcgg aggggacaga agctggctcc acaacaccat caacactccc 2040
   caaggaccag gctctgctca tagagccaaa agtgagggcg aaggagaagt ctcaacaccg 2100
   tcgacccaag ataatcgatc aggtgaggag agtagagtct ctgggagaac aagcaagcca 2160
   gaggcagaag cacatgctgg aaaccttgat aaacaaaata tacactgggc ctttagggga 2220
   agaactggta caaactctgt atctcaggat ctggacgatg gaggagactc cggaatcctt 2280
   gaaaatcctc caaatgagag aggatatccg agatcaggta ttgaagatga aaacagagag 2340
   atggctgcgc accctgataa gaggggagaa gaccaagctg aaggacttcc agaagaggta 2400
   cgaggaggta catccctacc tgatgaagga gaaggtggag caagtaataa tggaagaagc 2460
   atggagcctg gcagctcaca tagtgcaaga gtaactgggg tcctggtgat tcctagcccc 2520
   gaacttgaag aggctgtgct acggaggaac aaaagaagac ctaccaacag tgggtccaaa 2580
   cctcttactc cagcaaccgt gcctggcacc cggtccccac cgctgaatcg ttacaacagc 2640
   acagggtcac caccaggaaa acccccatct acacaggatg agcacatcaa ctctggggac 2700
   acccccgccg tcagggtcaa agaccggaaa ccatcaatag ggactcgctc tgtctcagat 2760
   tgtccagcca acggccgccc aatccatccg ggtatagaga ccgactcaac aaaaaagggc 2820
   ataggagaga acacatcatc tatgaaagat atggctacat tgttgacgag tcttggtgta 2880
   atccagtctg ctcaagaatt cgagtcatcc cgagacgcga gttatgtgtt tgcaagacgt 2940
   gccctaaagt ctgcaaacta tgcagagatg acattcaatg tatgcggcct gatcctttct 3000
   gccgagaaat cttccgctcg taaggtagat gagaacaaac aactgctcaa acagatccaa 3060
   gagagcgtgg aatcattccg ggatatttac aagagattct ctgagtatca gaaagaacag 3120
   aactcattgc tgatgtccaa cctatctaca cttcatatca tcacagatag aggtggcaag 3180
   actgacaaca cagactccct tacaaggtcc ccctccgttt ttgcaaaatc aaaagagaac 3240
   aagactaagg ctaccaggtt tgacccatct atggagacct tagaagatat gaagtacaaa 3300
   ccggacctaa tccgagagga tgaatttaga gatgagatcc gcaacccggt gtaccaagag 3360
   agggacacag aacccagggc ctcaaacgca tcacgtctct tcccctccaa agagaagccc 3420
   acaatgcact ctctcaggct cgtcatagag agcagtcccc taagcagagc tgagaaagca 3480
   gcatatgtga aatcattatc caagtgcaag acagaccaag aggttaaggc agtcatggaa 3540
   ctcgtagaag aggacataga gtcactgacc aactagatcc cgggtgaggc atcccaccat 3600
   cctcagtcac agagagaccc aatctaccat cagcatcagc cagtaaagat taagaaaaac 3660
   ttagggtgaa agaaatttca ccgctagcac ctaggtctga catggctgtc agcgacgcgc 3720
   tgctcccatc tttctccacg ttcgcgtctg gcccggcggg aagggagaag acactgcgtc 3780
   aagcaggtgc cccgaataac cgctggcggg aggagctctc ccacatgaag cgacttcccc 3840
   cagtgcttcc cggccgcccc tatgacctgg cggcggcgac cgtggccaca gacctggaga 3900
   gcggcggagc cggtgcggct tgcggcggta gcaacctggc gcccctacct cggagagaga 3960
   ccgaggagtt caacgatctc ctggacctgg actttattct ctccaattcg ctgacccatc 4020
   ctccggagtc agtggccgcc accgtgtcct cgtcagcgtc agcctcctct tcgtcgtcgc 4080
   cgtcgagcag cggccctgcc agcgcgccct ccacctgcag cttcacctat ccgatccggg 4140
   ccgggaacga cccgggcgtg gcgccgggcg gcacgggcgg aggcctcctc tatggcaggg 4200
   agtccgctcc ccctccgacg gctcccttca acctggcgga catcaacgac gtgagcccct 4260
   cgggcggctt cgtggccgag ctcctgcggc cagaattgga cccggtgtac attccgccgc 4320
   agcagccgca gccgccaggt ggcgggctga tgggcaagtt cgtgctgaag gcgtcgctga 4380
   gcgcccctgg cagcgagtac ggcagcccgt cggtcatcag cgtcagcaaa ggcagccctg 4440
   acggcagcca cccggtggtg gtggcgccct acaacggcgg gccgccgcgc acgtgcccca 4500
   agatcaagca ggaggcggtc tcttcgtgca cccacttggg cgctggaccc cctctcagca 4560
   atggccaccg gccggctgca cacgacttcc ccctggggcg gcagctcccc agcaggacta 4620
   ccccgaccct gggtcttgag gaagtgctga gcagcaggga ctgtcaccct gccctgccgc 4680
   ttcctcccgg cttccatccc cacccggggc ccaattaccc atccttcctg cccgatcaga 4740
   tgcagccgca agtcccgccg ctccattacc aagagctcat gccacccggt tcctgcatgc 4800
   cagaggagcc caagccaaag aggggaagac gatcgtggcc ccggaaaagg accgccaccc 4860
   acacttgtga ttacgcgggc tgcggcaaaa cctacacaaa gagttcccat ctcaaggcac 4920
   acctgcgaac ccacacaggt gagaaacctt accactgtga ctgggacggc tgtggatgga 4980
   aattcgcccg ctcagatgaa ctgaccaggc actaccgtaa acacacgggg caccgcccgt 5040
   tccagtgcca aaaatgcgac cgagcatttt ccaggtcgga ccacctcgcc ttacacatga 5100
   agaggcattt ttaaagacgt cgattaagaa aaacttaggg tgaaagttca tcgcggccgc 5160
<210> 10
   <211> 39
   <212> DNA
   <213> Artificial
<220>
   <223> Primer
<400> 10
   actagctagc ttagacgctg gatttttttc gggtagtgg 39
<210> 11
   <211> 30
   <212> DNA
   <213> Artificial
<220>
   <223> Primer
<400> 11
   gtccgacgtc cttacgcaca agagttccgt 30
<210> 12
   <211> 15696
   <212> DNA
   <213> Artificial
<220>
   <223> Template cDNA for SeVp(Mp+myc,delta-M::Klf4,delta-F::Oct4,delta-H N::Sox2)
<400> 12
   accaaacaag agaagaaaca tgtatggaat atataatgaa gtttaagaaa aacttagggt     60
   caaagtatcc accctgagga gcaggttcca gatccttttc tttgctgcca aagttcacga    120
   tggccgggtt gttgagcacc ttcgatacat ttagctctag gaggagcgaa agtattaata    180
   agtcgggagg aggtgctgtt atccccggcc agaggagcac agtctcagtg ttcatactag    240
   gcccaagtgt gactgatgat gcagacaagt tattcattgc aacaaccttc ctagctcact    300
   cattggacac agataagcag cactctcaga gaggagggtt cctcgtctct ctgcttgcca    360
   tggcttacag tagtccagaa ttgtacttga caacaaacgg agtaaacgcc gatgtcaaat    420
   atgtgatcta caacatagag aaagacccta agaggacgaa gacagacgga ttcattgtga    480
   agacgagaga tatggaatat gagaggacca cagaatggct gtttggacct atggtcaaca    540
   agagcccact cttccagggt caacgggatg ctgcagaccc tgacacactc cttcaaatct    600
   atgggtatcc tgcatgccta ggagcaataa ttgtccaagt ctggattgtg ctggtgaagg    660
   ccatcacaag cagcgccggc ttaaggaaag ggttcttcaa caggttagag gcgttcagac    720
   aagacggcac cgtgaaaggt gccttagttt tcactgggga gacagttgag gggataggct    780
   cggttatgag atctcagcaa agccttgtat ctctcatggt tgagaccctt gtgactatga    840
   atactgcaag atctgatctc accacattag agaagaacat ccagatcgtt gggaactaca    900
   tccgagatgc agggctggct tccttcatga acactattaa atatggggtg gagacaaaga    960
   tggcagctct aacgttgtca aacctgaggc ccgatattaa taagcttaga agcctcatag   1020
   acacctacct gtcaaaaggc cccagagctc cctttatctg tatcctcaag gaccctgttc   1080
   atggtgaatt tgctccaggc aattatcctg cactatggag ttacgccatg ggagtcgccg   1140
   tcgtacagaa caagtcaatg cagcagtacg tcacagggag gacatacctt gatatggaaa   1200
   tgttcttact aggacaagcc gtggcaaagg atgctgaatc gaagatcagc agtgccttgg   1260
   aagatgagtt aggagtgacg gatacagcca aggagaggct cagacatcat ctggcaaact   1320
   tgtccggtgg ggatggtgct taccacaaac caacaggcgg tggtgcaatt gaggtagctc   1380
   tagacaatgc cgatatcgac ctggaaacag aagctcatgc ggaccaggac gctaggggtt   1440
   ggggtggaga tagtggtgaa agatgggcac gtcaggtgag tggtggccac tttgtcacac   1500
   tacatggggc tgaacggtta gaggaggaaa ccaatgatga ggatgtatca gacatagaga   1560
   gaagaatagc catgagactc gcagagagac ggcaagagga ttctgcaacc catggagatg   1620
   aaggccgcaa taacggtgtt gatcacgaag aagatgacga tgccgcagca gcagctggga   1680
   taggaggaat ctaggatcat acgaggcctc aaggtacttg atccgcagta agaaaaactt   1740
   agggtgaaag ttcatccacc gatcggctca ggcaaggcca cacccaaccc caccgaccac   1800
   acccagcagt cgagacagcc acggcttcgg ctacacttac cgcatggatc aagatgcctt   1860
   cattcttaaa gaagattctg aagttgagag gaaggcgcca ggaggacgag agtcgctctc   1920
   ggatgttatc ggattcctcg atgctgtcct gtcgaatgaa ccaactgaca tcggagggga   1980
   cagaagctgg ctccacaaca ccatcaacac tccccaagga ccaggctctg ctcatagagc   2040
   caaaagtgag ggcgaaggag aagtctcaac accgtcgacc caagataatc gatcaggtga   2100
   ggagagtaga gtctctggga gaacaagcaa gccagaggca gaagcacatg ctggaaacct   2160
   tgataaacaa aatatacact gggcctttag gggaagaact ggtacaaact ctgtatctca   2220
   ggatctggac gatggaggag actccggaat ccttgaaaat cctccaaatg agagaggata   2280
   tccgagatca ggtattgaag atgaaaacag agagatggct gcgcaccctg ataagagggg   2340
   agaagaccaa gctgaaggac ttccagaaga ggtacgagga ggtacatccc tacctgatga   2400
   aggagaaggt ggagcaagta ataatggaag aagcatggag cctggcagct cacatagtgc   2460
   aagagtaact ggggtcctgg tgattcctag ccccgaactt gaagaggctg tgctacggag   2520
   gaacaaaaga agacctacca acagtgggtc caaacctctt actccagcaa ccgtgcctgg   2580
   cacccggtcc ccaccgctga atcgttacaa cagcacaggg tcaccaccag gaaaaccccc   2640
   atctacacag gatgagcaca tcaactctgg ggacaccccc gccgtcaggg tcaaagaccg   2700
   gaaaccatca atagggactc gctctgtctc agattgtcca gccaacggcc gcccaatcca   2760
   tccgggtata gagaccgact caacaaaaaa gggcatagga gagaacacat catctatgaa   2820
   agatatggct acattgttga cgagtcttgg tgtaatccag tctgctcaag aattcgagtc   2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcccacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcttagacgc tggatttttt tcgggtagtg gaaaaccagc agcctcccgc gacgatgccc 3720
   ctcaacgtta gcttcaccaa caggaactat gacctcgact acgactcggt gcagccgtat 3780
   ttctactgcg acgaggagga gaacttctac cagcagcagc agcagagcga gctgcagccc 3840
   ccggcgccca gcgaggatat ctggaagaaa ttcgagctgc tgcccacccc gcccctgtcc 3900
   cctagccgcc gctccgggct ctgctcgccc tcctacgttg cggtcacacc cttctccctt 3960
   cggggagaca acgacggcgg tggcgggagc ttctccacgg ccgaccagct ggagatggtg 4020
   accgagctgc tgggaggaga catggtgaac cagagtttca tctgcgaccc ggacgacgag 4080
   accttcatca aaaacatcat catccaggac tgtatgtgga gcggcttctc ggccgccgcc 4140
   aagctcgtct cagagaagct ggcctcctac caggctgcgc gcaaagacag cggcagcccg 4200
   aaccccgccc gcggccacag cgtctgctcc acctccagct tgtacctgca ggatctgagc 4260
   gccgccgcct cagagtgcat cgacccctcg gtggtcttcc cctaccctct caacgacagc 4320
   agctcgccca agtcctgcgc ctcgcaagac tccagcgcct tctctccgtc ctcggattct 4380
   ctgctctcct cgacggagtc ctccccgcag ggcagccccg agcccctggt gctccatgag 4440
   gagacaccgc ccaccaccag cagcgactct gaggaggaac aagaagatga ggaagaaatc 4500
   gatgttgttt ctgtggaaaa gaggcaggct cctggcaaaa ggtcagagtc tggatcacct 4560
   tctgctggag gccacagcaa acctcctcac agcccactgg tcctcaagag gtgccacgtc 4620
   tccacacatc agcacaacta cgcagcgcct ccctccactc ggaaggacta tcctgctgcc 4680
   aagagggtca agttggacag tgtcagagtc ctgagacaga tcagcaacaa ccgaaaatgc 4740
   accagcccca ggtcctcgga caccgaggag aatgtcaaga ggcgaacaca caacgtcttg 4800
   gagcgccaga ggaggaacga gctaaaacgg agcttttttg ccctgcgtga ccagatcccg 4860
   gagttggaaa acaatgaaaa ggcccccaag gtagttatcc ttaaaaaagc cacagcatac 4920
   atcctgtccg tccaagcaga ggagcaaaag ctcatttctg aagaggactt gttgcggaaa 4980
   cgacgagaac agttgaaaca caaacttgaa cagctacgga actcttgtgc gtaaggacgt 5040
   cgattaagaa aaacttaggg tgaaagttca tcgcggccgc ttgctagcag tctgacatgg 5100
   ctgtcagcga cgcgctgctc ccatctttct ccacgttcgc gtctggcccg gcgggaaggg 5160
   agaagacact gcgtcaagca ggtgccccga ataaccgctg gcgggaggag ctctcccaca 5220
   tgaagcgact tcccccagtg cttcccggcc gcccctatga cctggcggcg gcgaccgtgg 5280
   ccacagacct ggagagcggc ggagccggtg cggcttgcgg cggtagcaac ctggcgcccc 5340
   tacctcggag agagaccgag gagttcaacg atctcctgga cctggacttt attctctcca 5400
   attcgctgac ccatcctccg gagtcagtgg ccgccaccgt gtcctcgtca gcgtcagcct 5460
   cctcttcgtc gtcgccgtcg agcagcggcc ctgccagcgc gccctccacc tgcagcttca 5520
   cctatccgat ccgggccggg aacgacccgg gcgtggcgcc gggcggcacg ggcggaggcc 5580
   tcctctatgg cagggagtcc gctccccctc cgacggctcc cttcaacctg gcggacatca 5640
   acgacgtgag cccctcgggc ggcttcgtgg ccgagctcct gcggccagaa ttggacccgg 5700
   tgtacattcc gccgcagcag ccgcagccgc caggtggcgg gctgatgggc aagttcgtgc 5760
   tgaaggcgtc gctgagcgcc cctggcagcg agtacggcag cccgtcggtc atcagcgtca 5820
   gcaaaggcag ccctgacggc agccacccgg tggtggtggc gccctacaac ggcgggccgc 5880
   cgcgcacgtg ccccaagatc aagcaggagg cggtctcttc gtgcacccac ttgggcgctg 5940
   gaccccctct cagcaatggc caccggccgg ctgcacacga cttccccctg gggcggcagc 6000
   tccccagcag gactaccccg accctgggtc ttgaggaagt gctgagcagc agggactgtc 6060
   accctgccct gccgcttcct cccggcttcc atccccaccc ggggcccaat tacccatcct 6120
   tcctgcccga tcagatgcag ccgcaagtcc cgccgctcca ttaccaagag ctcatgccac 6180
   ccggttcctg catgccagag gagcccaagc caaagagggg aagacgatcg tggccccgga 6240
   aaaggaccgc cacccacact tgtgattacg cgggctgcgg caaaacctac acaaagagtt 6300
   cccatctcaa ggcacacctg cgaacccaca caggtgagaa accttaccac tgtgactggg 6360
   acggctgtgg atggaaattc gcccgctcag atgaactgac caggcactac cgtaaacaca 6420
   cggggcaccg cccgttccag tgccaaaaat gcgaccgagc attttccagg tcggaccacc 6480
   tcgccttaca catgaagagg catttttaaa cgcgtcagag acctgcaaca atgtctcaag 6540
   cagacaccac ctggcagtcg gagccaccgg gtcactcctt gtcttaaata agaaaaactt 6600
   agggataaag tcccttagat ctagcctagg ttccccatgg cgggacacct ggcttcggat 6660
   ttcgccttct cgccccctcc aggtggtgga ggtgatgggc caggggggcc ggagccgggc 6720
   tgggttgatc ctcggacctg gctaagcttc caaggccctc ctggagggcc aggaatcggg 6780
   ccgggggttg ggccaggctc tgaggtgtgg gggattcccc catgcccccc gccgtatgag 6840
   ttctgtgggg ggatggcgta ctgtgggccc caggttggag tggggctagt gccccaaggc 6900
   ggcttggaga cctctcagcc tgagggcgaa gcaggagtcg gggtggagag caactccgat 6960
   ggggcctccc cggagccctg caccgtcacc cctggtgccg tgaagctgga gaaggagaag 7020
   ctggagcaaa acccggagga gtcccaggac atcaaagctc tgcagaaaga actcgagcaa 7080
   tttgccaagc tcctgaagca gaagaggatc accctgggat atacacaggc cgatgtgggg 7140
   ctcaccctgg gggttctatt tgggaaggta ttcagccaaa cgaccatctg ccgctttgag 7200
   gctctgcagc ttagcttcaa gaacatgtgt aagctgcggc ccttgctgca gaagtgggtg 7260
   gaggaagctg acaacaatga aaatcttcag gagatatgca aagcagaaac cctcgtgcag 7320
   gcccgaaaga gaaagcgaac cagtatcgag aaccgagtga gaggcaacct ggagaatttg 7380
   ttcctgcagt gcccgaaacc cacactgcag cagatcagcc acatcgccca gcagcttggg 7440
   ctcgagaagg atgtggtccg agtgtggttc tgtaaccggc gccagaaggg caagcgatca 7500
   agcagcgact atgcacaacg agaggatttt gaggctgctg ggtctccttt ctcaggggga 7560
   ccagtgtcct ttcctctggc cccagggccc cattttggta ccccaggcta tgggagccct 7620
   cacttcactg cactgtactc ctcggtccct ttccctgagg gggaagcctt tccccctgtc 7680
   tccgtcacca ctctgggctc tcccatgcat tcaaactgag gacgtcagat ctgtatataa 7740
   taagaaaaac ttagggtgaa agtgaggttg cgcggtattt tagctagccc gcatgtacaa 7800
   catgatggag acggagctga agccgccggg cccgcagcaa acttcggggg gcggcggcgg 7860
   caactccacc gcggcggcgg ccggcggcaa ccagaaaaac agcccggacc gcgtcaagcg 7920
   gcccatgaat gccttcatgg tgtggtcccg cgggcagcgg cgcaagatgg cccaggagaa 7980
   ccccaagatg cacaactcgg agatcagcaa gcgcctgggc gccgagtgga aacttttgtc 8040
   ggagacggag aagcggccgt tcatcgacga ggctaagcgg ctgcgagcgc tgcacatgaa 8100
   ggagcacccg gattataaat accggccccg gcggaaaacc aagacgctca tgaagaagga 8160
   taagtacacg ctgcccggcg ggctgctggc ccccggcggc aatagcatgg cgagcggggt 8220
   cggggtgggc gccggcctgg gcgcgggcgt gaaccagcgc atggacagtt acgcgcacat 8280
   gaacggctgg agcaacggca gctacagcat gatgcaggac cagctgggct acccgcagca 8340
   cccgggcctc aatgcgcacg gcgcagcgca gatgcagccc atgcaccgct acgacgtgag 8400
   cgccctgcag tacaactcca tgaccagctc gcagacctac atgaacggct cgcccaccta 8460
   cagcatgtcc tactcgcagc agggcacccc tggcatggct cttggctcca tgggttcggt 8520
   ggtcaagtcc gaggccagct ccagcccccc tgtggttacc tcttcctccc actccagggc 8580
   gccctgccag gccggggacc tccgggacat gatcagcatg tatctccccg gcgccgaggt 8640
   gccggaaccc gccgccccca gcagacttca catgtcccag cactaccaga gcggcccggt 8700
   gcccggcacg gccattaacg gcacactgcc cctctcacac atgtgagacc ggtgtcggct 8760
   ttgctgacac tagagtcatc tccgaacatc cacaatatct ctcagtctct tacgtctctc 8820
   acagtattaa gaaaaaccca gggtgaatgg gaagcttgcc ataggtcatg gatgggcagg 8880
   agtcctccca aaacccttct gacatactct atccagaatg ccacctgaac tctcccatag 8940
   tcagggggaa gatagcacag ttgcacgtct tgttagatgt gaaccagccc tacagactaa 9000
   aggacgacag cataataaat attacaaagc acaaaattag gaacggagga ttgtcccctc 9060
   gtcaaattaa gatcaggtct ctgggtaagg ctcttcaacg cacaataaag gatttagacc 9120
   gatacacctt tgaaccgtac ccaacctact ctcaggaatt acttaggctt gatataccag 9180
   agatatgtga caaaatccga tccgtcttcg cggtctcgga tcggctgacc agggagttat 9240
   ctagtgggtt ccaggatctt tggttgaata tcttcaagca actaggcaat atagaaggaa 9300
   gagaggggta cgatccgttg caggatatcg gcaccatccc ggagataact gataaataca 9360
   gcaggaatag atggtatagg ccattcctaa cttggttcag catcaaatat gacatgcggt 9420
   ggatgcagaa gaccagaccg gggggacccc tcgatacctc taattcacat aacctcctag 9480
   aatgcaaatc atacactcta gtaacatacg gagatcttat catgatactg aacaagttga 9540
   cattgacagg gtatatccta acccctgagc tggtcttgat gtattgtgat gttgtagagg 9600
   gaaggtggaa tatgtctgct gcagggcatc tagataagaa gtccattggg ataacaagca 9660
   aaggtgagga attatgggaa ctagtggatt ccctcttctc aagtcttgga gaggaaatat 9720
   acaatgtcat cgcactattg gagcccctat cacttgctct catacaacta aatgatccag 9780
   ttatacctct acgtggggca tttatgaggc atgtgttgac agagctacag gctgttttaa 9840
   caagtaggga cgtgtacaca gatgctgaag cagacactat tgtggagtcg ttactcgcca 9900
   ttttccatgg aacctctatt gatgagaaag cagagatctt ttccttcttt aggacatttg 9960
   gccaccccag cttagaggct gtcactgccg ccgacaaggt aagggcccat atgtatgcac 10020
   aaaaggcaat aaagcttaag accctatacg agtgtcatgc agttttttgc actatcatca 10080
   taaatgggta tagagagagg catggcggac agtggccccc ctgtgacttc cctgatcacg 10140
   tgtgtctaga actaaggaac gctcaagggt ccaatacggc aatctcttat gaatgtgctg 10200
   tagacaacta tacaagtttc ataggcttca agtttcggaa gtttatagaa ccacaactag 10260
   atgaagatct cacaatatat atgaaagaca aagcactatc ccccaggaag gaggcatggg 10320
   actctgtata cccggatagt aatctgtact ataaagcccc agaatctgaa gagacccggc 10380
   ggcttattga agtgttcata aatgatgaga atttcaaccc agaagaaatt atcaattatg 10440
   tggagtcagg agattggttg aaagacgaga agttcaacat ctcgtacagt ctcaaagaga 10500
   aagagatcaa gcaagagggt cgtctattcg caaaaatgac ttataagatg cgagccgtac 10560
   aggtgctggc agagacacta ctggctaaag gaataggaga gctgttcagc gaaaatggga 10620
   tggttaaagg agagatagac ctacttaaaa gattgactac tctttctgtc tcaggagtcc 10680
   ccaggactga ttcagtgtac aataactcta aatcatcaga gaagagaaac gaaggcatga 10740
   aaaagaagaa ctctgggggg tactgggacg aaaagaagag gtccagacat gaattcaagg 10800
   caacagattc atcaacagac ggctatgaaa cgttaagttg cttcctcaca acagacctca 10860
   agaaatactg cttaaactgg agatttgaaa gtactgcatt gtttggtcag agatgcaacg 10920
   agatatttgg cttcaagacc ttctttaact ggatgcatcc agtccttgaa aggtgtacaa 10980
   tatatgttgg ggatccttac tgtccagtcg ccgaccggat gcatcgacaa ctccaggatc 11040
   atgcagactc tggcattttc atacataatc ctaggggggg catagaaggt tactgccaga 11100
   agctgtggac cttaatctca atcagtgcaa tccacctagc agctgtgaga gtgggtgtca 11160
   gggtctctgc aatggttcag ggtgacaatc aagctatagc cgtgacatca agagtacctg 11220
   tagctcagac ttacaagcag aagaaaaatc atgtctataa ggagatcacc aaatattttg 11280
   gtgctctaag acacgtcatg tttgatgtag ggcacgagct aaaattgaac gagaccatca 11340
   ttagtagcaa gatgtttgtc tatagtaaaa gaatatacta tgatgggaag attttaccac 11400
   agtgcctgaa agccttgacc aggtgtgtat tctggtccga gacactggta gatgaaaaca 11460
   gatctgcttg ttcgaacatc tcaacatcca tagcaaaagc tatcgaaaat gggtattctc 11520
   ctatactagg ctactgcatt gcgttgtata agacctgtca gcaggtgtgc atatcactag 11580
   ggatgactat aaatccaact atcagcccga ccgtaagaga tcaatacttt aagggtaaga 11640
   attggctgag atgtgcagtg ttgattccag caaatgttgg aggattcaac tacatgtcta 11700
   catctagatg ctttgttaga aatattggag accccgcagt agcagcccta gctgatctca 11760
   aaagattcat cagagcggat ctgttagaca agcaggtact atacagggtc atgaatcaag 11820
   aacccggtga ctctagcttt ctagattggg cttcagaccc ttattcatgt aacctcccgc 11880
   attctcagag tataactacg attataaaga atatcactgc tagatctgtg ctgcaggaat 11940
   ccccgaatcc tctactgtct ggtctcttca ccgagactag tggagaagag gatctcaacc 12000
   tggcctcgtt ccttatggac cggaaagtca tcctgccgag agtggctcat gagatcctgg 12060
   gtaattcctt aactggagtt agggaggcga ttgcagggat gcttgatacg accaagtctc 12120
   tagtgagatc cagcgttaag aaaggaggat tatcatatgg gatattgagg aggcttgtca 12180
   attatgatct attgcagtac gagacactga ctagaactct caggaaaccg gtgaaagaca 12240
   acatcgaata tgagtatatg tgttcagttg agctagctgt cggtctaagg cagaaaatgt 12300
   ggatccacct aacttacggg agacccatac atgggctaga aacaccagac cctttagagc 12360
   tcttgagggg aacatttatc gaaggttcag aggtgtgcaa gctttgcagg tctgagggag 12420
   cagaccccat ctatacatgg ttctatctcc ctgacaatat agacctggac acgcttacaa 12480
   acggatgtcc ggctataaga atcccctatt ttggatcagc cactgatgaa aggtcggaag 12540
   cccaactcgg gtatgtaaga aatctaagca aacccgcaaa ggctgccatc cggatagcta 12600
   tggtgtatac gtgggcctac gggactgatg agatatcgtg gatggaagcc gctcttatag 12660
   cccaaacaag agctaatctg agcttagaga atctaaagct gctgactcct gtttcaacct 12720
   ccactaatct atctcatagg ttgaaagata cggcaaccca gatgaagttc tctagtgcaa 12780
   cactagtccg tgcaagtcgg ttcataacaa tatcaaatga taacatggca ctcaaagaag 12840
   caggggagtc gaaggatact aatctcgtgt atcagcagat tatgctaact gggctaagct 12900
   tgttcgagtt caatatgaga tataagaaag gttccttagg gaagccactg atattgcact 12960
   tacatcttaa taacgggtgc tgtataatgg agtccccaca ggaggcgaat atccccccaa 13020
   ggtccacatt agatttagag attacacaag agaacaataa attgatctat gatcctgatc 13080
   cactcaagga tgtggacctt gagctattta gcaaggtcag agatgttgta catacagttg 13140
   acatgactta ttggtcagat gatgaagtta tcagagcaac cagtatctgt actgcaatga 13200
   cgatagctga tacaatgtct caattagata gagacaacct aaaagagatg atcgcgctag 13260
   taaatgacga tgatgtcaac agcctgatta ctgagtttat ggtgattgat gttcctttat 13320
   tttgctcaac gttcgggggt attctagtca atcagtttgc atactcactc tacggcttaa 13380
   acatcagagg aagggaagaa atatggggac atgtagtccg gattcttaaa gatacctccc 13440
   acgcagttct aaaagtctta tctaatgctc tatctcatcc caaaatcttc aaacgattct 13500
   ggaatgcagg tgtcgtggaa cctgtgtatg ggcctaacct ctcaaatcag gacaagatac 13560
   tcttggccct ctctgtctgt gaatattctg tggatctatt catgcacgat tggcaagggg 13620
   gtgtaccgct tgagatcttt atctgtgaca atgacccaga tgtggccgac atgaggaggt 13680
   cctctttctt ggcaagacat cttgcatacc tatgcagcgt ggcagagata tctagggatg 13740
   ggccaagatt agaatcaatg aactctctag agaggctcga gtcactaaag agttacctgg 13800
   aactcacatt tcttgatgac ccggtactga ggtacagtca gttgactggc ctagtcatca 13860
   aagtattccc atctactttg acctatatcc ggaagtcatc tataaaagtg ttaaggacaa 13920
   gaggtatagg agtccctgaa gtcttagaag attgggatcc cgaggcagat aatgcactgt 13980
   tagatggtat cgcggcagaa atacaacaga atattccttt gggacatcag actagagccc 14040
   ctttttgggg gttgagagta tccaagtcac aggtactgcg tctccggggg tacaaggaga 14100
   tcacaagagg tgagataggc agatcaggcg ttggtctgac gttaccattc gatggaagat 14160
   atctatctca ccagctgagg ctctttggca tcaacagtac tagctgcttg aaagcacttg 14220
   aacttaccta cctattgagc cccttagttg acaaggataa agataggcta tatttagggg 14280
   aaggagctgg ggccatgctt tcctgttatg acgctactct tggcccatgc atcaactatt 14340
   ataactcagg ggtatactct tgtgatgtca atgggcagag agagttaaat atatatcctg 14400
   ctgaggtggc actggtggga aagaaattaa acaatgttac tagtctgggt caaagagtta 14460
   aagtgttatt caacgggaat cctggctcga catggattgg aaatgatgag tgtgaggctt 14520
   tgatttggaa tgaattgcag aatagctcga taggcctagt ccactgtgac atggagggag 14580
   gagatcataa ggatgatcaa gttgtactgc atgagcatta cagtgtaatc cggatcgcgt 14640
   atctggtggg ggatcgagac gttgtgctta taagcaagat tgctcctagg ctgggcacgg 14700
   attggaccag gcagctcagc ctatatctga gatactggga cgaggttaac ctaatagtgc 14760
   ttaaaacatc taaccctgct tccacagaga tgtatctcct atcgaggcat cccaaatctg 14820
   acattataga ggacagcaag acggtgttag ctagtctcct ccctttgtca aaagaagata 14880
   gcatcaagat agaaaagtgg atcttaatag agaaggcaaa ggctcacgaa tgggttactc 14940
   gggaattgag agaaggaagc tcttcatcag ggatgcttag accttaccat caagcactgc 15000
   agacgtttgg ctttgaacca aacttgtata aattgagcag agatttcttg tccaccatga 15060
   acatagctga tacacacaac tgcatgatag ctttcaacag ggttttgaag gatacaatct 15120
   tcgaatgggc tagaataact gagtcagata aaaggcttaa actaactggt aagtatgacc 15180
   tgtatcctgt gagagattca ggcaaattga agacagtttc tagaagactt gtgctatctt 15240
   ggatatcttt atctatgtcc acaagattgg taactgggtc attccctgac cagaagtttg 15300
   aagcaagact tcaattggga atagtttcat tatcatcccg tgaaatcagg aacctgaggg 15360
   ttatcacaaa aactttatta gaccggtttg aggatattat acatagtata acgtacagat 15420
   tcctcaccaa agaaataaag attttgatga agattttagg ggcagtcaag atgttcgggg 15480
   ccaggcaaaa tgaatacacg accgtgattg atgatggatc actgggtgat atcgagccat 15540
   atgacagctc gtaataatta gtccctatcg tgcagaacga tcgaagctcc gcggtacctg 15600
   gaagtcttgg actgatccat atgacaatag taagaaaaac ttacaagaag acaagaaaat 15660
   ttaaaagaat acatatctct taaactcttg tctggt 15696
<210> 13
   <211> 39
   <212> DNA
   <213> Artificial
<220>
   <223> Primer
<400> 13
   actagctagc ttagacgctg gatttttttc gggtagtgg 39
<210> 14
   <211> 30
   <212> DNA
   <213> Artificial
<220>
   <223> Primer
<400> 14
   gtccaccggt cttacgcaca agagttccgt 30
<210> 15
   <211> 35
   <212> DNA
   <213> Artificial
<220>
   <223> Prmer
<400> 15
   agtacctagg cgcatgtaca acatgatgga gacgg 35
<210> 16
   <211> 33
   <212> DNA
   <213> Artificial
<220>
   <223> Primer
<400> 16
   gtccgacgtc ctcacatgtg tgagaggggc agt 33
<210> 17
   <211> 40
   <212> DNA
   <213> Artificial
<220>
   <223> Primer
<400> 17
   actagctagc ggttccccat ggcgggacac ctggcttcgg 40
<210> 18
   <211> 35
   <212> DNA
   <213> Artificial
<220>
   <223> Primer
<400> 18
   ggtccacgcg ttcagtttga atgcatggga gagcc 35
<210> 19
   <211> 15702
   <212> DNA
   <213> Artificial
<220>
   <223> Full length genome cDNA of Sendai virus vector-version2
<400> 19
   accaaacaag agaagaaaca tgtatggaat atataatgaa gttagacagg attttagggt      60
   caaagtatcc accctgagga gcaggttcca gatccttttc tttgctgcca aagttcacga     120
   tggccgggtt gttgagcacc ttcgatacat ttagctctag gaggagcgaa agtattaata     180
   agtcgggagg aggtgctgtt atccccggcc agaggagcac agtctcagtg ttcatactag     240
   gcccaagtgt gactgatgat gcagacaagt tattcattgc aacaaccttc ctagctcact     300
   cattggacac agataagcag cactctcaga gaggagggtt cctcgtctct ctgcttgcca     360
   tggcttacag tagtccagaa ttgtacttga caacaaacgg agtaaacgcc gatgtcaaat     420
   atgtgatcta caacatagag aaagacccta agaggacgaa gacagacgga ttcattgtga     480
   agacgagaga tatggaatat gagaggacca cagaatggct gtttggacct atggtcaaca     540
   agagcccact cttccagggt caacgggatg ctgcagaccc tgacacactc cttcaaatct     600
   atgggtatcc tgcatgccta ggagcaataa ttgtccaagt ctggattgtg ctggtgaagg     660
   ccatcacaag cagcgccggc ttaaggaaag ggttcttcaa caggttagag gcgttcagac     720
   aagacggcac cgtgaaaggt gccttagttt tcactgggga gacagttgag gggataggct     780
   cggttatgag atctcagcaa agccttgtat ctctcatggt tgagaccctt gtgactatga     840
   atactgcaag atctgatctc accacattag agaagaacat ccagatcgtt gggaactaca     900
   tccgagatgc agggctggct tccttcatga acactattaa atatggggtg gagacaaaga     960
   tggcagctct aacgttgtca aacctgaggc ccgatattaa taagcttaga agcctcatag    1020
   acacctacct gtcaaaaggc cccagagctc cctttatctg tatcctcaag gaccctgttc    1080
   atggtgaatt tgctccaggc aattatcctg cactatggag ttacgccatg ggagtcgccg    1140
   tcgtacagaa caagtcaatg cagcagtacg tcacagggag gacatacctt gatatggaaa    1200
   tgttcttact aggacaagcc gtggcaaagg atgctgaatc gaagatcagc agtgccttgg    1260
   aagatgagtt aggagtgacg gatacagcca aggagaggct cagacatcat ctggcaaact    1320
   tgtccggtgg ggatggtgct taccacaaac caacaggcgg tggtgcaatt gaggtagctc    1380
   tagacaatgc cgatatcgac ctggaaacag aagctcatgc ggaccaggac gctaggggtt    1440
   ggggtggaga tagtggtgaa agatgggcac gtcaggtgag tggtggccac tttgtcacac    1500
   tacatggggc tgaacggtta gaggaggaaa ccaatgatga ggatgtatca gacatagaga    1560
   gaagaatagc catgagactc gcagagagac ggcaagagga ttctgcaacc catggagatg    1620
   aaggccgcaa taacggtgtt gatcacgaag aagatgacga tgccgcagca gcagctggga    1680
   taggaggaat ctaggatcat acgaggcctc aaggtacttg atccgcagta agaaaaactt 1740
   agggtgaaag ttcatccacc gatcggctca ggcaaggcca cacccaaccc caccgaccac 1800
   acccagcagt cgagacagcc acggcttcgg ctacacttac cgcatggatc aagatgcctt 1860
   cattcttaaa gaagattctg aagttgagag gaaggcgcca ggaggacgag agtcgctctc   1920
   ggatgttatc ggattcctcg atgctgtcct gtcgaatgaa ccaactgaca tcggagggga   1980
   cagaagctgg ctccacaaca ccatcaacac tccccaagga ccaggctctg ctcatagagc 2040
   caaaagtgag ggcgaaggag aagtctcaac accgtcgacc caagataatc gatcaggtga 2100
   ggagagtaga gtctctggga gaacaagcaa gccagaggca gaagcacatg ctggaaacct 2160
   tgataaacaa aatatacact gggcctttag gggaagaact ggtacaaact ctgtatctca 2220
   ggatctggac gatggaggag actccggaat ccttgaaaat cctccaaatg agagaggata 2280
   tccgagatca ggtattgaag atgaaaacag agagatggct gcgcaccctg ataagagggg 2340
   agaagaccaa gctgaaggac ttccagaaga ggtacgagga ggtacatccc tacctgatga 2400
   aggagaaggt ggagcaagta ataatggaag aagcatggag cctggcagct cacatagtgc 2460
   aagagtaact ggggtcctgg tgattcctag ccccgaactt gaagaggctg tgctacggag 2520
   gaacaaaaga agacctacca acagtgggtc caaacctctt actccagcaa ccgtgcctgg 2580
   cacccggtcc ccaccgctga atcgttacaa cagcacaggg tcaccaccag gaaaaccccc 2640
   atctacacag gatgagcaca tcaactctgg ggacaccccc gccgtcaggg tcaaagaccg 2700
   gaaaccatca atagggactc gctctgtctc agattgtcca gccaacggcc gcccaatcca 2760
   tccgggtata gagaccgact caacaaaaaa gggcatagga gagaacacat catctatgaa 2820
   agatatggct acattgttga cgagtcttgg tgtaatccag tctgctcaag aattcgagtc 2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcacacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcacctaggt ctgacatggc tgtcagcgac gcgctgctcc catctttctc cacgttcgcg 3720
   tctggcccgg cgggaaggga gaagacactg cgtcaagcag gtgccccgaa taaccgctgg 3780
   cgggaggagc tctcccacat gaagcgactt cccccagtgc ttcccggccg cccctatgac 3840
   ctggcggcgg cgaccgtggc cacagacctg gagagcggcg gagccggtgc ggcttgcggc 3900
   ggtagcaacc tggcgcccct acctcggaga gagaccgagg agttcaacga tctcctggac 3960
   ctggacttta ttctctccaa ttcgctgacc catcctccgg agtcagtggc cgccaccgtg 4020
   tcctcgtcag cgtcagcctc ctcttcgtcg tcgccgtcga gcagcggccc tgccagcgcg 4080
   ccctccacct gcagcttcac ctatccgatc cgggccggga acgacccggg cgtggcgccg 4140
   ggcggcacgg gcggaggcct cctctatggc agggagtccg ctccccctcc gacggctccc 4200
   ttcaacctgg cggacatcaa cgacgtgagc ccctcgggcg gcttcgtggc cgagctcctg 4260
   cggccagaat tggacccggt gtacattccg ccgcagcagc cgcagccgcc aggtggcggg 4320
   ctgatgggca agttcgtgct gaaggcgtcg ctgagcgccc ctggcagcga gtacggcagc 4380
   ccgtcggtca tcagcgtcag caaaggcagc cctgacggca gccacccggt ggtggtggcg 4440
   ccctacaacg gcgggccgcc gcgcacgtgc cccaagatca agcaggaggc ggtctcttcg 4500
   tgcacccact tgggcgctgg accccctctc agcaatggcc accggccggc tgcacacgac 4560
   ttccccctgg ggcggcagct ccccagcagg actaccccga ccctgggtct tgaggaagtg 4620
   ctgagcagca gggactgtca ccctgccctg ccgcttcctc ccggcttcca tccccacccg 4680
   gggcccaatt acccatcctt cctgcccgat cagatgcagc cgcaagtccc gccgctccat 4740
   taccaagagc tcatgccacc cggttcctgc atgccagagg agcccaagcc aaagagggga 4800
   agacgatcgt ggccccggaa aaggaccgcc acccacactt gtgattacgc gggctgcggc 4860
   aaaacctaca caaagagttc ccatctcaag gcacacctgc gaacccacac aggtgagaaa 4920
   ccttaccact gtgactggga cggctgtgga tggaaattcg cccgctcaga tgaactgacc 4980
   aggcactacc gtaaacacac ggggcaccgc ccgttccagt gccaaaaatg cgaccgagca 5040
   ttttccaggt cggaccacct cgccttacac atgaagaggc atttttaaag acgtcgatta 5100
   agaaaaactt agggtgaaag ttcatcgcgg ccgcttgcta gcggttcccc atggcgggac 5160
   acctggcttc ggatttcgcc ttctcgcccc ctccaggtgg tggaggtgat gggccagggg 5220
   ggccggagcc gggctgggtt gatcctcgga cctggctaag cttccaaggc cctcctggag 5280
   ggccaggaat cgggccgggg gttgggccag gctctgaggt gtgggggatt cccccatgcc 5340
   ccccgccgta tgagttctgt ggggggatgg cgtactgtgg gccccaggtt ggagtggggc 5400
   tagtgcccca aggcggcttg gagacctctc agcctgaggg cgaagcagga gtcggggtgg 5460
   agagcaactc cgatggggcc tccccggagc cctgcaccgt cacccctggt gccgtgaagc 5520
   tggagaagga gaagctggag caaaacccgg aggagtccca ggacatcaaa gctctgcaga 5580
   aagaactcga gcaatttgcc aagctcctga agcagaagag gatcaccctg ggatatacac 5640
   aggccgatgt ggggctcacc ctgggggttc tatttgggaa ggtattcagc caaacgacca 5700
   tctgccgctt tgaggctctg cagcttagct tcaagaacat gtgtaagctg cggcccttgc 5760
   tgcagaagtg ggtggaggaa gctgacaaca atgaaaatct tcaggagata tgcaaagcag 5820
   aaaccctcgt gcaggcccga aagagaaagc gaaccagtat cgagaaccga gtgagaggca 5880
   acctggagaa tttgttcctg cagtgcccga aacccacact gcagcagatc agccacatcg 5940
   cccagcagct tgggctcgag aaggatgtgg tccgagtgtg gttctgtaac cggcgccaga 6000
   agggcaagcg atcaagcagc gactatgcac aacgagagga ttttgaggct gctgggtctc 6060
   ctttctcagg gggaccagtg tcctttcctc tggccccagg gccccatttt ggtaccccag 6120
   gctatgggag ccctcacttc actgcactgt actcctcggt ccctttccct gagggggaag 6180
   cctttccccc tgtctccgtc accactctgg gctctcccat gcattcaaac tgaacgcgtc 6240
   agagacctgc aacaatgtct caagcagaca ccacctggca gtcggagcca ccgggtcact 6300
   ccttgtctta aataagaaaa acttagggat aaagtccctt agatctagcc taggcgcatg 6360
   tacaacatga tggagacgga gctgaagccg ccgggcccgc agcaaacttc ggggggcggc 6420
   ggcggcaact ccaccgcggc ggcggccggc ggcaaccaga aaaacagccc ggaccgcgtc 6480
   aagcggccca tgaatgcctt catggtgtgg tcccgcgggc agcggcgcaa gatggcccag 6540
   gagaacccca agatgcacaa ctcggagatc agcaagcgcc tgggcgccga gtggaaactt 6600
   ttgtcggaga cggagaagcg gccgttcatc gacgaggcta agcggctgcg agcgctgcac 6660
   atgaaggagc acccggatta taaataccgg ccccggcgga aaaccaagac gctcatgaag 6720
   aaggataagt acacgctgcc cggcgggctg ctggcccccg gcggcaatag catggcgagc 6780
   ggggtcgggg tgggcgccgg cctgggcgcg ggcgtgaacc agcgcatgga cagttacgcg 6840
   cacatgaacg gctggagcaa cggcagctac agcatgatgc aggaccagct gggctacccg 6900
   cagcacccgg gcctcaatgc gcacggcgca gcgcagatgc agcccatgca ccgctacgac 6960
   gtgagcgccc tgcagtacaa ctccatgacc agctcgcaga cctacatgaa cggctcgccc 7020
   acctacagca tgtcctactc gcagcagggc acccctggca tggctcttgg ctccatgggt 7080
   tcggtggtca agtccgaggc cagctccagc ccccctgtgg ttacctcttc ctcccactcc 7140
   agggcgccct gccaggccgg ggacctccgg gacatgatca gcatgtatct ccccggcgcc 7200
   gaggtgccgg aacccgccgc ccccagcaga cttcacatgt cccagcacta ccagagcggc 7260
   ccggtgcccg gcacggccat taacggcaca ctgcccctct cacacatgtg aggacgtcag 7320
   atctgtatat aataagaaaa acttagggtg aaagtgaggt tgcgcggtat tttagctagc 7380
   ttagacgctg gatttttttc gggtagtgga aaaccagcag cctcccgcga cgatgcccct 7440
   caacgttagc ttcaccaaca ggaactatga cctcgactac gactcggtgc agccgtattt 7500
   ctactgcgac gaggaggaga acttctacca gcagcagcag cagagcgagc tgcagccccc 7560
   ggcgcccagc gaggatatct ggaagaaatt cgagctgctg cccaccccgc ccctgtcccc 7620
   tagccgccgc tccgggctct gctcgccctc ctacgttgcg gtcacaccct tctcccttcg 7680
   gggagacaac gacggcggtg gcgggagctt ctccacggcc gaccagctgg agatggtgac 7740
   cgagctgctg ggaggagaca tggtgaacca gagtttcatc tgcgacccgg acgacgagac 7800
   cttcatcaaa aacatcatca tccaggactg tatgtggagc ggcttctcgg ccgccgccaa 7860
   gctcgtctca gagaagctgg cctcctacca ggctgcgcgc aaagacagcg gcagcccgaa 7920
   ccccgcccgc ggccacagcg tctgctccac ctccagcttg tacctgcagg atctgagcgc 7980
   cgccgcctca gagtgcatcg acccctcggt ggtcttcccc taccctctca acgacagcag 8040
   ctcgcccaag tcctgcgcct cgcaagactc cagcgccttc tctccgtcct cggattctct 8100
   gctctcctcg acggagtcct ccccgcaggg cagccccgag cccctggtgc tccatgagga 8160
   gacaccgccc accaccagca gcgactctga ggaggaacaa gaagatgagg aagaaatcga 8220
   tgttgtttct gtggaaaaga ggcaggctcc tggcaaaagg tcagagtctg gatcaccttc 8280
   tgctggaggc cacagcaaac ctcctcacag cccactggtc ctcaagaggt gccacgtctc 8340
   cacacatcag cacaactacg cagcgcctcc ctccactcgg aaggactatc ctgctgccaa 8400
   gagggtcaag ttggacagtg tcagagtcct gagacagatc agcaacaacc gaaaatgcac 8460
   cagccccagg tcctcggaca ccgaggagaa tgtcaagagg cgaacacaca acgtcttgga 8520
   gcgccagagg aggaacgagc taaaacggag cttttttgcc ctgcgtgacc agatcccgga 8580
   gttggaaaac aatgaaaagg cccccaaggt agttatcctt aaaaaagcca cagcatacat 8640
   cctgtccgtc caagcagagg agcaaaagct catttctgaa gaggacttgt tgcggaaacg 8700
   acgagaacag ttgaaacaca aacttgaaca gctacggaac tcttgtgcgt aagaccggtg 8760
   tcggctttgc tgacactaga gtcatctccg aacatccaca atatctctca gtctcttacg 8820
   tctctcacag tattaagaaa aacccagggt gaatgggaag cttgccatag gtcatggatg 8880
   ggcaggagtc ctcccaaaac ccttctgaca tactctatcc agaatgccac ctgaactctc 8940
   ccatagtcag ggggaagata gcacagttgc acgtcttgtt agatgtgaac cagccctaca 9000
   gactaaagga cgacagcata ataaatatta caaagcacaa aattaggaac ggaggattgt 9060
   cccctcgtca aattaagatc aggtctctgg gtaaggctct tcaacgcaca ataaaggatt 9120
   tagaccgata cacctttgaa ccgtacccaa cctactctca ggaattactt aggcttgata 9180
   taccagagat atgtgacaaa atccgatccg tcttcgcggt ctcggatcgg ctgaccaggg 9240
   agttatctag tgggttccag gatctttggt tgaatatctt caagcaacta ggcaatatag 9300
   aaggaagaga ggggtacgat ccgttgcagg atatcggcac catcccggag ataactgata 9360
   aatacagcag gaatagatgg tataggccat tcctaacttg gttcagcatc aaatatgaca 9420
   tgcggtggat gcagaagacc agaccggggg gacccctcga tacctctaat tcacataacc 9480
   tcctagaatg caaatcatac actctagtaa catacggaga tcttatcatg atactgaaca 9540
   agttgacatt gacagggtat atcctaaccc ctgagctggt cttgatgtat tgtgatgttg 9600
   tagagggaag gtggaatatg tctgctgcag ggcatctaga taagaagtcc attgggataa 9660
   caagcaaagg tgaggaatta tgggaactag tggattccct cttctcaagt cttggagagg 9720
   aaatatacaa tgtcatcgca ctattggagc ccctatcact tgctctcata caactaaatg 9780
   atccagttat acctctacgt ggggcattta tgaggcatgt gttgacagag ctacaggctg 9840
   ttttaacaag tagggacgtg tacacagatg ctgaagcaga cactattgtg gagtcgttac 9900
   tcgccatttt ccatggaacc tctattgatg agaaagcaga gatcttttcc ttctttagga 9960
   catttggcca ccccagctta gaggctgtca ctgccgccga caaggtaagg gcccatatgt 10020
   atgcacaaaa ggcaataaag cttaagaccc tatacgagtg tcatgcagtt ttttgcacta 10080
   tcatcataaa tgggtataga gagaggcatg gcggacagtg gcccccctgt gacttccctg 10140
   atcacgtgtg tctagaacta aggaacgctc aagggtccaa tacggcaatc tcttatgaat 10200
   gtgctgtaga caactataca agtttcatag gcttcaagtt tcggaagttt atagaaccac 10260
   aactagatga agatctcaca atatatatga aagacaaagc actatccccc aggaaggagg  10320
   catgggactc tgtatacccg gatagtaatc tgtactataa agccccagaa tctgaagaga  10380
   cccggcggct tattgaagtg ttcataaatg atgagaattt caacccagaa gaaattatca 10440
   attatgtgga gtcaggagat tggttgaaag acgagaagtt caacatctcg tacagtctca 10500
   aagagaaaga gatcaagcaa gagggtcgtc tattcgcaaa aatgacttat aagatgcgag 10560
   ccgtacaggt gctggcagag acactactgg ctaaaggaat aggagagctg ttcagcgaaa 10620
   atgggatggt taaaggagag atagacctac ttaaaagatt gactactctt tctgtctcag 10680
   gagtccccag gactgattca gtgtacaata actctaaatc atcagagaag agaaacgaag 10740
   gcatgaaaaa gaagaactct ggggggtact gggacgaaaa gaagaggtcc agacatgaat 10800
   tcaaggcaac agattcatca acagacggct atgaaacgtt aagttgcttc ctcacaacag 10860
   acctcaagaa atactgctta aactggagat ttgaaagtac tgcattgttt ggtcagagat 10920
   gcaacgagat atttggcttc aagaccttct ttaactggat gcatccagtc cttgaaaggt 10980
   gtacaatata tgttggggat ccttactgtc cagtcgccga ccggatgcat cgacaactcc 11040
   aggatcatgc agactctggc attttcatac ataatcctag ggggggcata gaaggttact 11100
   gccagaagct gtggacctta atctcaatca gtgcaatcca cctagcagct gtgagagtgg 11160
   gtgtcagggt ctctgcaatg gttcagggtg acaatcaagc tatagccgtg acatcaagag 11220
   tacctgtagc tcagacttac aagcagaaga aaaatcatgt ctataaggag atcaccaaat 11280
   attttggtgc tctaagacac gtcatgtttg atgtagggca cgagctaaaa ttgaacgaga 11340
   ccatcattag tagcaagatg tttgtctata gtaaaagaat atactatgat gggaagattt 11400
   taccacagtg cctgaaagcc ttgaccaggt gtgtattctg gtccgagaca ctggtagatg 11460
   aaaacagatc tgcttgttcg aacatctcaa catccatagc aaaagctatc gaaaatgggt 11520
   attctcctat actaggctac tgcattgcgt tgtataagac ctgtcagcag gtgtgcatat 11580
   cactagggat gactataaat ccaactatca gcccgaccgt aagagatcaa tactttaagg 11640
   gtaagaattg gctgagatgt gcagtgttga ttccagcaaa tgttggagga ttcaactaca 11700
   tgtctacatc tagatgcttt gttagaaata ttggagaccc cgcagtagca gccctagctg 11760
   atctcaaaag attcatcaga gcggatctgt tagacaagca ggtactatac agggtcatga 11820
   atcaagaacc cggtgactct agctttctag attgggcttc agacccttat tcatgtaacc 11880
   tcccgcattc tcagagtata actacgatta taaagaatat cactgctaga tctgtgctgc 11940
   aggaatcccc gaatcctcta ctgtctggtc tcttcaccga gactagtgga gaagaggatc 12000
   tcaacctggc ctcgttcctt atggaccgga aagtcatcct gccgagagtg gctcatgaga 12060
   tcctgggtaa ttccttaact ggagttaggg aggcgattgc agggatgctt gatacgacca 12120
   agtctctagt gagatccagc gttaagaaag gaggattatc atatgggata ttgaggaggc 12180
   ttgtcaatta tgatctattg cagtacgaga cactgactag aactctcagg aaaccggtga 12240
   aagacaacat cgaatatgag tatatgtgtt cagttgagct agctgtcggt ctaaggcaga 12300
   aaatgtggat ccacctaact tacgggagac ccatacatgg gctagaaaca ccagaccctt 12360
   tagagctctt gaggggaaca tttatcgaag gttcagaggt gtgcaagctt tgcaggtctg 12420
   agggagcaga ccccatctat acatggttct atctccctga caatatagac ctggacacgc 12480
   ttacaaacgg atgtccggct ataagaatcc cctattttgg atcagccact gatgaaaggt 12540
   cggaagccca actcgggtat gtaagaaatc taagcaaacc cgcaaaggct gccatccgga 12600
   tagctatggt gtatacgtgg gcctacggga ctgatgagat atcgtggatg gaagccgctc 12660
   ttatagccca aacaagagct aatctgagct tagagaatct aaagctgctg actcctgttt 12720
   caacctccac taatctatct cataggttga aagatacggc aacccagatg aagttctcta 12780
   gtgcaacact agtccgtgca agtcggttca taacaatatc aaatgataac atggcactca 12840
   aagaagcagg ggagtcgaag gatactaatc tcgtgtatca gcagattatg ctaactgggc 12900
   taagcttgtt cgagttcaat atgagatata agaaaggttc cttagggaag ccactgatat 12960
   tgcacttaca tcttaataac gggtgctgta taatggagtc cccacaggag gcgaatatcc 13020
   ccccaaggtc cacattagat ttagagatta cacaagagaa caataaattg atctatgatc 13080
   ctgatccact caaggatgtg gaccttgagc tatttagcaa ggtcagagat gttgtacata 13140
   cagttgacat gacttattgg tcagatgatg aagttatcag agcaaccagt atctgtactg 13200
   caatgacgat agctgataca atgtctcaat tagatagaga caacctaaaa gagatgatcg 13260
   cgctagtaaa tgacgatgat gtcaacagcc tgattactga gtttatggtg attgatgttc 13320
   ctttattttg ctcaacgttc gggggtattc tagtcaatca gtttgcatac tcactctacg 13380
   gcttaaacat cagaggaagg gaagaaatat ggggacatgt agtccggatt cttaaagata 13440
   cctcccacgc agttctaaaa gtcttatcta atgctctatc tcatcccaaa atcttcaaac 13500
   gattctggaa tgcaggtgtc gtggaacctg tgtatgggcc taacctctca aatcaggaca 13560
   agatactctt ggccctctct gtctgtgaat attctgtgga tctattcatg cacgattggc 13620
   aagggggtgt accgcttgag atctttatct gtgacaatga cccagatgtg gccgacatga 13680
   ggaggtcctc tttcttggca agacatcttg catacctatg cagcgtggca gagatatcta 13740
   gggatgggcc aagattagaa tcaatgaact ctctagagag gctcgagtca ctaaagagtt 13800
   acctggaact cacatttctt gatgacccgg tactgaggta cagtcagttg actggcctag 13860
   tcatcaaagt attcccatct actttgacct atatccggaa gtcatctata aaagtgttaa 13920
   ggacaagagg tataggagtc cctgaagtct tagaagattg ggatcccgag gcagataatg 13980
   cactgttaga tggtatcgcg gcagaaatac aacagaatat tcctttggga catcagacta 14040
   gagccccttt ttgggggttg agagtatcca agtcacaggt actgcgtctc cgggggtaca 14100
   aggagatcac aagaggtgag ataggcagat caggcgttgg tctgacgtta ccattcgatg 14160
   gaagatatct atctcaccag ctgaggctct ttggcatcaa cagtactagc tgcttgaaag 14220
   cacttgaact tacctaccta ttgagcccct tagttgacaa ggataaagat aggctatatt 14280
   taggggaagg agctggggcc atgctttcct gttatgacgc tactcttggc ccatgcatca 14340
   actattataa ctcaggggta tactcttgtg atgtcaatgg gcagagagag ttaaatatat 14400
   atcctgctga ggtggcactg gtgggaaaga aattaaacaa tgttactagt ctgggtcaaa 14460
   gagttaaagt gttattcaac gggaatcctg gctcgacatg gattggaaat gatgagtgtg 14520
   aggctttgat ttggaatgaa ttgcagaata gctcgatagg cctagtccac tgtgacatgg 14580
   agggaggaga tcataaggat gatcaagttg tactgcatga gcattacagt gtaatccgga 14640
   tcgcgtatct ggtgggggat cgagacgttg tgcttataag caagattgct cctaggctgg 14700
   gcacggattg gaccaggcag ctcagcctat atctgagata ctgggacgag gttaacctaa 14760
   tagtgcttaa aacatctaac cctgcttcca cagagatgta tctcctatcg aggcatccca 14820
   aatctgacat tatagaggac agcaagacgg tgttagctag tctcctccct ttgtcaaaag 14880
   aagatagcat caagatagaa aagtggatct taatagagaa ggcaaaggct cacgaatggg 14940
   ttactcggga attgagagaa ggaagctctt catcagggat gcttagacct taccatcaag 15000
   cactgcagac gtttggcttt gaaccaaact tgtataaatt gagcagagat ttcttgtcca 15060
   ccatgaacat agctgataca cacaactgca tgatagcttt caacagggtt ttgaaggata 15120
   caatcttcga atgggctaga ataactgagt cagataaaag gcttaaacta actggtaagt 15180
   atgacctgta tcctgtgaga gattcaggca aattgaagac agtttctaga agacttgtgc 15240
   tatcttggat atctttatct atgtccacaa gattggtaac tgggtcattc cctgaccaga 15300
   agtttgaagc aagacttcaa ttgggaatag tttcattatc atcccgtgaa atcaggaacc 15360
   tgagggttat cacaaaaact ttattagacc ggtttgagga tattatacat agtataacgt 15420
   acagattcct caccaaagaa ataaagattt tgatgaagat tttaggggca gtcaagatgt 15480
   tcggggccag gcaaaatgaa tacacgaccg tgattgatga tggatcactg ggtgatatcg 15540
   agccatatga cagctcgtaa taattagtcc ctatcgtgca gaacgatcga agctccgcgg 15600
   tacctggaag tcttggactg atccatatga caatagtaag aaaaacttac aagaagacaa 15660
   gaaaatttaa aagaatacat atctcttaaa ctcttgtctg gt 15702
<210> 20
   <211> 63
   <212> DNA
   <213> Artificial
<220>
   <223> Oligo DNA for introducing target sequence of mir-302a
<400> 20
   ccggttatca ccaaaacatg gaagcactta cgattcacca aaacatggaa gcacttaggt 60
   acc 63
<210> 21
   <211> 53
   <212> DNA
   <213> Artificial
<220>
   <223> Oligo DNA for introducing target sequence of mir-302a
<400> 21
   taagtgcttc catgttttgg tgaatcgtaa gtgcttccat gttttggtga taa 53
<210> 22
   <211> 51
   <212> DNA
   <213> Artificial
<220>
   <223> Oligo DNA for introducing target sequence of mir-302a
<400> 22
   tcaccaaaac atggaagcac ttacgattca ccaaaacatg gaagcactta a 51
<210> 23
   <211> 61
   <212> DNA
   <213> Artificial
<220>
   <223> Oligo DNA for introducing target sequence of mir-302a
<400> 23
   ccggttaagt gcttccatgt tttggtgaat cgtaagtgct tccatgtttt ggtgaggtac 60
   c 61
<210> 24
   <211> 6576
   <212> DNA
   <213> Artificial
<220>
   <223> pNK15( vector used to making Sendai virus vector having target s equence of mir-302a)
<400> 24
   atcggcacca tcccggagat aactgataaa tacagcagga atagatggta taggccattc     60
   ctaacttggt tcagcatcaa atatgacatg cggtggatgc agaagaccag accgggggga    120
   cccctcgata cctctaattc acataacctc ctagaatgca aatcatacac tctagtaaca    180
   tacggagatc ttatcatgat actgaacaag ttgacattga cagggtatat cctaacccct    240
   gagctggtct tgatgtattg tgatgttgta gagggaaggt ggaatatgtc tgctgcaggg    300
   catctagata agaagtccat tgggataaca agcaaaggtg aggaattatg ggaactagtg    360
   gattccctct tctcaagtct tggagaggaa atatacaatg tcatcgcact attggagccc    420
   ctatcacttg ctctcataca actaaatgat ccagttatac ctctacgtgg ggcatttatg    480
   aggcatgtgt tgacagagct acaggctgtt ttaacaagta gggacgtgta cacagatgct    540
   gaagcagaca ctattgtgga gtcgttactc gccattttcc atggaacctc tattgatgag    600
   aaagcagaga tcttttcctt ctttaggaca tttggccacc ccagcttaga ggctgtcact    660
   gccgccgaca aggtaagggc ccatatgtat gcacaaaagg caataaagct taagacccta    720
   tacgagtgtc atgcagtttt ttgcactatc atcataaatg ggtatagaga gaggcatggc    780
   ggacagtggc ccccctgtga cttccctgat cacgtgtgtc tagaactaag gaacgctcaa    840
   gggtccaata cggcaatctc ttatgaatgt gctgtagaca actatacaag tttcataggc    900
   ttcaagtttc ggaagtttat agaaccacaa ctagatgaag atctcacaat atatatgaaa    960
   gacaaagcac tatcccccag gaaggaggca tgggactctg tatacccgga tagtaatctg   1020
   tactataaag ccccagaatc tgaagagacc cggcggctta ttgaagtgtt cataaatgat   1080
   gagaatttca acccagaaga aattatcaat tatgtggagt caggagattg gttgaaagac 1140
   gagaagttca acatctcgta cagtctcaaa gagaaagaga tcaagcaaga gggtcgtcta 1200
   ttcgcaaaaa tgacttataa gatgcgagcc gtacaggtgc tggcagagac actactggct 1260
   aaaggaatag gagagctgtt cagcgaaaat gggatggtta aaggagagat agacctactt 1320
   aaaagattga ctactctttc tgtctcagga gtccccagga ctgattcagt gtacaataac 1380
   tctaaatcat cagagaagag aaacgaaggc atgaaaaaga agaactctgg ggggtactgg 1440
   gacgaaaaga agaggtccag acatgaattc aaggcaacag attcatcaac agacggctat 1500
   gaaacgttaa gttgcttcct cacaacagac ctcaagaaat actgcttaaa ctggagattt 1560
   gaaagtactg cattgtttgg tcagagatgc aacgagatat ttggcttcaa gaccttcttt 1620
   aactggatgc atccagtcct tgaaaggtgt acaatatatg ttggggatcc ttactgtcca 1680
   gtcgccgacc ggatgcatcg acaactccag gatcatgcag actctggcat tttcatacat 1740
   aatcctaggg gggcatagaa aggttactgc cagaagctgt ggaccttaat ctcaatcagt 1800
   gcaatccacc tagcagctgt gagagtgggt gtcagggtct ctgcaatggt tcagggtgac 1860
   aatcaagcta tagccgtgac atcaagagta cctgtagctc agacttacaa gcagaagaaa 1920
   aatcatgtct ataaggagat caccaaatat tttggtgctc taagacacgt catgtttgat 1980
   gtagggcacg agctaaaatt gaacgagacc atcattagta gcaagatgtt tgtctatagt 2040
   aaaagaatat actatgatgg gaagatttta ccacagtgcc tgaaagcctt gaccaggtgt 2100
   gtattctggt ccgagacact ggtagatgaa aacagatctg cttgttcgaa catctcaaca 2160
   tccatagcaa aagctatcga aaatgggtat tctcctatac taggctactg cattgcgttg 2220
   tataagacct gtcagcaggt gtgcatatca ctagggatga ctataaatcc aactatcagc 2280
   ccgaccgtaa gagatcaata ctttaagggt aagaattggc tgagatgtgc agtgttgatt 2340
   ccagcaaatg ttggaggatt caactacatg tctacatcta gatgctttgt tagaaatatt 2400
   ggagaccccg cagtagcagc cctagctgat ctcaaaagat tcatcagagc ggatctgtta 2460
   gacaagcagg tactatacag ggtcatgaat caagaacccg gtgactctag ctttctagat 2520
   tgggcttcag acccttattc atgtaacctc ccgcattctc agagtataac tacgattata 2580
   aagaatatca ctgctagatc tgtgctgcag gaatccccga atcctctact gtctggtctc 2640
   ttcaccgaga ctagtggaga agaggatctc aacctggcct cgttccttat ggaccggaaa 2700
   gtcatcctgc cgagagtggc tcatgagatc ctgggtaatt ccttaactgg agttagggag 2760
   gcgattgcag ggatgcttga tacgaccaag tctctagtga gatccagcgt taagaaagga 2820
   ggattatcat atgggatatt gaggaggctt gtcaattatg atctattgca gtacgagaca 2880
   ctgactagaa ctctcaggaa accggtgaaa gacaacatcg aatatgagta tatgtgttca 2940
   gttgagctag ctgtcggtct aaggcagaaa atgtggatcc acctaactta cgggagaccc 3000
   atacatgggc tagaaacacc agacccttta gagctcttga ggggaacatt tatcgaaggt 3060
   tcagaggtgt gcaagctttg caggtctgag ggagcagacc ccatctatac atggttctat 3120
   ctccctgaca atatagacct ggacacgctt acaaacggat gtccggctat aagaatcccc 3180
   tattttggat cagccactga tgaaaggtcg gaagcccaac tcgggtatgt aagaaatcta 3240
   agcaaacccg caaaggctgc catccggata gctatggtgt atacgtgggc ctacgggact 3300
   gatgagatat cgtggatgga agccgctctt atagcccaaa caagagctaa tctgagctta 3360
   gagaatctaa agctgctgac tcctgtttca acctccacta atctatctca taggttgaaa 3420
   gatacggcaa cccagatgaa gttctctagt gcaacactag tccgtgcaag tcggttcata 3480
   acaatatcaa atgataacat ggcactcaaa gaagcagggg agtcgaagga tactaatctc 3540
   gtgtatcagc agattatgct aactgggcta agcttgttcg agttcaatat gagatataag 3600
   aaaggttcct tagggaagcc actgatattg cacttacatc ttaataacgg gtgctgtata 3660
   atggagtccc cacaggaggc gaatatcccc ccaaggtcca cattagattt agagattaca 3720
   caagagaaca ataaattgat ctatgatcct gatccactca aggatgtgga ccttgagcta 3780
   tttagcaagg tcagagatgt tgtacataca gttgacatga cttattggtc agatgatgaa 3840
   gttatcagag caaccagtat ctgtactgca atgacgatag ctgatacaat gtctcaatta 3900
   gatagagaca acctaaaaga gatgatcgcg ctagtaaatg acgatgatgt caacagcctg 3960
   attactgagt ttatggtgat tgatgttcct ttattttgct caacgttcgg gggtattcta 4020
   gtcaatcagt ttgcatactc actctacggc ttaaacatca gaggaaggga agaaatatgg 4080
   ggacatgtag tccggattct taaagatacc tcccacgcag ttctaaaagt cttatctaat 4140
   gctctatctc atcccaaaat cttcaaacga ttctggaatg caggtgtcgt ggaacctgtg 4200
   tatgggccta acctctcaaa tcaggacaag atactcttgg ccctctctgt ctgtgaatat 4260
   tctgtggatc tattcatgca cgattggcaa gggggtgtac cgcttgagat ctttatctgt 4320
   gacaatgacc cagatgtggc cgacatgagg aggtcctctt tcttggcaag acatcttgca 4380
   tacctatgca gcgtggcaga gatatctagg gatgggccaa gattagaatc aatgaactct 4440
   ctagagaggc tcgagtcact aaagagttac ctggaactca catttcttga tgacccggta 4500
   ctgaggtaca gtcagttgac tggcctagtc atcaaagtat tcccatctac tttgacctat 4560
   atccggaagt catctataaa agtgttaagg acaagaggta taggagtccc tgaagtctta 4620
   gaagattggg atcccgaggc agataatgca ctgttagatg gtatcgcggc agaaatacaa 4680
   cagaatattc ctttgggaca tcagactaga gccccttttt gggggttgag agtatccaag 4740
   tcacaggtac tgcgtctccg ggggtacaag gagatcacaa gaggtgagat aggcagatca 4800
   ggcgttggtc tgacgttacc attcgatgga agatatctat ctcaccagct gaggctcttt 4860
   ggcatcaaca gtactagctg cttgaaagca cttgaactta cctacctatt gagcccctta 4920
   gttgacaagg ataaagatag gctatattta ggggaaggag ctggggccat gctttcctgt 4980
   tatgacgcta ctcttggccc atgcatcaac tattataact caggggtata ctcttgtgat 5040
   gtcaatgggc agagagagtt aaatatatat cctgctgagg tggcactggt gggaaagaaa 5100
   ttaaacaatg ttactagtct gggtcaaaga gttaaagtgt tattcaacgg gaatcctggc 5160
   tcgacatgga ttggaaatga tgagtgtgag gctttgattt ggaatgaatt gcagaatagc 5220
   tcgataggcc tagtccactg tgacatggag ggaggagatc ataaggatga tcaagttgta 5280
   ctgcatgagc attacagtgt aatccggatc gcgtatctgg tgggggatcg agacgttgtg 5340
   cttataagca agattgctcc taggctgggc acggattgga ccaggcagct cagcctatat 5400
   ctgagatact gggacgaggt taacctaata gtgcttaaaa catctaaccc tgcttccaca 5460
   gagatgtatc tcctatcgag gcatcccaaa tctgacatta tagaggacag caagacggtg 5520
   ttagctagtc tcctcccttt gtcaaaagaa gatagcatca agatagaaaa gtggatctta 5580
   atagagaagg caaaggctca cgaatgggtt actcgggaat tgagagaagg aagctcttca 5640
   tcagggatgc ttagacctta ccatcaagca ctgcagacgt ttggctttga accaaacttg 5700
   tataaattga gcagagattt cttgtccacc atgaacatag ctgatacaca caactgcatg 5760
   atagctttca acagggtttt gaaggataca atcttcgaat gggctagaat aactgagtca 5820
   gataaaaggc ttaaactaac tggtaagtat gacctgtatc ctgtgagaga ttcaggcaaa 5880
   ttgaagacag tttctagaag acttgtgcta tcttggatat ctttatctat gtccacaaga 5940
   ttggtaactg ggtcattccc tgaccagaag tttgaagcaa gacttcaatt gggaatagtt 6000
   tcattatcat cccgtgaaat caggaacctg agggttatca caaaaacttt attagaccgg 6060
   tttgaggata ttatacatag tataacgtac agattcctca ccaaagaaat aaagattttg 6120
   atgaagattt taggggcagt caagatgttc ggggccaggc aaaatgaata cacgaccgtg 6180
   attgatgatg gatcactggg tgatatcgag ccatatgaca gctcgtaata attagtccct 6240
   atcgtgcaga acgatcgaag ctccgcggta cctggaagtc ttggactgat ccatatgaca 6300
   atagtaagaa aaacttacaa gaagacaaga aaatttaaaa gaatacatat ctcttaaact 6360
   cttgtctggt gtcctgtttc gaaaacgaaa cagagaacca gtaccagaga aacacacgtt 6420
   gtggtatatt acctggttct cgagcaccac caccaccacc actgagatcc ggctgctaac 6480
   aaagcccgaa aggaagctga gttggctgct gccaccgctg agcaataact agcataaccc 6540
   cttggggcct ctaaacgggt cttgaggggt tttttg 6576
<210> 25
   <211> 31
   <212> DNA
   <213> Artificial
<220>
   <223> Primer
<400> 25
   gacagctcgt aatcccgggt ccctatcgtg c 31
<210> 26
   <211> 31
   <212> DNA
   <213> Artificial
<220>
   <223> Primer
<400> 26
   gcacgatagg gacccgggat tacgagctgt c 31
<210> 27
   <211> 15810
   <212> DNA
   <213> Artificial
<220>
   <223> Full length genome cDNA of Sendai virus vector-version3
<400> 27
   accaaacaag agaagaaaca tgtatggaat atataatgaa gtttaagaaa aacttagggt     60
   caaagtatcc accctgagga gcaggttcca gatccttttc tttgctgcca aagttcacga    120
   tggccgggtt gttgagcacc ttcgatacat ttagctctag gaggagcgaa agtattaata    180
   agtcgggagg aggtgctgtt atccccggcc agaggagcac agtctcagtg ttcatactag    240
   gcccaagtgt gactgatgat gcagacaagt tattcattgc aacaaccttc ctagctcact    300
   cattggacac agataagcag cactctcaga gaggagggtt cctcgtctct ctgcttgcca    360
   tggcttacag tagtccagaa ttgtacttga caacaaacgg agtaaacgcc gatgtcaaat    420
   atgtgatcta caacatagag aaagacccta agaggacgaa gacagacgga ttcattgtga    480
   agacgagaga tatggaatat gagaggacca cagaatggct gtttggacct atggtcaaca    540
   agagcccact cttccagggt caacgggatg ctgcagaccc tgacacactc cttcaaatct    600
   atgggtatcc tgcatgccta ggagcaataa ttgtccaagt ctggattgtg ctggtgaagg    660
   ccatcacaag cagcgccggc ttaaggaaag ggttcttcaa caggttagag gcgttcagac    720
   aagacggcac cgtgaaaggt gccttagttt tcactgggga gacagttgag gggataggct     780
   cggttatgag atctcagcaa agccttgtat ctctcatggt tgagaccctt gtgactatga    840
   atactgcaag atctgatctc accacattag agaagaacat ccagatcgtt gggaactaca    900
   tccgagatgc agggctggct tccttcatga acactattaa atatggggtg gagacaaaga    960
   tggcagctct aacgttgtca aacctgaggc ccgatattaa taagcttaga agcctcatag   1020
   acacctacct gtcaaaaggc cccagagctc cctttatctg tatcctcaag gaccctgttc 1080
   atggtgaatt tgctccaggc aattatcctg cactatggag ttacgccatg ggagtcgccg 1140
   tcgtacagaa caagtcaatg cagcagtacg tcacagggag gacatacctt gatatggaaa 1200
   tgttcttact aggacaagcc gtggcaaagg atgctgaatc gaagatcagc agtgccttgg 1260
   aagatgagtt aggagtgacg gatacagcca aggagaggct cagacatcat ctggcaaact 1320
   tgtccggtgg ggatggtgct taccacaaac caacaggcgg tggtgcaatt gaggtagctc 1380
   tagacaatgc cgatatcgac ctggaaacag aagctcatgc ggaccaggac gctaggggtt 1440
   ggggtggaga tagtggtgaa agatgggcac gtcaggtgag tggtggccac tttgtcacac 1500
   tacatggggc tgaacggtta gaggaggaaa ccaatgatga ggatgtatca gacatagaga 1560
   gaagaatagc catgagactc gcagagagac ggcaagagga ttctgcaacc catggagatg 1620
   aaggccgcaa taacggtgtt gatcacgaag aagatgacga tgccgcagca gcagctggga 1680
   taggaggaat ctaggatcat acgaggcctc aaggtacttg atccgcagta agaaaaactt 1740
   agggtgaaag ttcatccacc gatcggctca ggcaaggcca cacccaaccc caccgaccac 1800
   acccagcagt cgagacagcc acggcttcgg ctacacttac cgcatggatc aagatgcctt 1860
   cattcttaaa gaagattctg aagttgagag gaaggcgcca ggaggacgag agtcgctctc 1920
   ggatgttatc ggattcctcg atgctgtcct gtcgaatgaa ccaactgaca tcggagggga 1980
   cagaagctgg ctccacaaca ccatcaacac tccccaagga ccaggctctg ctcatagagc 2040
   caaaagtgag ggcgaaggag aagtctcaac accgtcgacc caagataatc gatcaggtga 2100
   ggagagtaga gtctctggga gaacaagcaa gccagaggca gaagcacatg ctggaaacct 2160
   tgataaacaa aatatacact gggcctttag gggaagaact ggtacaaact ctgtatctca 2220
   ggatctggac gatggaggag actccggaat ccttgaaaat cctccaaatg agagaggata 2280
   tccgagatca ggtattgaag atgaaaacag agagatggct gcgcaccctg ataagagggg 2340
   agaagaccaa gctgaaggac ttccagaaga ggtacgagga ggtacatccc tacctgatga 2400
   aggagaaggt ggagcaagta ataatggaag aagcatggag cctggcagct cacatagtgc 2460
   aagagtaact ggggtcctgg tgattcctag ccccgaactt gaagaggctg tgctacggag 2520
   gaacaaaaga agacctacca acagtgggtc caaacctctt actccagcaa ccgtgcctgg 2580
   cacccggtcc ccaccgctga atcgttacaa cagcacaggg tcaccaccag gaaaaccccc 2640
   atctacacag gatgagcaca tcaactctgg ggacaccccc gccgtcaggg tcaaagaccg 2700
   gaaaccatca atagggactc gctctgtctc agattgtcca gccaacggcc gcccaatcca 2760
   tccgggtata gagaccgact caacaaaaaa gggcatagga gagaacacat catctatgaa 2820
   agatatggct acattgttga cgagtcttgg tgtaatccag tctgctcaag aattcgagtc 2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcccacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcttagacga tggatttttt tcgggtagtg gaaaaccagc agcctcccgc gacgatgccc 3720
   ctcaacgtta gcttcaccaa caggaactat gacctcgact acgactcggt gcagccgtat 3780
   ttctactgcg acgaggagga gaacttctac cagcagcagc agcagagcga gctgcagccc 3840
   ccggcgccca gcgaggatat ctggaagaaa ttcgagctgc tgcccacccc gcccctgtcc 3900
   cctagccgcc gctccgggct ctgctcgccc tcctacgttg cggtcacacc cttctccctt 3960
   cggggagaca acgacggcgg tggcgggagc ttctccacgg ccgaccagct ggagatggtg 4020
   accgagctgc tgggaggaga catggtgaac cagagtttca tctgcgaccc ggacgacgag 4080
   accttcatca aaaacatcat catccaggac tgtatgtgga gcggcttctc ggccgccgcc 4140
   aagctcgtct cagagaagct ggcctcctac caggctgcgc gcaaagacag cggcagcccg 4200
   aaccccgccc gcggccacag cgtctgctcc acctccagct tgtacctgca ggatctgagc 4260
   gccgccgcct cagagtgcat cgacccctcg gtggtcttcc cctaccctct caacgacagc 4320
   agctcgccca agtcctgcgc ctcgcaagac tccagcgcct tctctccgtc ctcggattct 4380
   ctgctctcct cgacggagtc ctccccgcag ggcagccccg agcccctggt gctccatgag 4440
   gagacaccgc ccaccaccag cagcgactct gaggaggaac aagaagatga ggaagaaatc 4500
   gatgttgttt ctgtggaaaa gaggcaggct cctggcaaaa ggtcagagtc tggatcacct 4560
   tctgctggag gccacagcaa acctcctcac agcccactgg tcctcaagag gtgccacgtc 4620
   tccacacatc agcacaacta cgcagcgcct ccctccactc ggaaggacta tcctgctgcc 4680
   aagagggtca agttggacag tgtcagagtc ctgagacaga tcagcaacaa ccgaaaatgc 4740
   accagcccca ggtcctcgga caccgaggag aatgtcaaga ggcgaacaca caacgtcttg 4800
   gagcgccaga ggaggaacga gctaaaacgg agcttttttg ccctgcgtga ccagatcccg 4860
   gagttggaaa acaatgaaaa ggcccccaag gtagttatcc ttaaaaaagc cacagcatac 4920
   atcctgtccg tccaagcaga ggagcaaaag ctcatttctg aagaggactt gttgcggaaa 4980
   cgacgagaac agttgaaaca caaacttgaa cagctacgga actcttgtgc gtaaggacgt 5040
   cgattaagaa aaacttaggg tgaaagttca tcgcggccgc ttgctagcag tctgacatgg 5100
   ctgtcagcga cgcgctgctc ccatctttct ccacgttcgc gtctggcccg gcgggaaggg 5160
   agaagacact gcgtcaagca ggtgccccga ataaccgctg gcgggaggag ctctcccaca 5220
   tgaagcgact tcccccagtg cttcccggcc gcccctatga cctggcggcg gcgaccgtgg 5280
   ccacagacct ggagagcggc ggagccggtg cggcttgcgg cggtagcaac ctggcgcccc 5340
   tacctcggag agagaccgag gagttcaacg atctcctgga cctggacttt attctctcca 5400
   attcgctgac ccatcctccg gagtcagtgg ccgccaccgt gtcctcgtca gcgtcagcct 5460
   cctcttcgtc gtcgccgtcg agcagcggcc ctgccagcgc gccctccacc tgcagcttca 5520
   cctatccgat ccgggccggg aacgacccgg gcgtggcgcc gggcggcacg ggcggaggcc 5580
   tcctctatgg cagggagtcc gctccccctc cgacggctcc cttcaacctg gcggacatca 5640
   acgacgtgag cccctcgggc ggcttcgtgg ccgagctcct gcggccagaa ttggacccgg 5700
   tgtacattcc gccgcagcag ccgcagccgc caggtggcgg gctgatgggc aagttcgtgc 5760
   tgaaggcgtc gctgagcgcc cctggcagcg agtacggcag cccgtcggtc atcagcgtca 5820
   gcaaaggcag ccctgacggc agccacccgg tggtggtggc gccctacaac ggcgggccgc 5880
   cgcgcacgtg ccccaagatc aagcaggagg cggtctcttc gtgcacccac ttgggcgctg 5940
   gaccccctct cagcaatggc caccggccgg ctgcacacga cttccccctg gggcggcagc 6000
   tccccagcag gactaccccg accctgggtc ttgaggaagt gctgagcagc agggactgtc 6060
   accctgccct gccgcttcct cccggcttcc atccccaccc ggggcccaat tacccatcct 6120
   tcctgcccga tcagatgcag ccgcaagtcc cgccgctcca ttaccaagag ctcatgccac 6180
   ccggttcctg catgccagag gagcccaagc caaagagggg aagacgatcg tggccccgga 6240
   aaaggaccgc cacccacact tgtgattacg cgggctgcgg caaaacctac acaaagagtt 6300
   cccatctcaa ggcacacctg cgaacccaca caggtgagaa accttaccac tgtgactggg 6360
   acggctgtgg atggaaattc gcccgctcag atgaactgac caggcactac cgtaaacaca 6420
   cggggcaccg cccgttccag tgccaaaaat gcgaccgagc attttccagg tcggaccacc 6480
   tcgccttaca catgaagagg catttttaaa cgcgtcagag acctgcaaca atgtctcaag 6540
   cagacaccac ctggcagtcg gagccaccgg gtcactcctt gtcttaaata agaaaaactt 6600
   agggataaag tcccttagat ctagcctagg ttccccatgg cgggacacct ggcttcggat 6660
   ttcgccttct cgccccctcc aggtggtgga ggtgatgggc caggggggcc ggagccgggc 6720
   tgggttgatc ctcggacctg gctaagcttc caaggccctc ctggagggcc aggaatcggg 6780
   ccgggggttg ggccaggctc tgaggtgtgg gggattcccc catgcccccc gccgtatgag 6840
   ttctgtgggg ggatggcgta ctgtgggccc caggttggag tggggctagt gccccaaggc 6900
   ggcttggaga cctctcagcc tgagggcgaa gcaggagtcg gggtggagag caactccgat 6960
   ggggcctccc cggagccctg caccgtcacc cctggtgccg tgaagctgga gaaggagaag 7020
   ctggagcaaa acccggagga gtcccaggac atcaaagctc tgcagaaaga actcgagcaa 7080
   tttgccaagc tcctgaagca gaagaggatc accctgggat atacacaggc cgatgtgggg 7140
   ctcaccctgg gggttctatt tgggaaggta ttcagccaaa cgaccatctg ccgctttgag 7200
   gctctgcagc ttagcttcaa gaacatgtgt aagctgcggc ccttgctgca gaagtgggtg 7260
   gaggaagctg acaacaatga aaatcttcag gagatatgca aagcagaaac cctcgtgcag 7320
   gcccgaaaga gaaagcgaac cagtatcgag aaccgagtga gaggcaacct ggagaatttg 7380
   ttcctgcagt gcccgaaacc cacactgcag cagatcagcc acatcgccca gcagcttggg 7440
   ctcgagaagg atgtggtccg agtgtggttc tgtaaccggc gccagaaggg caagcgatca 7500
   agcagcgact atgcacaacg agaggatttt gaggctgctg ggtctccttt ctcaggggga 7560
   ccagtgtcct ttcctctggc cccagggccc cattttggta ccccaggcta tgggagccct 7620
   cacttcactg cactgtactc ctcggtccct ttccctgagg gggaagcctt tccccctgtc 7680
   tccgtcacca ctctgggctc tcccatgcat tcaaactgag gacgtcagat ctgtatataa 7740
   taagaaaaac ttagggtgaa agtgaggttg cgcggtattt tagctagccc gcatgtacaa 7800
   catgatggag acggagctga agccgccggg cccgcagcaa acttcggggg gcggcggcgg 7860
   caactccacc gcggcggcgg ccggcggcaa ccagaaaaac agcccggacc gcgtcaagcg 7920
   gcccatgaat gccttcatgg tgtggtcccg cgggcagcgg cgcaagatgg cccaggagaa 7980
   ccccaagatg cacaactcgg agatcagcaa gcgcctgggc gccgagtgga aacttttgtc 8040
   ggagacggag aagcggccgt tcatcgacga ggctaagcgg ctgcgagcgc tgcacatgaa 8100
   ggagcacccg gattataaat accggccccg gcggaaaacc aagacgctca tgaagaagga 8160
   taagtacacg ctgcccggcg ggctgctggc ccccggcggc aatagcatgg cgagcggggt 8220
   cggggtgggc gccggcctgg gcgcgggcgt gaaccagcgc atggacagtt acgcgcacat 8280
   gaacggctgg agcaacggca gctacagcat gatgcaggac cagctgggct acccgcagca 8340
   cccgggcctc aatgcgcacg gcgcagcgca gatgcagccc atgcaccgct acgacgtgag 8400
   cgccctgcag tacaactcca tgaccagctc gcagacctac atgaacggct cgcccaccta 8460
   cagcatgtcc tactcgcagc agggcacccc tggcatggct cttggctcca tgggttcggt 8520
   ggtcaagtcc gaggccagct ccagcccccc tgtggttacc tcttcctccc actccagggc 8580
   gccctgccag gccggggacc tccgggacat gatcagcatg tatctccccg gcgccgaggt 8640
   gccggaaccc gccgccccca gcagacttca catgtcccag cactaccaga gcggcccggt 8700
   gcccggcacg gccattaacg gcacactgcc cctctcacac atgtgagacc ggtgtcggct 8760
   ttgctgacac tagagtcatc tccgaacatc cacaatatct ctcagtctct tacgtctctc 8820
   acagtattaa gaaaaaccca gggtgaatgg gaagcttgcc ataggtcatg gatgggcagg 8880
   agtcctccca aaacccttct gacatactct atccagaatg ccacctgaac tctcccatag 8940
   tcagggggaa gatagcacag ttgcacgtct tgttagatgt gaaccagccc tacagactaa 9000
   aggacgacag cataataaat attacaaagc acaaaattag gaacggagga ttgtcccctc 9060
   gtcaaattaa gatcaggtct ctgggtaagg ctcttcaacg cacaataaag gatttagacc 9120
   gatacacctt tgaaccgtac ccaacctact ctcaggaatt acttaggctt gatataccag 9180
   agatatgtga caaaatccga tccgtcttcg cggtctcgga tcggctgacc agggagttat 9240
   ctagtgggtt ccaggatctt tggttgaata tcttcaagca actaggcaat atagaaggaa 9300
   gagaggggta cgatccgttg caggatatcg gcaccatccc ggagataact gataaataca 9360
   gcaggaatag atggtatagg ccattcctaa cttggttcag catcaaatat gacatgcggt 9420
   ggatgcagaa gaccagaccg gggggacccc tcgatacctc taattcacat aacctcctag 9480
   aatgcaaatc atacactcta gtaacatacg gagatcttat catgatactg aacaagttga 9540
   cattgacagg gtatatccta acccctgagc tggtcttgat gtattgtgat gttgtagagg 9600
   gaaggtggaa tatgtctgct gcagggcatc tagataagaa gtccattggg ataacaagca 9660
   aaggtgagga attatgggaa ctagtggatt ccctcttctc aagtcttgga gaggaaatat 9720
   acaatgtcat cgcactattg gagcccctat cacttgctct catacaacta aatgatccag 9780
   ttatacctct acgtggggca tttatgaggc atgtgttgac agagctacag gctgttttaa 9840
   caagtaggga cgtgtacaca gatgctgaag cagacactat tgtggagtcg ttactcgcca 9900
   ttttccatgg aacctctatt gatgagaaag cagagatctt ttccttcttt aggacatttg 9960
   gccaccccag cttagaggct gtcactgccg ccgacaaggt aagggcccat atgtatgcac 10020
   aaaaggcaat aaagcttaag accctatacg agtgtcatgc agttttttgc actatcatca 10080
   taaatgggta tagagagagg catggcggac agtggccccc ctgtgacttc cctgatcacg 10140
   tgtgtctaga actaaggaac gctcaagggt ccaatacggc aatctcttat gaatgtgctg 10200
   tagacaacta tacaagtttc ataggcttca agtttcggaa gtttatagaa ccacaactag 10260
   atgaagatct cacaatatat atgaaagaca aagcactatc ccccaggaag gaggcatggg 10320
   actctgtata cccggatagt aatctgtact ataaagcccc agaatctgaa gagacccggc 10380
   ggcttattga agtgttcata aatgatgaga atttcaaccc agaagaaatt atcaattatg 10440
   tggagtcagg agattggttg aaagacgaga agttcaacat ctcgtacagt ctcaaagaga 10500
   aagagatcaa gcaagagggt cgtctattcg caaaaatgac ttataagatg cgagccgtac 10560
   aggtgctggc agagacacta ctggctaaag gaataggaga gctgttcagc gaaaatggga 10620
   tggttaaagg agagatagac ctacttaaaa gattgactac tctttctgtc tcaggagtcc 10680
   ccaggactga ttcagtgtac aataactcta aatcatcaga gaagagaaac gaaggcatga 10740
   aaaagaagaa ctctgggggg tactgggacg aaaagaagag gtccagacat gaattcaagg 10800
   caacagattc atcaacagac ggctatgaaa cgttaagttg cttcctcaca acagacctca 10860
   agaaatactg cttaaactgg agatttgaaa gtactgcatt gtttggtcag agatgcaacg 10920
   agatatttgg cttcaagacc ttctttaact ggatgcatcc agtccttgaa aggtgtacaa 10980
   tatatgttgg ggatccttac tgtccagtcg ccgaccggat gcatcgacaa ctccaggatc 11040
   atgcagactc tggcattttc atacataatc ctaggggggg catagaaggt tactgccaga 11100
   agctgtggac cttaatctca atcagtgcaa tccacctagc agctgtgaga gtgggtgtca 11160
   gggtctctgc aatggttcag ggtgacaatc aagctatagc cgtgacatca agagtacctg 11220
   tagctcagac ttacaagcag aagaaaaatc atgtctataa ggagatcacc aaatattttg 11280
   gtgctctaag acacgtcatg tttgatgtag ggcacgagct aaaattgaac gagaccatca 11340
   ttagtagcaa gatgtttgtc tatagtaaaa gaatatacta tgatgggaag attttaccac 11400
   agtgcctgaa agccttgacc aggtgtgtat tctggtccga gacactggta gatgaaaaca 11460
   gatctgcttg ttcgaacatc tcaacatcca tagcaaaagc tatcgaaaat gggtattctc 11520
   ctatactagg ctactgcatt gcgttgtata agacctgtca gcaggtgtgc atatcactag 11580
   ggatgactat aaatccaact atcagcccga ccgtaagaga tcaatacttt aagggtaaga 11640
   attggctgag atgtgcagtg ttgattccag caaatgttgg aggattcaac tacatgtcta 11700
   catctagatg ctttgttaga aatattggag accccgcagt agcagcccta gctgatctca 11760
   aaagattcat cagagcggat ctgttagaca agcaggtact atacagggtc atgaatcaag 11820
   aacccggtga ctctagcttt ctagattggg cttcagaccc ttattcatgt aacctcccgc 11880
   attctcagag tataactacg attataaaga atatcactgc tagatctgtg ctgcaggaat 11940
   ccccgaatcc tctactgtct ggtctcttca ccgagactag tggagaagag gatctcaacc 12000
   tggcctcgtt ccttatggac cggaaagtca tcctgccgag agtggctcat gagatcctgg 12060
   gtaattcctt aactggagtt agggaggcga ttgcagggat gcttgatacg accaagtctc 12120
   tagtgagatc cagcgttaag aaaggaggat tatcatatgg gatattgagg aggcttgtca 12180
   attatgatct attgcagtac gagacactga ctagaactct caggaaaccg gtgaaagaca 12240
   acatcgaata tgagtatatg tgttcagttg agctagctgt cggtctaagg cagaaaatgt 12300
   ggatccacct aacttacggg agacccatac atgggctaga aacaccagac cctttagagc 12360
   tcttgagggg aacatttatc gaaggttcag aggtgtgcaa gctttgcagg tctgagggag 12420
   cagaccccat ctatacatgg ttctatctcc ctgacaatat agacctggac acgcttacaa 12480
   acggatgtcc ggctataaga atcccctatt ttggatcagc cactgatgaa aggtcggaag 12540
   cccaactcgg gtatgtaaga aatctaagca aacccgcaaa ggctgccatc cggatagcta 12600
   tggtgtatac gtgggcctac gggactgatg agatatcgtg gatggaagcc gctcttatag 12660
   cccaaacaag agctaatctg agcttagaga atctaaagct gctgactcct gtttcaacct 12720
   ccactaatct atctcatagg ttgaaagata cggcaaccca gatgaagttc tctagtgcaa 12780
   cactagtccg tgcaagtcgg ttcataacaa tatcaaatga taacatggca ctcaaagaag 12840
   caggggagtc gaaggatact aatctcgtgt atcagcagat tatgctaact gggctaagct 12900
   tgttcgagtt caatatgaga tataagaaag gttccttagg gaagccactg atattgcact 12960
   tacatcttaa taacgggtgc tgtataatgg agtccccaca ggaggcgaat atccccccaa 13020
   ggtccacatt agatttagag attacacaag agaacaataa attgatctat gatcctgatc 13080
   cactcaagga tgtggacctt gagctattta gcaaggtcag agatgttgta catacagttg 13140
   acatgactta ttggtcagat gatgaagtta tcagagcaac cagtatctgt actgcaatga 13200
   cgatagctga tacaatgtct caattagata gagacaacct aaaagagatg atcgcgctag 13260
   taaatgacga tgatgtcaac agcctgatta ctgagtttat ggtgattgat gttcctttat 13320
   tttgctcaac gttcgggggt attctagtca atcagtttgc atactcactc tacggcttaa 13380
   acatcagagg aagggaagaa atatggggac atgtagtccg gattcttaaa gatacctccc 13440
   acgcagttct aaaagtctta tctaatgctc tatctcatcc caaaatcttc aaacgattct 13500
   ggaatgcagg tgtcgtggaa cctgtgtatg ggcctaacct ctcaaatcag gacaagatac 13560
   tcttggccct ctctgtctgt gaatattctg tggatctatt catgcacgat tggcaagggg 13620
   gtgtaccgct tgagatcttt atctgtgaca atgacccaga tgtggccgac atgaggaggt 13680
   cctctttctt ggcaagacat cttgcatacc tatgcagcgt ggcagagata tctagggatg 13740
   ggccaagatt agaatcaatg aactctctag agaggctcga gtcactaaag agttacctgg 13800
   aactcacatt tcttgatgac ccggtactga ggtacagtca gttgactggc ctagtcatca 13860
   aagtattccc atctactttg acctatatcc ggaagtcatc tataaaagtg ttaaggacaa 13920
   gaggtatagg agtccctgaa gtcttagaag attgggatcc cgaggcagat aatgcactgt 13980
   tagatggtat cgcggcagaa atacaacaga atattccttt gggacatcag actagagccc 14040
   ctttttgggg gttgagagta tccaagtcac aggtactgcg tctccggggg tacaaggaga 14100
   tcacaagagg tgagataggc agatcaggcg ttggtctgac gttaccattc gatggaagat 14160
   atctatctca ccagctgagg ctctttggca tcaacagtac tagctgcttg aaagcacttg 14220
   aacttaccta cctattgagc cccttagttg acaaggataa agataggcta tatttagggg 14280
   aaggagctgg ggccatgctt tcctgttatg acgctactct tggcccatgc atcaactatt 14340
   ataactcagg ggtatactct tgtgatgtca atgggcagag agagttaaat atatatcctg 14400
   ctgaggtggc actggtggga aagaaattaa acaatgttac tagtctgggt caaagagtta 14460
   aagtgttatt caacgggaat cctggctcga catggattgg aaatgatgag tgtgaggctt 14520
   tgatttggaa tgaattgcag aatagctcga taggcctagt ccactgtgac atggagggag 14580
   gagatcataa ggatgatcaa gttgtactgc atgagcatta cagtgtaatc cggatcgcgt 14640
   atctggtggg ggatcgagac gttgtgctta taagcaagat tgctcctagg ctgggcacgg 14700
   attggaccag gcagctcagc ctatatctga gatactggga cgaggttaac ctaatagtgc 14760
   ttaaaacatc taaccctgct tccacagaga tgtatctcct atcgaggcat cccaaatctg 14820
   acattataga ggacagcaag acggtgttag ctagtctcct ccctttgtca aaagaagata 14880
   gcatcaagat agaaaagtgg atcttaatag agaaggcaaa ggctcacgaa tgggttactc 14940
   gggaattgag agaaggaagc tcttcatcag ggatgcttag accttaccat caagcactgc 15000
   agacgtttgg ctttgaacca aacttgtata aattgagcag agatttcttg tccaccatga 15060
   acatagctga tacacacaac tgcatgatag ctttcaacag ggttttgaag gatacaatct 15120
   tcgaatgggc tagaataact gagtcagata aaaggcttaa actaactggt aagtatgacc 15180
   tgtatcctgt gagagattca ggcaaattga agacagtttc tagaagactt gtgctatctt 15240
   ggatatcttt atctatgtcc acaagattgg taactgggtc attccctgac cagaagtttg 15300
   aagcaagact tcaattggga atagtttcat tatcatcccg tgaaatcagg aacctgaggg 15360
   ttatcacaaa aactttatta gaccggtttg aggatattat acatagtata acgtacagat 15420
   tcctcaccaa agaaataaag attttgatga agattttagg ggcagtcaag atgttcgggg 15480
   ccaggcaaaa tgaatacacg accgtgattg atgatggatc actgggtgat atcgagccat 15540
   atgacagctc gtaataccgg ttatcaccaa aacatggaag cacttacgat tcaccaaaac 15600
   atggaagcac ttaggtacct caccaaaaca tggaagcact tacgattcac caaaacatgg 15660
   aagcacttaa ccggttccct atcgtgcaga acgatcgaag ctccgcggta cctggaagtc 15720
   ttggactgat ccatatgaca atagtaagaa aaacttacaa gaagacaaga aaatttaaaa 15780
   gaatacatat ctcttaaact cttgtctggt 15810
<210> 28
   <211> 15816
   <212> DNA
   <213> Artificial
<220>
   <223> cDNA complementary to full length genome of Sendai virus vector-v ersion4 for continuous expression of hOct4, hSox2, hKlf4 and hc-M yc
<400> 28
   accaaacaag agaagaaaca tgtatggaat atataatgaa gttagacagg attttagggt 60
   caaagtatcc accctgagga gcaggttcca gatccttttc tttgctgcca aagttcacga 120
   tggccgggtt gttgagcacc ttcgatacat ttagctctag gaggagcgaa agtattaata 180
   agtcgggagg aggtgctgtt atccccggcc agaggagcac agtctcagtg ttcatactag 240
   gcccaagtgt gactgatgat gcagacaagt tattcattgc aacaaccttc ctagctcact 300
   cattggacac agataagcag cactctcaga gaggagggtt cctcgtctct ctgcttgcca 360
   tggcttacag tagtccagaa ttgtacttga caacaaacgg agtaaacgcc gatgtcaaat 420
   atgtgatcta caacatagag aaagacccta agaggacgaa gacagacgga ttcattgtga 480
   agacgagaga tatggaatat gagaggacca cagaatggct gtttggacct atggtcaaca 540
   agagcccact cttccagggt caacgggatg ctgcagaccc tgacacactc cttcaaatct 600
   atgggtatcc tgcatgccta ggagcaataa ttgtccaagt ctggattgtg ctggtgaagg 660
   ccatcacaag cagcgccggc ttaaggaaag ggttcttcaa caggttagag gcgttcagac 720
   aagacggcac cgtgaaaggt gccttagttt tcactgggga gacagttgag ggatagggct 780
   cggttatgag atctcagcaa agccttgtat ctctcatggt tgagaccctt gtgactatga 840
   atactgcaag atctgatctc accacattag agaagaacat ccagatcgtt gggaactaca 900
   tccgagatgc agggctggct tccttcatga acactattaa atatggggtg gagacaaaga 960
   tggcagctct aacgttgtca aacctgaggc ccgatattaa taagcttaga agcctcatag 1020
   acacctacct gtcaaaaggc cccagagctc cctttatctg tatcctcaag gaccctgttc 1080
   atggtgaatt tgctccaggc aattatcctg cactatggag ttacgccatg ggagtcgccg 1140
   tcgtacagaa caagtcaatg cagcagtacg tcacagggag gacatacctt gatatggaaa 1200
   tgttcttact aggacaagcc gtggcaaagg atgctgaatc gaagatcagc agtgccttgg 1260
   aagatgagtt aggagtgacg gatacagcca aggagaggct cagacatcat ctggcaaact 1320
   tgtccggtgg ggatggtgct taccacaaac caacaggcgg tggtgcaatt gaggtagctc 1380
   tagacaatgc cgatatcgac ctggaaacag aagctcatgc ggaccaggac gctaggggtt 1440
   ggggtggaga tagtggtgaa agatgggcac gtcaggtgag tggtggccac tttgtcacac 1500
   tacatggggc tgaacggtta gaggaggaaa ccaatgatga ggatgtatca gacatagaga 1560
   gaagaatagc catgagactc gcagagagac ggcaagagga ttctgcaacc catggagatg 1620
   aaggccgcaa taacggtgtt gatcacgaag aagatgacga tgccgcagca gcagctggga 1680
   taggaggaat ctaggatcat acgaggcctc aaggtacttg atccgcagta agaaaaactt 1740
   agggtgaaag ttcatccacc gatcggctca ggcaaggcca cacccaaccc caccgaccac 1800
   acccagcagt cgagacagcc acggcttcgg ctacacttac cgcatggatc aagatgcctt 1860
   cattcttaaa gaagattctg aagttgagag gaaggcgcca ggaggacgag agtcgctctc 1920
   ggatgttatc ggattcctcg atgctgtcct gtcgaatgaa ccaactgaca tcggagggga 1980
   cagaagctgg ctccacaaca ccatcaacac tccccaagga ccaggctctg ctcatagagc 2040
   caaaagtgag ggcgaaggag aagtctcaac accgtcgacc caagataatc gatcaggtga 2100
   ggagagtaga gtctctggga gaacaagcaa gccagaggca gaagcacatg ctggaaacct 2160
   tgataaacaa aatatacact gggcctttag gggaagaact ggtacaaact ctgtatctca 2220
   ggatctggac gatggaggag actccggaat ccttgaaaat cctccaaatg agagaggata 2280
   tccgagatca ggtattgaag atgaaaacag agagatggct gcgcaccctg ataagagggg 2340
   agaagaccaa gctgaaggac ttccagaaga ggtacgagga ggtacatccc tacctgatga 2400
   aggagaaggt ggagcaagta ataatggaag aagcatggag cctggcagct cacatagtgc 2460
   aagagtaact ggggtcctgg tgattcctag ccccgaactt gaagaggctg tgctacggag 2520
   gaacaaaaga agacctacca acagtgggtc caaacctctt actccagcaa ccgtgcctgg 2580
   cacccggtcc ccaccgctga atcgttacaa cagcacaggg tcaccaccag gaaaaccccc 2640
   atctacacag gatgagcaca tcaactctgg ggacaccccc gccgtcaggg tcaaagaccg 2700
   gaaaccatca atagggactc gctctgtctc agattgtcca gccaacggcc gcccaatcca 2760
   tccgggtata gagaccgact caacaaaaaa gggcatagga gagaacacat catctatgaa 2820
   agatatggct acattgttga cgagtcttgg tgtaatccag tctgctcaag aattcgagtc 2880
   atcccgagac gcgagttatg tgtttgcaag acgtgcccta aagtctgcaa actatgcaga 2940
   gatgacattc aatgtatgcg gcctgatcct ttctgccgag aaatcttccg ctcgtaaggt 3000
   agatgagaac aaacaactgc tcaaacagat ccaagagagc gtggaatcat tccgggatat 3060
   ttacaagaga ttctctgagt atcagaaaga acagaactca ttgctgatgt ccaacctatc 3120
   tacacttcat atcatcacag atagaggtgg caagactgac aacacagact cccttacaag 3180
   gtccccctcc gtttttgcaa aatcaaaaga gaacaagact aaggctacca ggtttgaccc 3240
   atctatggag accttagaag atatgaagta caaaccggac ctaatccgag aggatgaatt 3300
   tagagatgag atccgcaacc cggtgtacca agagagggac acagaaccca gggcctcaaa 3360
   cgcatcacgt ctcttcccct ccaaagagaa gcacacaatg cactctctca ggctcgtcat 3420
   agagagcagt cccctaagca gagctgagaa agcagcatat gtgaaatcat tatccaagtg 3480
   caagacagac caagaggtta aggcagtcat ggaactcgta gaagaggaca tagagtcact 3540
   gaccaactag atcccgggtg aggcatccca ccatcctcag tcacagagag acccaatcta 3600
   ccatcagcat cagccagtaa agattaagaa aaacttaggg tgaaagaaat ttcaccgcta 3660
   gcacctaggt ctgacatggc tgtcagcgac gcgctgctcc catctttctc cacgttcgcg 3720
   tctggcccgg cgggaaggga gaagacactg cgtcaagcag gtgccccgaa taaccgctgg 3780
   cgggaggagc tctcccacat gaagcgactt cccccagtgc ttcccggccg cccctatgac 3840
   ctggcggcgg cgaccgtggc cacagacctg gagagcggcg gagccggtgc ggcttgcggc 3900
   ggtagcaacc tggcgcccct acctcggaga gagaccgagg agttcaacga tctcctggac 3960
   ctggacttta ttctctccaa ttcgctgacc catcctccgg agtcagtggc cgccaccgtg 4020
   tcctcgtcag cgtcagcctc ctcttcgtcg tcgccgtcga gcagcggccc tgccagcgcg 4080
   ccctccacct gcagcttcac ctatccgatc cgggccggga acgacccggg cgtggcgccg 4140
   ggcggcacgg gcggaggcct cctctatggc agggagtccg ctccccctcc gacggctccc 4200
   ttcaacctgg cggacatcaa cgacgtgagc ccctcgggcg gcttcgtggc cgagctcctg 4260
   cggccagaat tggacccggt gtacattccg ccgcagcagc cgcagccgcc aggtggcggg 4320
   ctgatgggca agttcgtgct gaaggcgtcg ctgagcgccc ctggcagcga gtacggcagc 4380
   ccgtcggtca tcagcgtcag caaaggcagc cctgacggca gccacccggt ggtggtggcg 4440
   ccctacaacg gcgggccgcc gcgcacgtgc cccaagatca agcaggaggc ggtctcttcg 4500
   tgcacccact tgggcgctgg accccctctc agcaatggcc accggccggc tgcacacgac 4560
   ttccccctgg ggcggcagct ccccagcagg actaccccga ccctgggtct tgaggaagtg 4620
   ctgagcagca gggactgtca ccctgccctg ccgcttcctc ccggcttcca tccccacccg 4680
   gggcccaatt acccatcctt cctgcccgat cagatgcagc cgcaagtccc gccgctccat 4740
   taccaagagc tcatgccacc cggttcctgc atgccagagg agcccaagcc aaagagggga 4800
   agacgatcgt ggccccggaa aaggaccgcc acccacactt gtgattacgc gggctgcggc 4860
   aaaacctaca caaagagttc ccatctcaag gcacacctgc gaacccacac aggtgagaaa 4920
   ccttaccact gtgactggga cggctgtgga tggaaattcg cccgctcaga tgaactgacc 4980
   aggcactacc gtaaacacac ggggcaccgc ccgttccagt gccaaaaatg cgaccgagca 5040
   ttttccaggt cggaccacct cgccttacac atgaagaggc atttttaaag acgtcgatta 5100
   agaaaaactt agggtgaaag ttcatcgcgg ccgcttgcta gcggttcccc atggcgggac 5160
   acctggcttc ggatttcgcc ttctcgcccc ctccaggtgg tggaggtgat gggccagggg 5220
   ggccggagcc gggctgggtt gatcctcgga cctggctaag cttccaaggc cctcctggag 5280
   ggccaggaat cgggccgggg gttgggccag gctctgaggt gtgggggatt cccccatgcc 5340
   ccccgccgta tgagttctgt ggggggatgg cgtactgtgg gccccaggtt ggagtggggc 5400
   tagtgcccca aggcggcttg gagacctctc agcctgaggg cgaagcagga gtcggggtgg 5460
   agagcaactc cgatggggcc tccccggagc cctgcaccgt cacccctggt gccgtgaagc 5520
   tggagaagga gaagctggag caaaacccgg aggagtccca ggacatcaaa gctctgcaga 5580
   aagaactcga gcaatttgcc aagctcctga agcagaagag gatcaccctg ggatatacac 5640
   aggccgatgt ggggctcacc ctgggggttc tatttgggaa ggtattcagc caaacgacca 5700
   tctgccgctt tgaggctctg cagcttagct tcaagaacat gtgtaagctg cggcccttgc 5760
   tgcagaagtg ggtggaggaa gctgacaaca atgaaaatct tcaggagata tgcaaagcag 5820
   aaaccctcgt gcaggcccga aagagaaagc gaaccagtat cgagaaccga gtgagaggca 5880
   acctggagaa tttgttcctg cagtgcccga aacccacact gcagcagatc agccacatcg 5940
   cccagcagct tgggctcgag aaggatgtgg tccgagtgtg gttctgtaac cggcgccaga 6000
   agggcaagcg atcaagcagc gactatgcac aacgagagga ttttgaggct gctgggtctc 6060
   ctttctcagg gggaccagtg tcctttcctc tggccccagg gccccatttt ggtaccccag 6120
   gctatgggag ccctcacttc actgcactgt actcctcggt ccctttccct gagggggaag 6180
   cctttccccc tgtctccgtc accactctgg gctctcccat gcattcaaac tgaacgcgtc 6240
   agagacctgc aacaatgtct caagcagaca ccacctggca gtcggagcca ccgggtcact 6300
   ccttgtctta aataagaaaa acttagggat aaagtccctt agatctagcc taggcgcatg 6360
   tacaacatga tggagacgga gctgaagccg ccgggcccgc agcaaacttc ggggggcggc 6420
   ggcggcaact ccaccgcggc ggcggccggc ggcaaccaga aaaacagccc ggaccgcgtc 6480
   aagcggccca tgaatgcctt catggtgtgg tcccgcgggc agcggcgcaa gatggcccag 6540
   gagaacccca agatgcacaa ctcggagatc agcaagcgcc tgggcgccga gtggaaactt 6600
   ttgtcggaga cggagaagcg gccgttcatc gacgaggcta agcggctgcg agcgctgcac 6660
   atgaaggagc acccggatta taaataccgg ccccggcgga aaaccaagac gctcatgaag 6720
   aaggataagt acacgctgcc cggcgggctg ctggcccccg gcggcaatag catggcgagc 6780
   ggggtcgggg tgggcgccgg cctgggcgcg ggcgtgaacc agcgcatgga cagttacgcg 6840
   cacatgaacg gctggagcaa cggcagctac agcatgatgc aggaccagct gggctacccg 6900
   cagcacccgg gcctcaatgc gcacggcgca gcgcagatgc agcccatgca ccgctacgac 6960
   gtgagcgccc tgcagtacaa ctccatgacc agctcgcaga cctacatgaa cggctcgccc 7020
   acctacagca tgtcctactc gcagcagggc acccctggca tggctcttgg ctccatgggt 7080
   tcggtggtca agtccgaggc cagctccagc ccccctgtgg ttacctcttc ctcccactcc 7140
   agggcgccct gccaggccgg ggacctccgg gacatgatca gcatgtatct ccccggcgcc 7200
   gaggtgccgg aacccgccgc ccccagcaga cttcacatgt cccagcacta ccagagcggc 7260
   ccggtgcccg gcacggccat taacggcaca ctgcccctct cacacatgtg aggacgtcag 7320
   atctgtatat aataagaaaa acttagggtg aaagtgaggt tgcgcggtat tttagctagc 7380
   ttagacgctg gatttttttc gggtagtgga aaaccagcag cctcccgcga cgatgcccct 7440
   caacgttagc ttcaccaaca ggaactatga cctcgactac gactcggtgc agccgtattt 7500
   ctactgcgac gaggaggaga acttctacca gcagcagcag cagagcgagc tgcagccccc 7560
   ggcgcccagc gaggatatct ggaagaaatt cgagctgctg cccaccccgc ccctgtcccc 7620
   tagccgccgc tccgggctct gctcgccctc ctacgttgcg gtcacaccct tctcccttcg 7680
   gggagacaac gacggcggtg gcgggagctt ctccacggcc gaccagctgg agatggtgac 7740
   cgagctgctg ggaggagaca tggtgaacca gagtttcatc tgcgacccgg acgacgagac 7800
   cttcatcaaa aacatcatca tccaggactg tatgtggagc ggcttctcgg ccgccgccaa 7860
   gctcgtctca gagaagctgg cctcctacca ggctgcgcgc aaagacagcg gcagcccgaa 7920
   ccccgcccgc ggccacagcg tctgctccac ctccagcttg tacctgcagg atctgagcgc 7980
   cgccgcctca gagtgcatcg acccctcggt ggtcttcccc taccctctca acgacagcag 8040
   ctcgcccaag tcctgcgcct cgcaagactc cagcgccttc tctccgtcct cggattctct 8100
   gctctcctcg acggagtcct ccccgcaggg cagccccgag cccctggtgc tccatgagga 8160
   gacaccgccc accaccagca gcgactctga ggaggaacaa gaagatgagg aagaaatcga 8220
   tgttgtttct gtggaaaaga ggcaggctcc tggcaaaagg tcagagtctg gatcaccttc 8280
   tgctggaggc cacagcaaac ctcctcacag cccactggtc ctcaagaggt gccacgtctc 8340
   cacacatcag cacaactacg cagcgcctcc ctccactcgg aaggactatc ctgctgccaa 8400
   gagggtcaag ttggacagtg tcagagtcct gagacagatc agcaacaacc gaaaatgcac 8460
   cagccccagg tcctcggaca ccgaggagaa tgtcaagagg cgaacacaca acgtcttgga 8520
   gcgccagagg aggaacgagc taaaacggag cttttttgcc ctgcgtgacc agatcccgga 8580
   gttggaaaac aatgaaaagg cccccaaggt agttatcctt aaaaaagcca cagcatacat 8640
   cctgtccgtc caagcagagg agcaaaagct catttctgaa gaggacttgt tgcggaaacg 8700
   acgagaacag ttgaaacaca aacttgaaca gctacggaac tcttgtgcgt aagaccggtg 8760
   tcggctttgc tgacactaga gtcatctccg aacatccaca atatctctca gtctcttacg 8820
   tctctcacag tattaagaaa aacccagggt gaatgggaag cttgccatag gtcatggatg 8880
   ggcaggagtc ctcccaaaac ccttctgaca tactctatcc agaatgccac ctgaactctc 8940
   ccatagtcag ggggaagata gcacagttgc acgtcttgtt agatgtgaac cagccctaca 9000
   gactaaagga cgacagcata ataaatatta caaagcacaa aattaggaac ggaggattgt 9060
   cccctcgtca aattaagatc aggtctctgg gtaaggctct tcaacgcaca ataaaggatt 9120
   tagaccgata cacctttgaa ccgtacccaa cctactctca ggaattactt aggcttgata 9180
   taccagagat atgtgacaaa atccgatccg tcttcgcggt ctcggatcgg ctgaccaggg 9240
   agttatctag tgggttccag gatctttggt tgaatatctt caagcaacta ggcaatatag 9300
   aaggaagaga ggggtacgat ccgttgcagg atatcggcac catcccggag ataactgata 9360
   aatacagcag gaatagatgg tataggccat tcctaacttg gttcagcatc aaatatgaca 9420
   tgcggtggat gcagaagacc agaccggggg gacccctcga tacctctaat tcacataacc 9480
   tcctagaatg caaatcatac actctagtaa catacggaga tcttatcatg atactgaaca 9540
   agttgacatt gacagggtat atcctaaccc ctgagctggt cttgatgtat tgtgatgttg 9600
   tagagggaag gtggaatatg tctgctgcag ggcatctaga taagaagtcc attgggataa 9660
   caagcaaagg tgaggaatta tgggaactag tggattccct cttctcaagt cttggagagg 9720
   aaatatacaa tgtcatcgca ctattggagc ccctatcact tgctctcata caactaaatg 9780
   atccagttat acctctacgt ggggcattta tgaggcatgt gttgacagag ctacaggctg 9840
   ttttaacaag tagggacgtg tacacagatg ctgaagcaga cactattgtg gagtcgttac 9900
   tcgccatttt ccatggaacc tctattgatg agaaagcaga gatcttttcc ttctttagga 9960
   catttggcca ccccagctta gaggctgtca ctgccgccga caaggtaagg gcccatatgt 10020
   atgcacaaaa ggcaataaag cttaagaccc tatacgagtg tcatgcagtt ttttgcacta 10080
   tcatcataaa tgggtataga gagaggcatg gcggacagtg gcccccctgt gacttccctg 10140
   atcacgtgtg tctagaacta aggaacgctc aagggtccaa tacggcaatc tcttatgaat 10200
   gtgctgtaga caactataca agtttcatag gcttcaagtt tcggaagttt atagaaccac 10260
   aactagatga agatctcaca atatatatga aagacaaagc actatccccc aggaaggagg 10320
   catgggactc tgtatacccg gatagtaatc tgtactataa agccccagaa tctgaagaga 10380
   cccggcggct tattgaagtg ttcataaatg atgagaattt caacccagaa gaaattatca 10440
   attatgtgga gtcaggagat tggttgaaag acgagaagtt caacatctcg tacagtctca 10500
   aagagaaaga gatcaagcaa gagggtcgtc tattcgcaaa aatgacttat aagatgcgag 10560
   ccgtacaggt gctggcagag acactactgg ctaaaggaat aggagagctg ttcagcgaaa 10620
   atgggatggt taaaggagag atagacctac ttaaaagatt gactactctt tctgtctcag 10680
   gagtccccag gactgattca gtgtacaata actctaaatc atcagagaag agaaacgaag 10740
   gcatgaaaaa gaagaactct ggggggtact gggacgaaaa gaagaggtcc agacatgaat 10800
   tcaaggcaac agattcatca acagacggct atgaaacgtt aagttgcttc ctcacaacag 10860
   acctcaagaa atactgctta aactggagat ttgaaagtac tgcattgttt ggtcagagat 10920
   gcaacgagat atttggcttc aagaccttct ttaactggat gcatccagtc cttgaaaggt 10980
   gtacaatata tgttggggat ccttactgtc cagtcgccga ccggatgcat cgacaactcc 11040
   aggatcatgc agactctggc attttcatac ataatcctag ggggggcata gaaggttact 11100
   gccagaagct gtggacctta atctcaatca gtgcaatcca cctagcagct gtgagagtgg 11160
   gtgtcagggt ctctgcaatg gttcagggtg acaatcaagc tatagccgtg acatcaagag 11220
   tacctgtagc tcagacttac aagcagaaga aaaatcatgt ctataaggag atcaccaaat 11280
   attttggtgc tctaagacac gtcatgtttg atgtagggca cgagctaaaa ttgaacgaga 11340
   ccatcattag tagcaagatg tttgtctata gtaaaagaat atactatgat gggaagattt 11400
   taccacagtg cctgaaagcc ttgaccaggt gtgtattctg gtccgagaca ctggtagatg 11460
   aaaacagatc tgcttgttcg aacatctcaa catccatagc aaaagctatc gaaaatgggt 11520
   attctcctat actaggctac tgcattgcgt tgtataagac ctgtcagcag gtgtgcatat 11580
   cactagggat gactataaat ccaactatca gcccgaccgt aagagatcaa tactttaagg 11640
   gtaagaattg gctgagatgt gcagtgttga ttccagcaaa tgttggagga ttcaactaca 11700
   tgtctacatc tagatgcttt gttagaaata ttggagaccc cgcagtagca gccctagctg 11760
   atctcaaaag attcatcaga gcggatctgt tagacaagca ggtactatac agggtcatga 11820
   atcaagaacc cggtgactct agctttctag attgggcttc agacccttat tcatgtaacc 11880
   tcccgcattc tcagagtata actacgatta taaagaatat cactgctaga tctgtgctgc 11940
   aggaatcccc gaatcctcta ctgtctggtc tcttcaccga gactagtgga gaagaggatc 12000
   tcaacctggc ctcgttcctt atggaccgga aagtcatcct gccgagagtg gctcatgaga 12060
   tcctgggtaa ttccttaact ggagttaggg aggcgattgc agggatgctt gatacgacca 12120
   agtctctagt gagatccagc gttaagaaag gaggattatc atatgggata ttgaggaggc 12180
   ttgtcaatta tgatctattg cagtacgaga cactgactag aactctcagg aaaccggtga 12240
   aagacaacat cgaatatgag tatatgtgtt cagttgagct agctgtcggt ctaaggcaga 12300
   aaatgtggat ccacctaact tacgggagac ccatacatgg gctagaaaca ccagaccctt 12360
   tagagctctt gaggggaaca tttatcgaag gttcagaggt gtgcaagctt tgcaggtctg 12420
   agggagcaga ccccatctat acatggttct atctccctga caatatagac ctggacacgc 12480
   ttacaaacgg atgtccggct ataagaatcc cctattttgg atcagccact gatgaaaggt 12540
   cggaagccca actcgggtat gtaagaaatc taagcaaacc cgcaaaggct gccatccgga 12600
   tagctatggt gtatacgtgg gcctacggga ctgatgagat atcgtggatg gaagccgctc 12660
   ttatagccca aacaagagct aatctgagct tagagaatct aaagctgctg actcctgttt 12720
   caacctccac taatctatct cataggttga aagatacggc aacccagatg aagttctcta 12780
   gtgcaacact agtccgtgca agtcggttca taacaatatc aaatgataac atggcactca 12840
   aagaagcagg ggagtcgaag gatactaatc tcgtgtatca gcagattatg ctaactgggc 12900
   taagcttgtt cgagttcaat atgagatata agaaaggttc cttagggaag ccactgatat 12960
   tgcacttaca tcttaataac gggtgctgta taatggagtc cccacaggag gcgaatatcc 13020
   ccccaaggtc cacattagat ttagagatta cacaagagaa caataaattg atctatgatc 13080
   ctgatccact caaggatgtg gaccttgagc tatttagcaa ggtcagagat gttgtacata 13140
   cagttgacat gacttattgg tcagatgatg aagttatcag agcaaccagt atctgtactg 13200
   caatgacgat agctgataca atgtctcaat tagatagaga caacctaaaa gagatgatcg 13260
   cgctagtaaa tgacgatgat gtcaacagcc tgattactga gtttatggtg attgatgttc 13320
   ctttattttg ctcaacgttc gggggtattc tagtcaatca gtttgcatac tcactctacg 13380
   gcttaaacat cagaggaagg gaagaaatat ggggacatgt agtccggatt cttaaagata 13440
   cctcccacgc agttctaaaa gtcttatcta atgctctatc tcatcccaaa atcttcaaac 13500
   gattctggaa tgcaggtgtc gtggaacctg tgtatgggcc taacctctca aatcaggaca 13560
   agatactctt ggccctctct gtctgtgaat attctgtgga tctattcatg cacgattggc 13620
   aagggggtgt accgcttgag atctttatct gtgacaatga cccagatgtg gccgacatga 13680
   ggaggtcctc tttcttggca agacatcttg catacctatg cagcgtggca gagatatcta 13740
   gggatgggcc aagattagaa tcaatgaact ctctagagag gctcgagtca ctaaagagtt 13800
   acctggaact cacatttctt gatgacccgg tactgaggta cagtcagttg actggcctag 13860
   tcatcaaagt attcccatct actttgacct atatccggaa gtcatctata aaagtgttaa 13920
   ggacaagagg tataggagtc cctgaagtct tagaagattg ggatcccgag gcagataatg 13980
   cactgttaga tggtatcgcg gcagaaatac aacagaatat tcctttggga catcagacta 14040
   gagccccttt ttgggggttg agagtatcca agtcacaggt actgcgtctc cgggggtaca 14100
   aggagatcac aagaggtgag ataggcagat caggcgttgg tctgacgtta ccattcgatg 14160
   gaagatatct atctcaccag ctgaggctct ttggcatcaa cagtactagc tgcttgaaag 14220
   cacttgaact tacctaccta ttgagcccct tagttgacaa ggataaagat aggctatatt 14280
   taggggaagg agctggggcc atgctttcct gttatgacgc tactcttggc ccatgcatca 14340
   actattataa ctcaggggta tactcttgtg atgtcaatgg gcagagagag ttaaatatat 14400
   atcctgctga ggtggcactg gtgggaaaga aattaaacaa tgttactagt ctgggtcaaa 14460
   gagttaaagt gttattcaac gggaatcctg gctcgacatg gattggaaat gatgagtgtg 14520
   aggctttgat ttggaatgaa ttgcagaata gctcgatagg cctagtccac tgtgacatgg 14580
   agggaggaga tcataaggat gatcaagttg tactgcatga gcattacagt gtaatccgga 14640
   tcgcgtatct ggtgggggat cgagacgttg tgcttataag caagattgct cctaggctgg 14700
   gcacggattg gaccaggcag ctcagcctat atctgagata ctgggacgag gttaacctaa 14760
   tagtgcttaa aacatctaac cctgcttcca cagagatgta tctcctatcg aggcatccca 14820
   aatctgacat tatagaggac agcaagacgg tgttagctag tctcctccct ttgtcaaaag 14880
   aagatagcat caagatagaa aagtggatct taatagagaa ggcaaaggct cacgaatggg 14940
   ttactcggga attgagagaa ggaagctctt catcagggat gcttagacct taccatcaag 15000
   cactgcagac gtttggcttt gaaccaaact tgtataaatt gagcagagat ttcttgtcca 15060
   ccatgaacat agctgataca cacaactgca tgatagcttt caacagggtt ttgaaggata  15120
   caatcttcga atgggctaga ataactgagt cagataaaag gcttaaacta actggtaagt  15180
   atgacctgta tcctgtgaga gattcaggca aattgaagac agtttctaga agacttgtgc  15240
   tatcttggat atctttatct atgtccacaa gattggtaac tgggtcattc cctgaccaga  15300
   agtttgaagc aagacttcaa ttgggaatag tttcattatc atcccgtgaa atcaggaacc  15360
   tgagggttat cacaaaaact ttattagacc ggtttgagga tattatacat agtataacgt  15420
   acagattcct caccaaagaa ataaagattt tgatgaagat tttaggggca gtcaagatgt  15480
   tcggggccag gcaaaatgaa tacacgaccg tgattgatga tggatcactg ggtgatatcg  15540
   agccatatga cagctcgtaa taccggttat caccaaaaca tggaagcact tacgattcac  15600
   caaaacatgg aagcacttag gtacctcacc aaaacatgga agcacttacg attcaccaaa  15660
   acatggaagc acttaaccgg ttccctatcg tgcagaacga tcgaagctcc gcggtacctg  15720
   gaagtcttgg actgatccat atgacaatag taagaaaaac ttacaagaag acaagaaaat  15780
   ttaaaagaat acatatctct taaactcttg tctggt                            15816
<210> 29
   <211> 21
   <212> RNA
   <213> Artificial
<220>
   <223> siRNA:Sense strand
<400> 29
   gguucagcau caaauaugaa g 21
<210> 30
   <211> 21
   <212> RNA
   <213> Artificial
<220>
   <223> siRNA:Anti-sense strand
<400> 30
   ucauauuuga ugcugaacca u 21
<210> 31
   <211> 20
   <212> DNA
   <213> Artificial
<220>
   <223> Primer
<400> 31
   agcatccgac tgtaaagaat 20
<210> 32
   <211> 20
   <212> DNA
   <213> Artificial
<220>
   <223> Primer
<400> 32
   cctctccaca gttatagaag 20
<210> 33
   <211> 20
   <212> DNA
   <213> Artificial
<220>
   <223> Primer
<400> 33
   aagaccctaa gaggacgaag 20
<210> 34
   <211> 25
   <212> DNA
   <213> Artificial
<220>
   <223> Primer
<400> 34
   actcccatgg cgtaactcca tagtg 25